(19) Europäisches Patentamt
European Patent Office
Office européen des brevets

(11) EP 3 315 581 B1

(12) **EUROPÄISCHE PATENTSCHRIFT**

(45) Veröffentlichungstag und Bekanntmachung des Hinweises auf die Patenterteilung:
**02.01.2019 Patentblatt 2019/01**

(51) Int Cl.:
***C09K 11/06*** *(2006.01)* ***H01L 51/50*** *(2006.01)*
***C07D 403/10*** *(2006.01)* ***C07D 403/14*** *(2006.01)*

(21) Anmeldenummer: **17199522.8**

(22) Anmeldetag: **01.11.2017**

(54) **ORGANISCHE MOLEKÜLE, INSBESONDERE ZUR VERWENDUNG IN ORGANISCHEN OPTOELEKTRONISCHEN VORRICHTUNGEN**

ORGANIC MOLECULES, IN PARTICULAR FOR USE IN ORGANIC OPTOELECTRONIC DEVICES

MOLÉCULES ORGANIQUES, EN PARTICULIER DESTINÉES À ÊTRE UTILISÉES DANS DES COMPOSANTS OPTOÉLECTRONIQUES ORGANIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.11.2016 DE 102016120802**
**06.02.2017 DE 102017102267**
**16.03.2017 DE 102017105693**

(43) Veröffentlichungstag der Anmeldung:
**02.05.2018 Patentblatt 2018/18**

(73) Patentinhaber: **CYNORA GMBH**
**76646 Bruchsal (DE)**

(72) Erfinder: **BERGMANN, Larissa**
**76199 Karlsruhe (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2018/016898 US-A1- 2015 243 903**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 315 581 B1

**Beschreibung**

Die Erfindung betrifft rein organische Moleküle und deren Verwendung in organischen lichtemittierenden Dioden (OLEDs) und in anderen organischen optoelektronischen Vorrichtungen.

**Beschreibung**

Aus der WO 2018/016898 A1, die am 25.01.2018 veröffentlicht wurde, ist die folgende Verbindung bekannt:

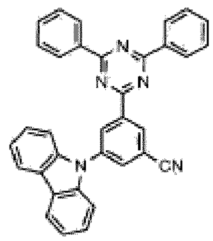

Der vorliegenden Erfindung lag die Aufgabe zu Grunde, Moleküle bereitzustellen, die sich zur Verwendung in optoelektronischen Vorrichtungen eignen.
Gelöst wird diese Aufgabe durch die hier beschriebene neue Klasse von organischen Molekülen.
Die erfindungsgemäßen organischen Moleküle sind rein organische Moleküle, weisen also keine Metallionen auf und grenzen sich so von den zur Verwendung in organischen optoelektronischen Vorrichtungen bekannten Metallkomplexverbindungen ab.
Die erfindungsgemäßen organischen Moleküle zeichnen sich durch Emissionen im blauen, himmelblauen oder grünen Spektralbereich aus. Die Photolumineszenzquantenausbeuten der erfindungsgemäßen organischen Moleküle betragen insbesondere 20 % und mehr. Die erfindungsgemäßen Moleküle zeigen insbesondere thermisch aktivierte verzögerte Fluoreszenz (TADF). Die Verwendung der erfindungsgemäßen Moleküle in einer optoelektronischen Vorrichtung, beispielsweise einer organischen lichtemittierenden Diode (OLED), führt zu höheren Effizienzen der Vorrichtung. Entsprechende OLEDs weisen eine höhere Stabilität auf als OLEDs mit bekannten Emittermaterialien und vergleichbarer Farbe.
Unter dem blauen Spektralbereich wird hier der sichtbare Bereich von 420 bis 470 nm verstanden. Unter dem himmelblauen Spektralbereich wird hier der Bereich von 470 nm bis 499 nm verstanden. Unter dem grünen Spektralbereich wird hier der Bereich von 500 nm bis 599 nm verstanden. Dabei liegt das Emissionsmaximum im jeweiligen Bereich.
Die organischen Moleküle enthalten eine erste chemische Einheit aufweisend eine oder bestehend aus einer Struktur gemäß Formel I:

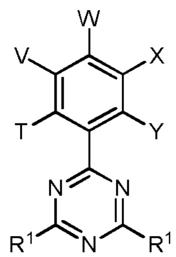

Formel I

und

- eine zweite chemische Einheit D aufweisend eine oder bestehend aus einer Struktur gemäß Formel II,

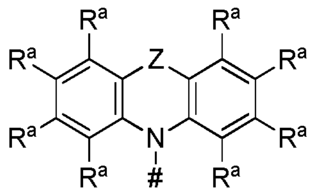

Formel II

Hierbei ist die erste chemische Einheit über eine Einfachbindung mit der zweiten chemischen Einheit D verknüpft.

T ist Anknüpfungspunkt der Einfachbindung zwischen der ersten chemischen Einheit und der zweiten chemischen Einheit D oder ausgewählt aus der Gruppe bestehend aus $R^2$ und CN.

V ist H oder Anknüpfungspunkt der Einfachbindung zwischen der ersten chemischen Einheit und der zweiten chemischen Einheit D.

W ist Anknüpfungspunkt der Einfachbindung zwischen der ersten chemischen Einheit und der zweiten chemischen Einheit D oder ausgewählt aus der Gruppe bestehend aus $R^2$ und CN.

X ist ausgewählt aus der Gruppe bestehend aus $R^2$ und CN.

Y ist ausgewählt aus der Gruppe bestehend aus $R^2$ und CN.

# ist Anknüpfungspunkt der Einfachbindung zwischen der zweiten chemischen Einheit D und der ersten chemischen Einheit.

Z ist bei jedem Auftreten gleich oder verschieden eine direkte Bindung oder ausgewählt aus der Gruppe bestehend aus $CR^3R^4$, $C{=}CR^3R^4$, C=O, $C{=}NR^3$, $NR^3$, O, $SiR^3R^4$, S, S(O) und $S(O)_2$.

$R^1$ ist bei jedem Auftreten gleich oder verschieden H, Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium ersetzt sein können, oder ein aromatisches oder heteroaromatisches Ringsystem mit 5 bis 15 aromatischen Ringatomen, das jeweils durch einen oder mehrere Reste $R^6$ substituiert sein kann.

$R^2$ ist bei jedem Auftreten gleich oder verschieden H, Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium ersetzt sein können, oder ein aromatisches oder heteroaromatisches Ringsystem mit 5 bis 15 aromatischen Ringatomen, das jeweils durch einen oder mehrere Reste $R^6$ substituiert sein kann.

$R^a$, $R^3$ und $R^4$ ist bei jedem Auftreten gleich oder verschieden H, Deuterium, $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann.

$R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C{=}CR^6$, C≡C, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^6$ substituiert sein kann, oder eine Aryloxy- oder Heteroaryloxygruppe mit 5 bis 60 aromatischen Ringatomen, die durch einen oder mehrere Reste $R^6$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^6$ substituiert sein kann.

$R^6$ ist bei jedem Auftreten gleich oder verschieden H, Deuterium, OH, $CF_3$, CN, F, eine lineare Alkyl-, Alkoxy- oder Thioalkoxygruppe mit 1 bis 5 C-Atomen oder eine lineare Alkenyl- oder Alkinylgruppe mit 2 bis 5 C-Atomen oder eine verzweigte oder cyclische Alkyl-, Alkenyl-, Alkinyl-, Alkoxy- oder Thioalkoxygruppe mit 3 bis 5 C-Atomen, wobei

ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen oder eine Aryloxy- oder Heteroaryloxygruppe mit 5 bis 60 aromatischen Ringatomen oder eine Diarylaminogruppe, Diheteroarylaminogruppe oder Arylheteroarylaminogruppe mit 10 bis 40 aromatischen Ringatomen.

Jeder der Reste $R^a$, $R^3$, $R^4$ oder $R^5$ kann auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden.

Genau ein Rest ausgewählt aus der Gruppe bestehend aus T, W, X und Y ist gleich CN und genau ein Rest ausgewählt aus der Gruppe bestehend aus T, V und W ist gleich dem Anknüpfungspunkt der Einfachbindung zwischen der ersten chemischen Einheit gemäß Formel I und der zweiten chemischen Einheit D.

Erfindungsgemäß ist W gleich H, wenn T gleich CN und V gleich Anknüpfungspunkt der Einfachbindung zwischen der ersten chemischen Einheit und der zweiten chemischen Einheit D ist.

Eine Verbindung der Formel

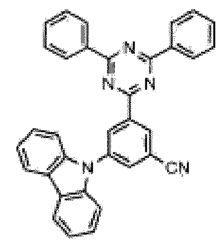

ist dabei ausgeschlossen.

Die erfindungsgemäßen organischen Moleküle zeichnen sich unter anderem durch eine gute thermische Stabilität und Sublimierbarkeit aus, insbesondere im Rahmen der Herstellung eines optoelektronischen Vorrichtung (z.B. einer OLED) beim Aufdampfen stabil sind.

In einer Ausführungsform ist $R^1$ bei jedem Auftreten gleich oder verschieden Methyl oder Phenyl.

In einer Ausführungsform ist $R^2$ bei jedem Auftreten gleich oder verschieden H, Methyl oder Phenyl.

In einer Ausführungsform ist $R^2$ bei jedem Auftreten gleich H.

In einer Ausführungsform ist W gleich CN.

In einer Ausführungsform ist X gleich CN.

In einer Ausführungsform ist X gleich CN und T ist gleich dem Anknüpfungspunkt der Einfachbindung zwischen der ersten chemischen Einheit und der zweiten chemischen Einheit D.

In einer weiteren Ausführungsform der organischen Moleküle weist die zweite chemische Gruppe D eine Struktur der Formel IIa auf bzw. besteht aus einer Struktur der Formel IIa:

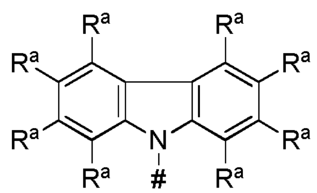

Formel IIa

wobei für # und $R^a$ die für Formel I und II genannten Definitionen gelten.

In einer weiteren Ausführungsform der erfindungsgemäßen organischen Moleküle weist die zweite chemische Einheit D eine Struktur der Formel IIb, der Formel IIb-2, der Formel IIb-3 oder der Formel IIb-4 auf oder besteht daraus:

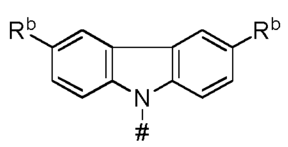

Formel IIb

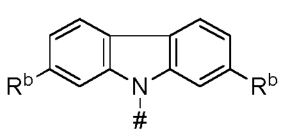

Formel IIb-2

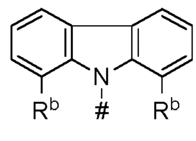

Formel IIb-3

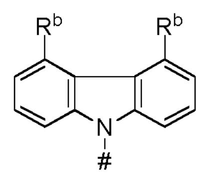

Formel IIb-4

wobei

$R^b$ bei jedem Auftreten gleich oder verschieden ist $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann. Ansonsten gelten die oben genannten Definitionen.

In einer weiteren Ausführungsform der erfindungsgemäßen organischen Moleküle weist die zweite chemische Einheit D eine Struktur der Formel IIc, der Formel IIc-2, der Formel IIc-3 oder der Formel IIc-4 auf oder besteht daraus:

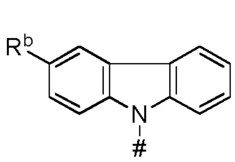
Formel IIc

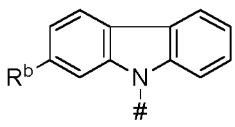
Formel IIc-2

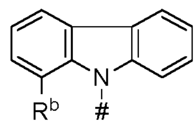
Formel IIc-3

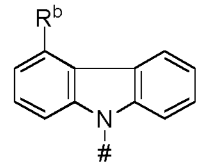
Formel IIc-4

wobei die oben genannten Definitionen gelten.

In einer weiteren Ausführungsform der erfindungsgemäßen organischen Moleküle ist $R^b$ bei jedem Auftreten unabhängig voneinander ausgewählt aus der Gruppe bestehend aus Me, $^i$Pr, $^t$Bu, CN, $CF_3$, Ph, das jeweils durch einen oder mehrere Reste ausgewählt aus Me, $^i$Pr, $^t$Bu, CN, $CF_3$ oder Ph substituiert sein kann, Pyridinyl, das jeweils durch einen oder mehrere Reste ausgewählt aus Me, $^i$Pr, $^t$Bu, CN, $CF_3$ oder Ph substituiert sein kann, Pyrimidinyl, das jeweils durch einen oder mehrere Reste ausgewählt aus Me, $^i$Pr, $^t$Bu, CN, $CF_3$ oder Ph substituiert sein kann, Carbazolyl, das jeweils durch einen oder mehrere Reste ausgewählt aus Me, $^i$Pr, $^t$Bu, CN, $CF_3$ oder Ph substituiert sein kann, Triazinyl, das jeweils durch einen oder mehrere Reste ausgewählt aus Me, $^i$Pr, $^t$Bu, CN, $CF_3$ oder Ph substituiert sein kann, und $N(Ph)_2$.

In einer weiteren Ausführungsform der erfindungsgemäßen organischen Moleküle ist $R^b$ bei jedem Auftreten unabhängig voneinander ausgewählt aus der Gruppe bestehend aus Me, $^i$Pr, $^t$Bu, CN, $CF_3$, Ph, das jeweils durch einen oder mehrere Reste ausgewählt aus Me, $^i$Pr, $^t$Bu, CN, $CF_3$ oder Ph substituiert sein kann, Pyridinyl, das jeweils durch einen oder mehrere Reste ausgewählt aus Me, $^i$Pr, $^t$Bu, CN, $CF_3$ oder Ph substituiert sein kann, Pyrimidinyl, das jeweils durch einen oder mehrere Reste ausgewählt aus Me, $^i$Pr, $^t$Bu, CN, $CF_3$ oder Ph substituiert sein kann und Triazinyl, das jeweils durch einen oder mehrere Reste ausgewählt aus Me, $^i$Pr, $^t$Bu, CN, $CF_3$ oder Ph substituiert sein kann.

In einer weiteren Ausführungsform ist $R^b$ bei jedem Auftreten unabhängig voneinander ausgewählt aus der Gruppe bestehend aus Me, $^t$Bu, CN, $CF_3$ und Ph, das jeweils durch einen oder mehrere Reste ausgewählt aus der Gruppe bestehend aus Me, $^i$Pr, $^t$Bu, CN, $CF_3$ und Ph substituiert sein kann.

Im Folgenden sind Beispiele der zweiten chemischen Gruppe D gezeigt:

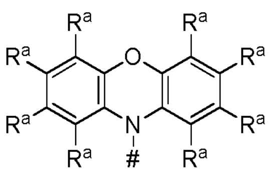

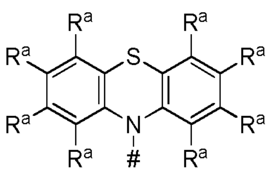

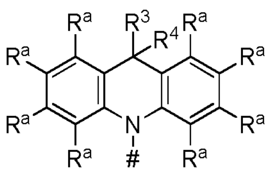

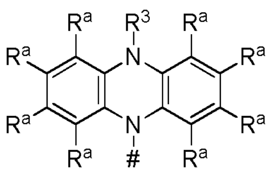

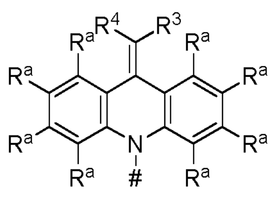

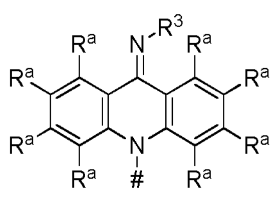

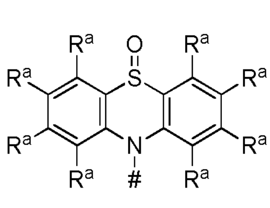

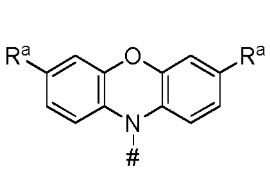
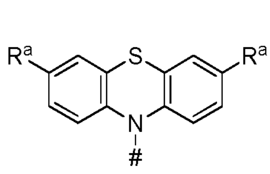
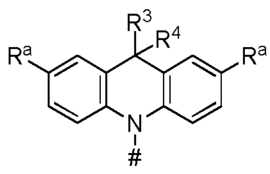
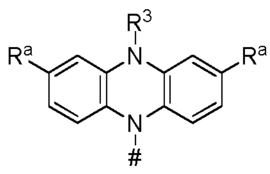

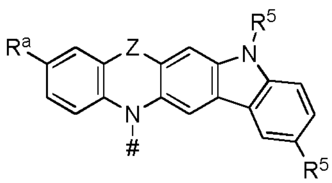
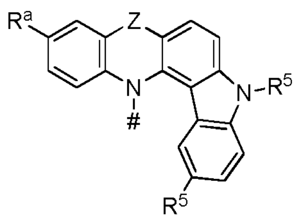
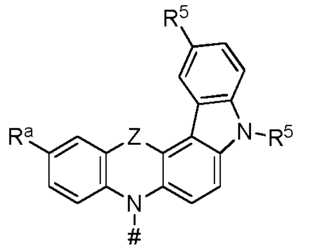

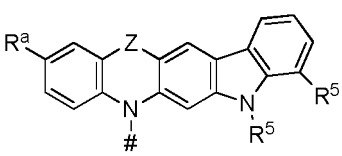
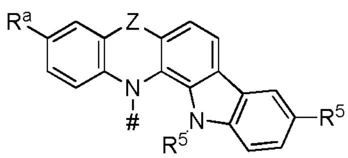
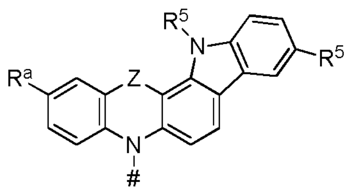

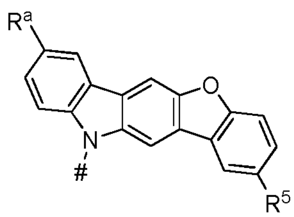
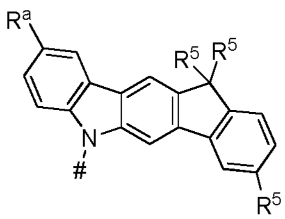
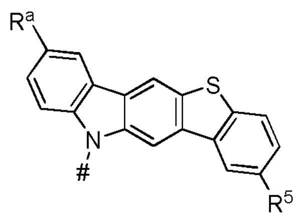

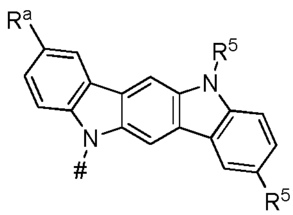

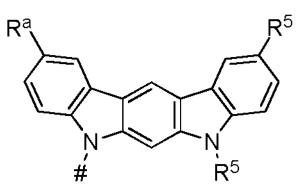
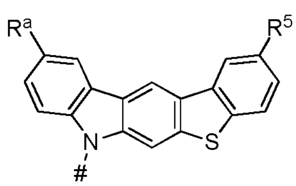
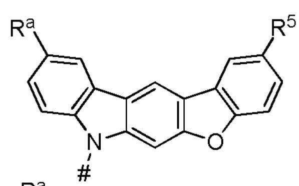

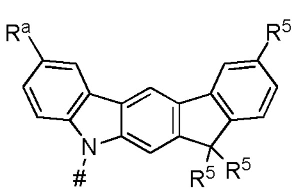
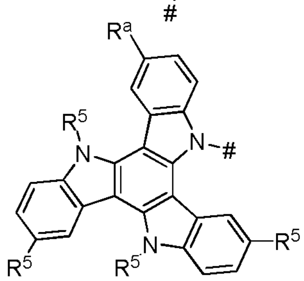

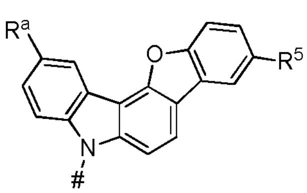
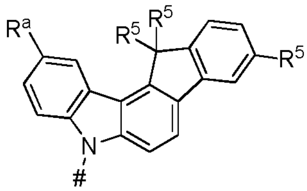
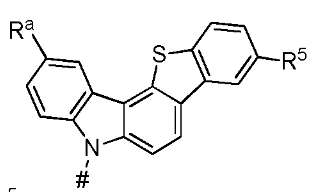

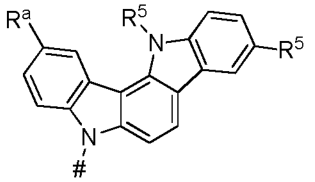
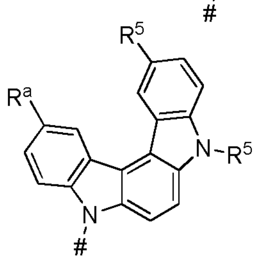

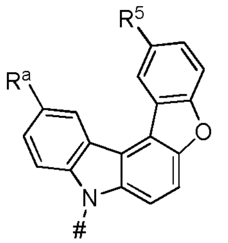
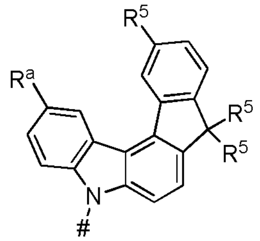
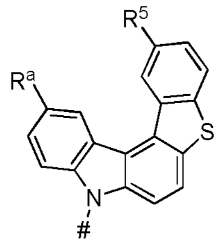

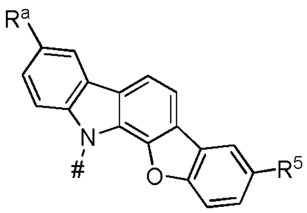
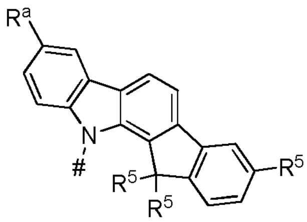
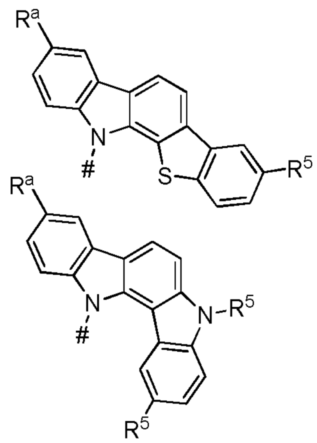

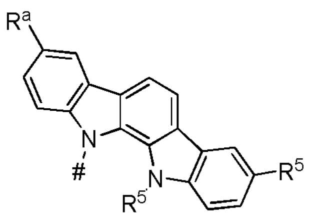

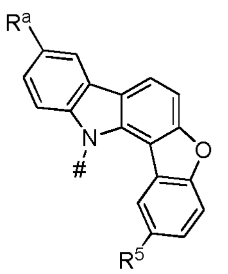
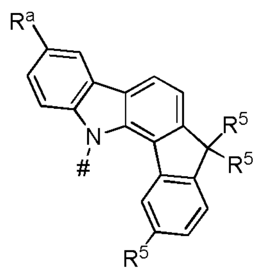
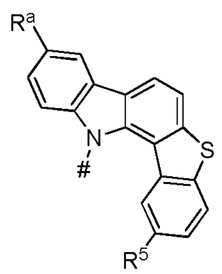

wobei für #, Z, $R^a$, $R^3$, $R^4$ und $R^5$ die oben genannten Definitionen gelten. In einer Ausführungsform ist der Rest $R^5$ bei jedem Auftreten gleich oder verschieden ausgewählt aus der Gruppe bestehend aus H, Methyl, Ethyl, Phenyl und Mesityl.

In einer Ausführungsform ist $R^a$ bei jedem Auftreten gleich oder verschieden ausgewählt aus der Gruppe bestehend aus H, Methyl (Me), i-Propyl ($CH(CH_3)_2$) ($^i$Pr), t-Butyl ($^t$Bu), Phenyl (Ph), CN, $CF_3$ und Diphenylamin ($NPh_2$).

In einer Ausführungsform weisen die erfindungsgemäßen organischen Moleküle eine Struktur der Formel III auf oder bestehen daraus:

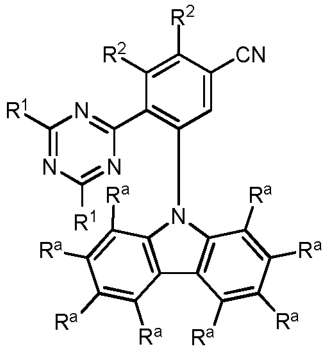

Formel III

wobei
die oben genannten Definitionen gelten.

In einer weiteren Ausführungsform weisen die erfindungsgemäßen organischen Moleküle eine Struktur der Formel IIIa auf oder bestehen daraus:

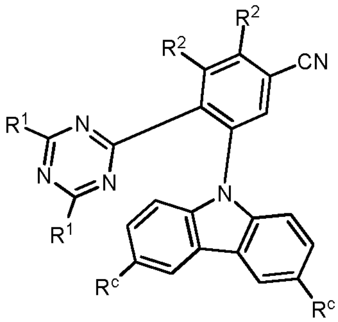

Formel IIIa

wobei $R^c$ bei jedem Auftreten unabhängig voneinander ausgewählt ist aus der Gruppe bestehend aus Me, $^i$Pr, $^t$Bu, CN, $CF_3$, Ph, das jeweils durch einen oder mehrere Reste ausgewählt aus Me, $^i$Pr, $^t$Bu, CN, $CF_3$ oder Ph substituiert sein kann, Pyridinyl, das jeweils durch einen oder mehrere Reste ausgewählt aus Me, $^i$Pr, $^t$Bu, CN, $CF_3$ oder Ph substituiert sein kann, Pyrimidinyl, das jeweils durch einen oder mehrere Reste ausgewählt aus Me, $^i$Pr, $^t$Bu, CN, $CF_3$ oder Ph substituiert sein kann, Carbazolyl, das jeweils durch einen oder mehrere Reste ausgewählt aus Me, $^i$Pr, $^t$Bu, CN, $CF_3$ oder Ph substituiert sein kann, Triazinyl, das jeweils durch einen oder mehrere Reste ausgewählt aus Me, $^i$Pr, $^t$Bu, CN, $CF_3$ oder Ph substituiert sein kann, und $N(Ph)_2$
und ansonsten die oben genannten Definitionen gelten.

In einer weiteren Ausführungsform weisen die erfindungsgemäßen organischen Moleküle eine Struktur der Formel IIIb auf oder bestehen daraus:

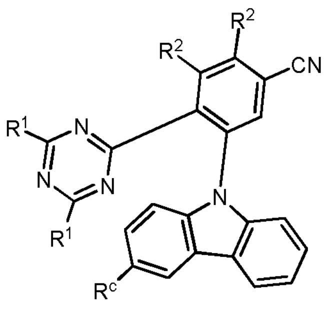

Formel IIIb

wobei die oben genannten Definitionen gelten.

In einer weiteren Ausführungsform weisen die erfindungsgemäßen organischen Moleküle eine Struktur der

Formel IIIc auf oder bestehen daraus:

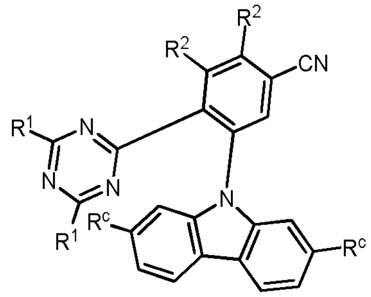

Formel IIIc

wobei die oben genannten Definitionen gelten.

In einer weiteren Ausführungsform weisen die erfindungsgemäßen organischen Moleküle eine Struktur der Formel IIId auf oder bestehen daraus:

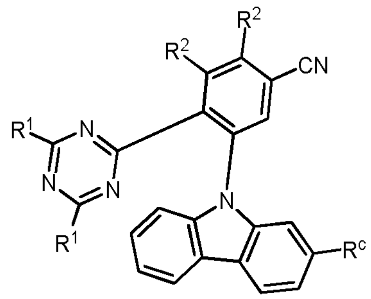

Formel IIId

wobei die oben genannten Definitionen gelten.

In einer weiteren Ausführungsform weisen die erfindungsgemäßen organischen Moleküle eine Struktur der Formel IIIe auf oder bestehen daraus:

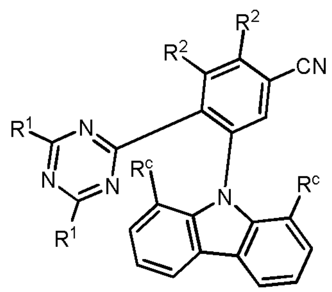

Formel IIIe

wobei die oben genannten Definitionen gelten.

In einer weiteren Ausführungsform weisen die erfindungsgemäßen organischen Moleküle eine Struktur der Formel IIIf auf oder bestehen aus dieser Struktur:

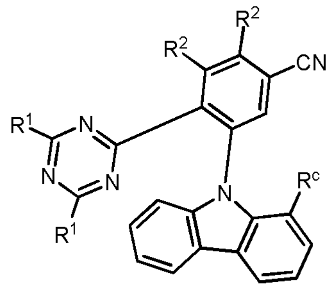

Formel IIIf

wobei die oben genannten Definitionen gelten.

In einer weiteren Ausführungsform weisen die erfindungsgemäßen organischen Moleküle eine Struktur der Formel IIIg auf oder bestehen daraus:

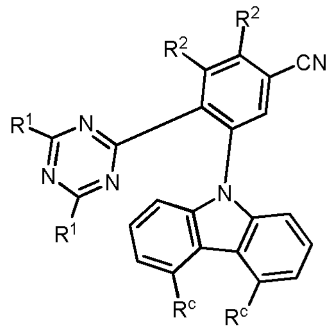

Formel IIIg

wobei die oben genannten Definitionen gelten.

In einer weiteren Ausführungsform weisen die erfindungsgemäßen organischen Moleküle eine Struktur der Formel IIIh auf oder bestehen aus dieser Struktur:

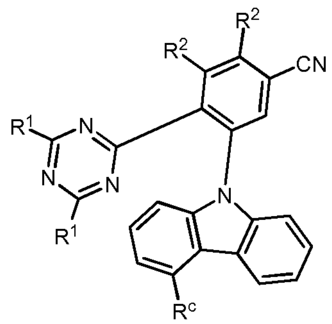

Formel IIIh

wobei die oben genannten Definitionen gelten.

In einer Ausführungsform weisen die erfindungsgemäßen organischen Moleküle eine Struktur der Formel IV auf oder bestehen daraus:

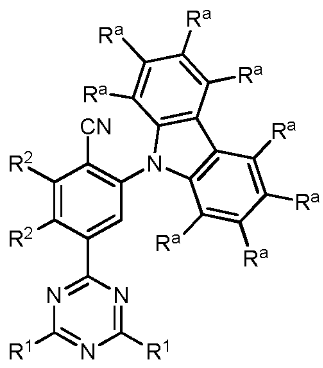

Formel IV

wobei die oben genannten Definitionen gelten.

In einer weiteren Ausführungsform weisen die erfindungsgemäßen organischen Moleküle eine Struktur der Formel IVa auf:

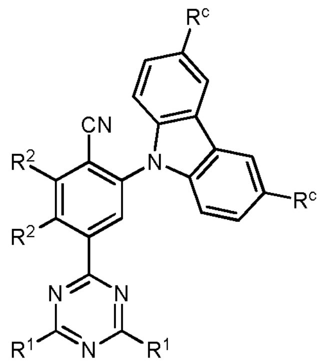

Formel IVa

wobei die oben genannten Definitionen gelten.

In einer weiteren Ausführungsform weisen die erfindungsgemäßen organischen Moleküle eine Struktur der Formel IVb auf oder bestehen daraus:

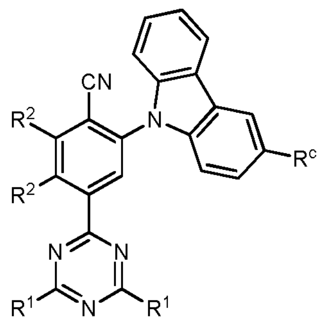

Formel IVb

wobei die oben genannten Definitionen gelten.

In einer weiteren Ausführungsform weisen die erfindungsgemäßen organischen Moleküle eine Struktur der Formel IVc auf oder bestehen daraus:

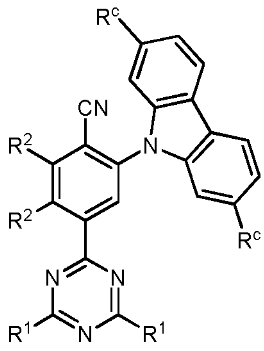

Formel IVc

wobei die oben genannten Definitionen gelten.

In einer weiteren Ausführungsform weisen die erfindungsgemäßen organischen Moleküle eine Struktur der Formel IVd auf oder bestehen daraus:

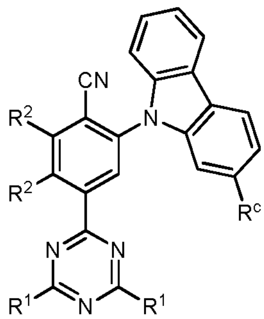

Formel IVd

wobei die oben genannten Definitionen gelten.

In einer weiteren Ausführungsform weisen die erfindungsgemäßen organischen Moleküle eine Struktur der Formel IVe auf oder bestehen daraus:

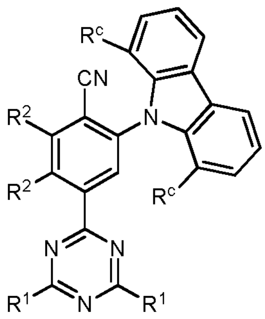

Formel IVe

wobei die oben genannten Definitionen gelten.

In einer weiteren Ausführungsform weisen die erfindungsgemäßen organischen Moleküle eine Struktur der Formel IVf auf oder bestehen daraus:

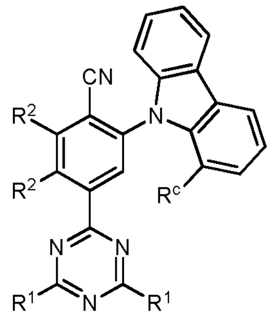

Formel IVf

wobei die oben genannten Definitionen gelten.

In einer weiteren Ausführungsform weisen die erfindungsgemäßen organischen Moleküle eine Struktur der Formel IVg auf oder bestehen daraus:

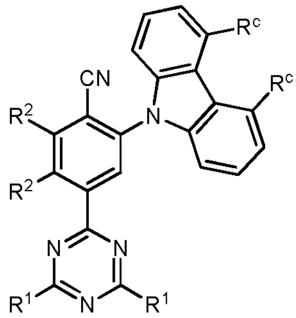

Formel IVg

wobei die oben genannten Definitionen gelten.

In einer weiteren Ausführungsform weisen die erfindungsgemäßen organischen Moleküle eine Struktur der Formel IVh auf oder bestehen daraus:

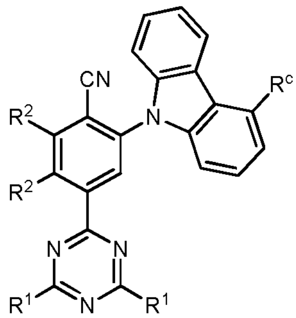

Formel IVh

wobei die oben genannten Definitionen gelten.

In einer Ausführungsform weisen die erfindungsgemäßen organischen Moleküle eine Struktur der Formel V auf oder bestehen daraus:

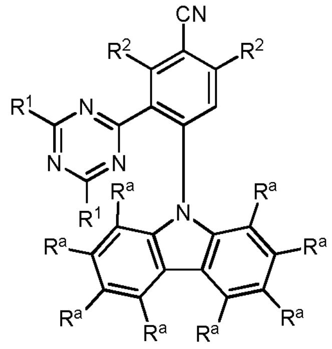

Formel V

wobei die oben genannten Definitionen gelten.

In einer weiteren Ausführungsform weisen die erfindungsgemäßen organischen Moleküle eine Struktur der Formel Va auf oder bestehen daraus:

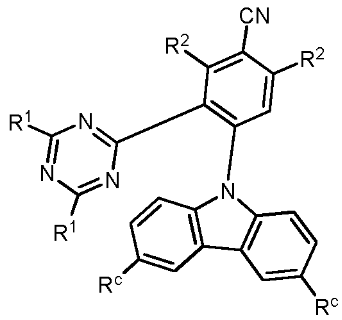

Formel Va

wobei die oben genannten Definitionen gelten.

In einer weiteren Ausführungsform weisen die erfindungsgemäßen organischen Moleküle eine Struktur der Formel Vb auf oder bestehen daraus:

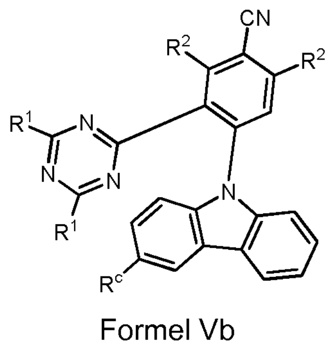

Formel Vb

wobei die oben genannten Definitionen gelten.

In einer weiteren Ausführungsform weisen die erfindungsgemäßen organischen Moleküle eine Struktur der Formel Vc auf oder bestehen daraus:

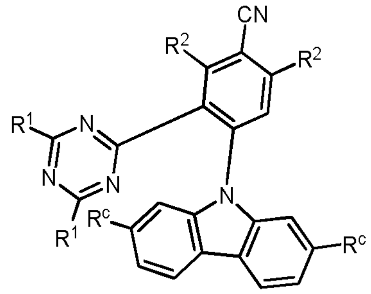

Formel Vc

wobei die oben genannten Definitionen gelten.

In einer weiteren Ausführungsform weisen die erfindungsgemäßen organischen Moleküle eine Struktur der Formel Vd auf oder bestehen daraus:

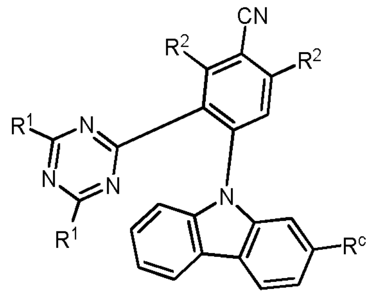

Formel Vd

wobei die oben genannten Definitionen gelten.

In einer weiteren Ausführungsform weisen die erfindungsgemäßen organischen Moleküle eine Struktur der Formel Ve auf oder bestehen daraus:

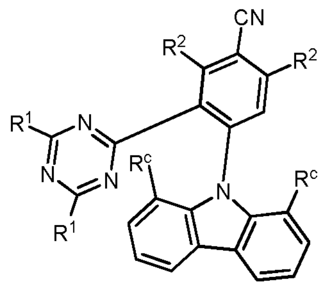

Formel Ve

wobei die oben genannten Definitionen gelten.

In einer weiteren Ausführungsform weisen die erfindungsgemäßen organischen Moleküle eine Struktur der Formel Vf auf oder bestehen aus dieser Struktur:

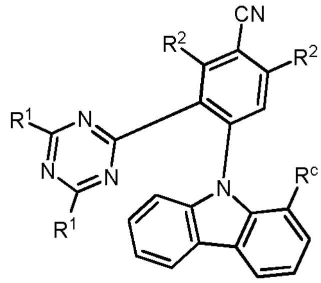

Formel Vf

wobei die oben genannten Definitionen gelten.

In einer weiteren Ausführungsform weisen die erfindungsgemäßen organischen Moleküle eine Struktur der Formel Vg auf oder bestehen daraus:

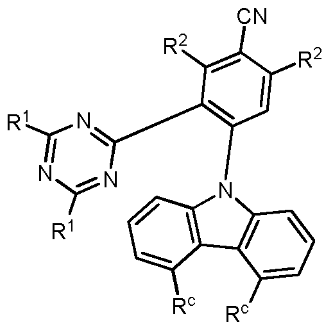

Formel Vg

wobei die oben genannten Definitionen gelten.

In einer weiteren Ausführungsform weisen die erfindungsgemäßen organischen Moleküle eine Struktur der Formel Vh auf oder bestehen aus dieser Struktur:

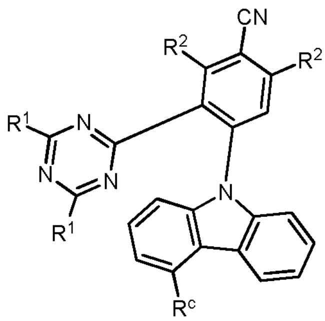

Formel Vh

wobei die oben genannten Definitionen gelten.

In einer Ausführungsform weisen die erfindungsgemäßen organischen Moleküle eine Struktur der Formel VI auf oder bestehen daraus:

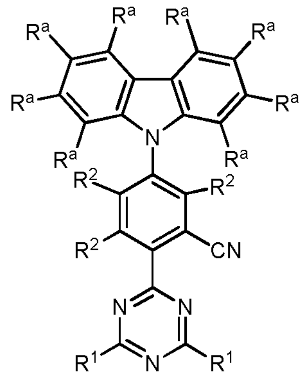

Formel VI

wobei die oben genannten Definitionen gelten.

In einer weiteren Ausführungsform weisen die erfindungsgemäßen organischen Moleküle eine Struktur der Formel VIa auf oder bestehen daraus:

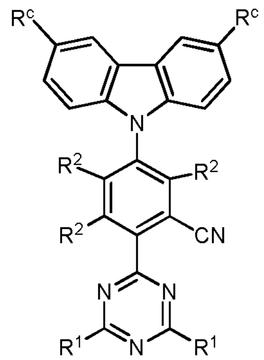

Formel VIa

wobei die oben genannten Definitionen gelten.

In einer weiteren Ausführungsform weisen die erfindungsgemäßen organischen Moleküle eine Struktur der Formel VIb auf oder bestehen daraus:

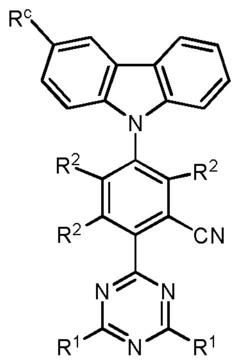

Formel VIb

wobei die oben genannten Definitionen gelten.

In einer weiteren Ausführungsform weisen die erfindungsgemäßen organischen Moleküle eine Struktur der Formel VIc auf oder bestehen daraus:

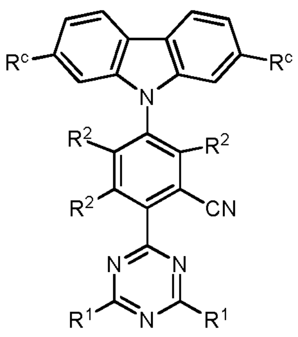

Formel VIc

wobei die oben genannten Definitionen gelten.

In einer weiteren Ausführungsform weisen die erfindungsgemäßen organischen Moleküle eine Struktur der Formel VId auf oder bestehen daraus:

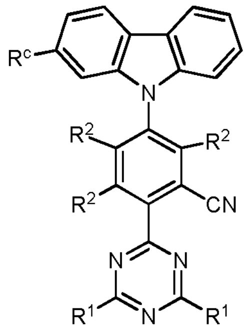

Formel VId

wobei die oben genannten Definitionen gelten.

In einer weiteren Ausführungsform weisen die erfindungsgemäßen organischen Moleküle eine Struktur der Formel VIe auf oder bestehen aus dieser Struktur:

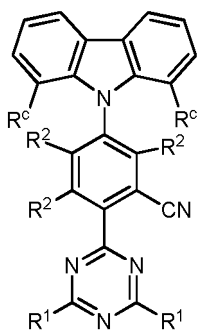

Formel VIe

wobei die oben genannten Definitionen gelten.

In einer weiteren Ausführungsform weisen die erfindungsgemäßen organischen Moleküle eine Struktur der Formel VIf auf oder bestehen daraus:

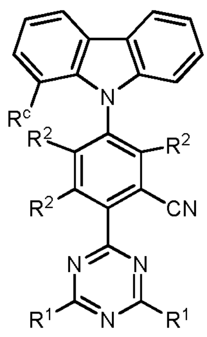

Formel VIf

wobei die oben genannten Definitionen gelten.

In einer weiteren Ausführungsform weisen die erfindungsgemäßen organischen Moleküle eine Struktur der Formel VIg auf oder bestehen daraus:

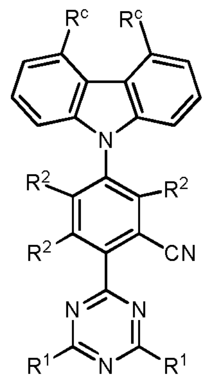

Formel VIg

wobei die oben genannten Definitionen gelten.

In einer weiteren Ausführungsform weisen die erfindungsgemäßen organischen Moleküle eine Struktur der Formel VIh auf oder bestehen daraus:

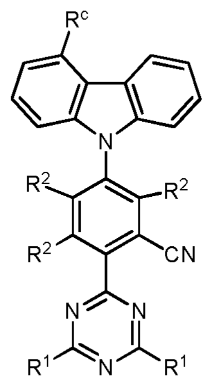

Formel VIh

wobei die oben genannten Definitionen gelten.

In einer Ausführungsform weisen die erfindungsgemäßen organischen Moleküle eine Struktur der Formel VII auf oder bestehen daraus:

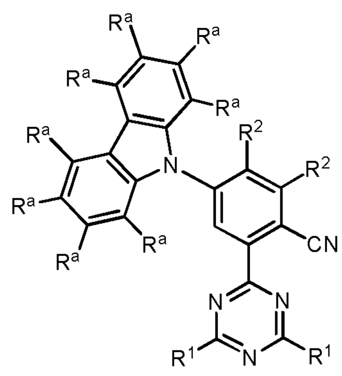

Formel VII

wobei die oben genannten Definitionen gelten.

In einer weiteren Ausführungsform weisen die erfindungsgemäßen organischen Moleküle eine Struktur der Formel VIIa auf oder bestehen daraus:

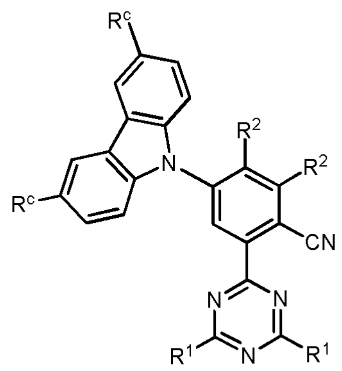

Formel VIIa

wobei die oben genannten Definitionen gelten.

In einer weiteren Ausführungsform weisen die erfindungsgemäßen organischen Moleküle eine Struktur der Formel VIIb auf oder bestehen daraus:

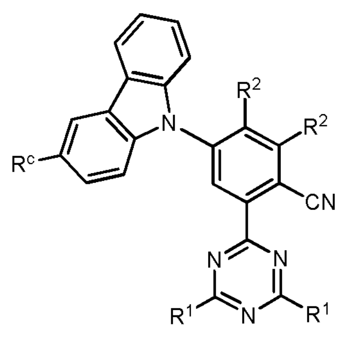

Formel VIIb

wobei die oben genannten Definitionen gelten.

In einer weiteren Ausführungsform weisen die erfindungsgemäßen organischen Moleküle eine Struktur der Formel VIIc auf oder bestehen daraus:

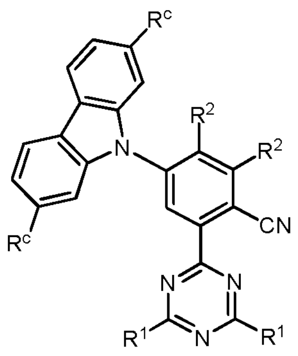

Formel VIIc

wobei die oben genannten Definitionen gelten.

In einer weiteren Ausführungsform weisen die erfindungsgemäßen organischen Moleküle eine Struktur der Formel VIId auf oder bestehen daraus:

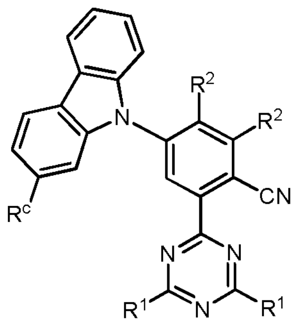

Formel VIId

wobei die oben genannten Definitionen gelten.

In einer weiteren Ausführungsform weisen die erfindungsgemäßen organischen Moleküle eine Struktur der Formel VIIe auf oder bestehen aus dieser Struktur:

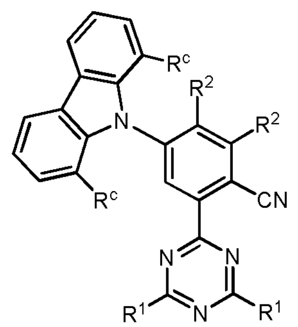

Formel VIIe

wobei die oben genannten Definitionen gelten.

In einer weiteren Ausführungsform weisen die erfindungsgemäßen organischen Moleküle eine Struktur der Formel VIIf auf oder bestehen daraus:

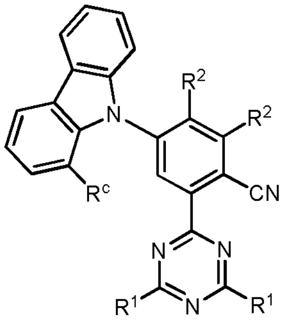

Formel VIIf

wobei die oben genannten Definitionen gelten.

In einer weiteren Ausführungsform weisen die erfindungsgemäßen organischen Moleküle eine Struktur der Formel VIIg auf oder bestehen daraus:

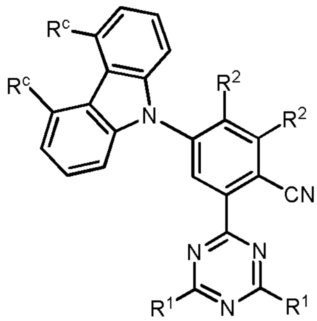

Formel VIIg

wobei die oben genannten Definitionen gelten.

In einer weiteren Ausführungsform weisen die erfindungsgemäßen organischen Moleküle eine Struktur der Formel VIIh auf oder bestehen daraus:

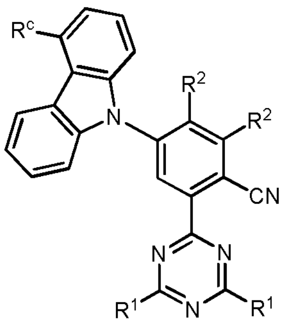

Formel VIIh

wobei die oben genannten Definitionen gelten.

In einer Ausführungsform weisen die erfindungsgemäßen organischen Moleküle eine Struktur der Formel VIII auf oder bestehen daraus:

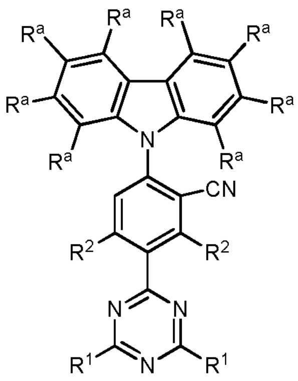

Formel VIII

wobei die oben genannten Definitionen gelten.

In einer weiteren Ausführungsform weisen die erfindungsgemäßen organischen Moleküle eine Struktur der Formel VIIIa auf oder bestehen daraus:

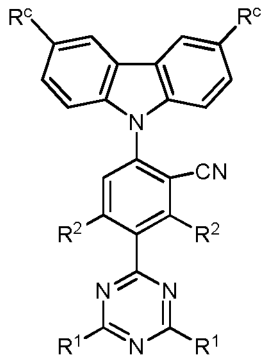

Formel VIIIa

wobei die oben genannten Definitionen gelten.

In einer weiteren Ausführungsform weisen die erfindungsgemäßen organischen Moleküle eine Struktur der Formel VIIIb auf oder bestehen daraus:

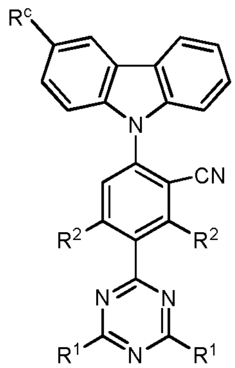

Formel VIIIb

wobei die oben genannten Definitionen gelten.

In einer weiteren Ausführungsform weisen die erfindungsgemäßen organischen Moleküle eine Struktur der Formel VIIIc auf oder bestehen daraus:

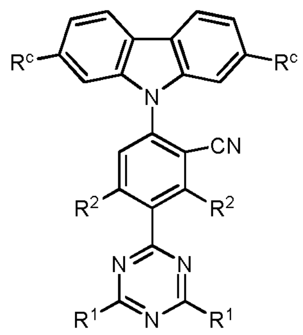

Formel VIIIc

wobei die oben genannten Definitionen gelten.

In einer weiteren Ausführungsform weisen die erfindungsgemäßen organischen Moleküle eine Struktur der Formel VIIId auf oder bestehen daraus:

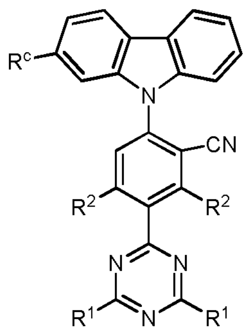

Formel VIIId

wobei die oben genannten Definitionen gelten.

In einer weiteren Ausführungsform weisen die erfindungsgemäßen organischen Moleküle eine Struktur der Formel VIIIe auf oder bestehen aus dieser Struktur:

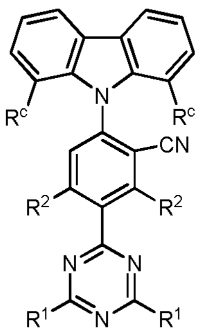

Formel VIIIe

wobei die oben genannten Definitionen gelten.

In einer weiteren Ausführungsform weisen die erfindungsgemäßen organischen Moleküle eine Struktur der Formel VIIIf auf oder bestehen daraus:

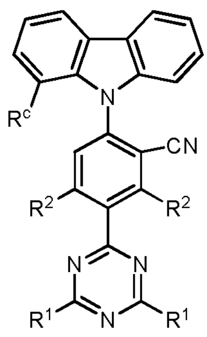

Formel VIIIf

wobei die oben genannten Definitionen gelten.

In einer weiteren Ausführungsform weisen die erfindungsgemäßen organischen Moleküle eine Struktur der Formel VIIIg auf oder bestehen daraus:

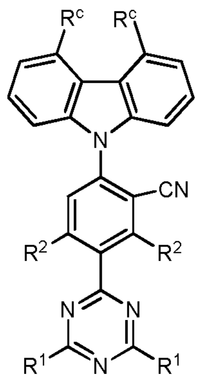

Formel VIIIg

wobei die oben genannten Definitionen gelten.

In einer weiteren Ausführungsform weisen die erfindungsgemäßen organischen Moleküle eine Struktur der Formel VIIIh auf oder bestehen daraus:

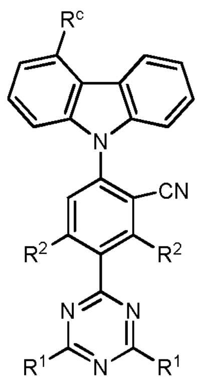

Formel VIIIh

wobei die oben genannten Definitionen gelten.

In einer Ausführungsform weisen die erfindungsgemäßen organischen Moleküle eine Struktur der Formel IX auf oder bestehen daraus:

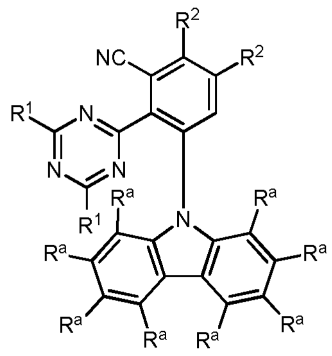

Formel IX

wobei die oben genannten Definitionen gelten.

In einer weiteren Ausführungsform weisen die erfindungsgemäßen organischen Moleküle eine Struktur der Formel IXa auf oder bestehen daraus:

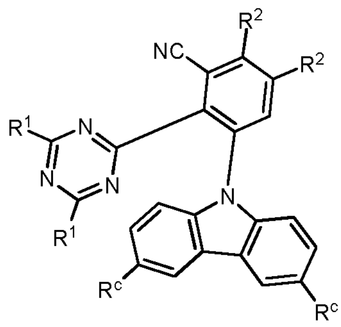

Formel IXa

wobei die oben genannten Definitionen gelten.

In einer weiteren Ausführungsform weisen die erfindungsgemäßen organischen Moleküle eine Struktur der Formel IXb auf oder bestehen daraus:

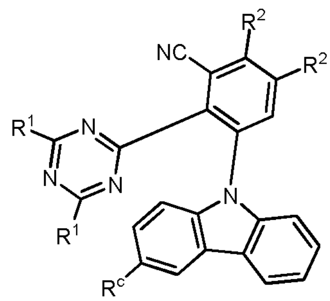

Formel IXb

wobei die oben genannten Definitionen gelten.

In einer weiteren Ausführungsform weisen die erfindungsgemäßen organischen Moleküle eine Struktur der Formel IXc auf oder bestehen daraus:

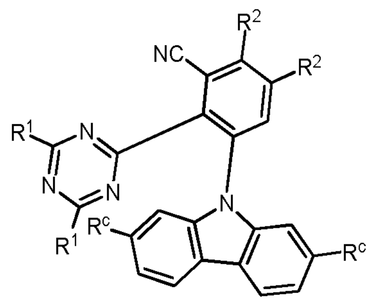

Formel IXc

wobei die oben genannten Definitionen gelten.

In einer weiteren Ausführungsform weisen die erfindungsgemäßen organischen Moleküle eine Struktur der Formel IXd auf oder bestehen daraus:

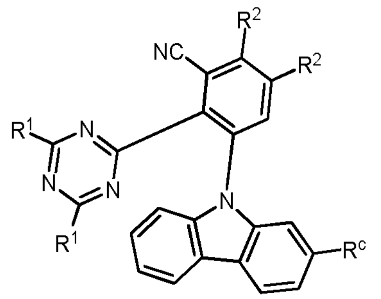

Formel IXd

wobei die oben genannten Definitionen gelten.

In einer weiteren Ausführungsform weisen die erfindungsgemäßen organischen Moleküle eine Struktur der Formel IXe auf oder bestehen daraus:

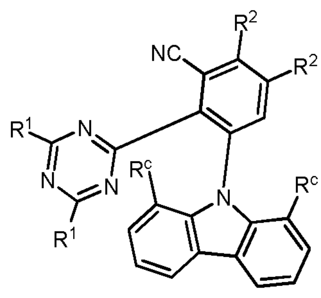

Formel IXe

wobei die oben genannten Definitionen gelten.

In einer weiteren Ausführungsform weisen die erfindungsgemäßen organischen Moleküle eine Struktur der Formel IXf auf oder bestehen aus dieser Struktur:

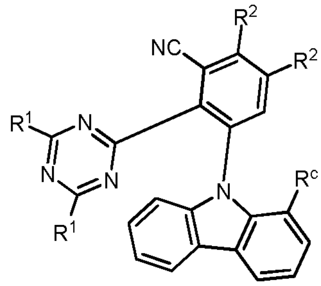

Formel IXf

wobei die oben genannten Definitionen gelten.

In einer weiteren Ausführungsform weisen die erfindungsgemäßen organischen Moleküle eine Struktur der Formel IXg auf oder bestehen daraus:

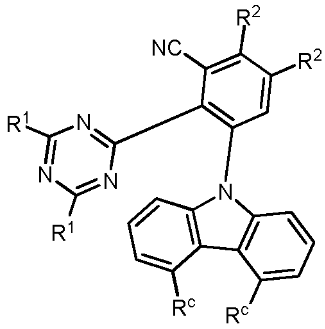

Formel IXg

wobei die oben genannten Definitionen gelten.

In einer weiteren Ausführungsform weisen die erfindungsgemäßen organischen Moleküle eine Struktur der Formel IXh auf oder bestehen aus dieser Struktur:

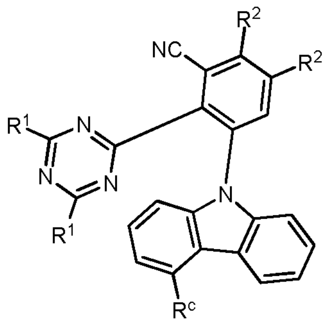

Formel IXh

wobei die oben genannten Definitionen gelten.

In einer weiteren Ausführungsform der erfindungsgemäßen organischen Moleküle ist $R^c$ bei jedem Auftreten unabhängig voneinander ausgewählt aus der Gruppe bestehend aus Me, $^i$Pr, $^t$Bu, CN, $CF_3$, Ph, das jeweils durch einen oder mehrere Reste ausgewählt aus Me, $^i$Pr, $^t$Bu, CN, $CF_3$ oder Ph substituiert sein kann, Pyridinyl, das jeweils durch einen oder mehrere Reste ausgewählt aus Me, $^i$Pr, $^t$Bu, CN, $CF_3$ oder Ph substituiert sein kann, Pyrimidinyl, das jeweils durch einen oder mehrere Reste ausgewählt aus Me, $^i$Pr, $^t$Bu, CN, $CF_3$ oder Ph substituiert sein kann und Triazinyl, das jeweils durch einen oder mehrere Reste ausgewählt aus Me, $^i$Pr, $^t$Bu, CN, $CF_3$ oder Ph substituiert sein kann.

In einer weiteren Ausführungsform ist $R^c$ bei jedem Auftreten unabhängig voneinander ausgewählt aus der Gruppe bestehend aus Me, $^t$Bu, CN, $CF_3$, Ph, das jeweils durch einen oder mehrere Reste ausgewählt aus Me, $^i$Pr, $^t$Bu, CN, $CF_3$ oder Ph substituiert sein kann, und Triazinyl, das jeweils durch einen oder mehrere Reste ausgewählt aus Me, $^i$Pr, $^t$Bu, CN, $CF_3$ oder Ph substituiert sein kann.

In einer weiteren Ausführungsform ist $R^c$ bei jedem Auftreten unabhängig voneinander ausgewählt aus der Gruppe bestehend aus Me, $^t$Bu, CN, $CF_3$ und Ph, das jeweils durch einen oder mehrere Reste ausgewählt aus Me, $^i$Pr, $^t$Bu, CN, $CF_3$ oder Ph substituiert sein kann.

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, 1,3,5-Triazin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Eine Ausführungsform der Erfindung betrifft organische Moleküle, welche einen $\Delta E(S_1-T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen und/oder eine Emissionslebensdauer von höchstens 150 $\mu$s, insbesondere von höchstens 100 $\mu$s, von höchsten 50 $\mu$s, oder von höchstens 10 $\mu$s aufweisen und/oder eine Hauptemissionsbande mit einer Halbwertsbreite kleiner als 0,55 eV, insbesondere kleiner als 0,50 eV, kleiner als 0,48 eV, oder kleiner als 0,45 eV aufweisen.

Insbesondere weisen die organischen Moleküle ein Emissionsmaximum bei zwischen 420 und 500 nm, zwischen 430 und 480 nm, oder zwischen 450 und 470 nm auf.

Insbesondere weisen die Moleküle einen "blue material index" (BMI), den Quotienten aus der PLQY (in %) und ihrer $CIE_y$-Farbkoordinate des von dem erfindungsgemäßen Molekül emittierten Lichts, von größer 150, insbesondere von größer 200, von größer 250 oder von größer 300 auf.

In einem weiteren Aspekt betrifft die Erfindung ein Verfahren zur Herstellung eines erfindungsgemäßen organischen Moleküls der hier beschriebenen Art (mit einer eventuellen Folgeumsetzung), wobei ein in 4- und 6-Position $R^1$-substituiertes 2-Halogen-1,3,5-triazin als Edukt eingesetzt wird. Erfindungsgemäße 2-Halogen-1,3,5-triazine sind 2-Chlor-1,3,5-triazin, 2-Brom-1,3,5-triazin und 2-Iod-1,3,5-triazin.

In einer Ausführungsform wird 2-Chlor-4,6-diphenyl-1,3,5-triazin, 2-Brom-4,6-diphenyl-1,3,5-triazin, 2-Iod-4,6-diphenyl-1,3,5-triazin, 2-Chlor-4,6-dimethyl-1,3,5-triazin, 2-Brom-4,6-dimethyl-1,3,5-triazin oder 2-Iod-4,6-dimethyl-1,3,5-triazin als Edukt eingesetzt.

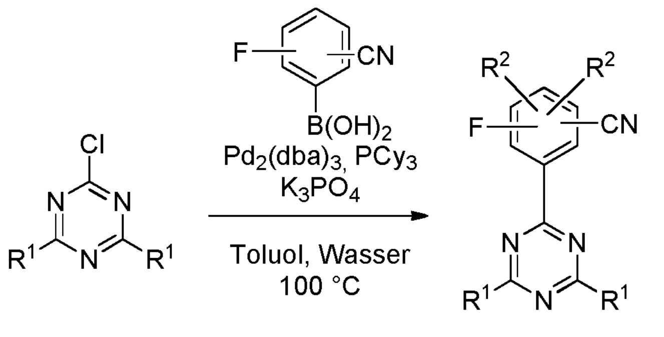


E1

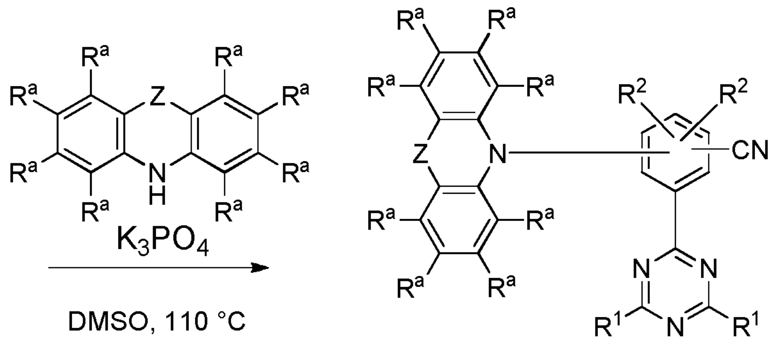


In einer Ausführungsform wird in 4- und 6-Position $R^1$-substituiertes 2-Halogen-1,3,5-triazin als Edukt mit einer Fluorcyanophenylboronsäure oder einem entsprechenden Fluorcyanophenylboronsäureester in einer Palladium-katalysierten Kreuzkupplungsreaktion umgesetzt. Hierbei können erfindungsgemäß beispielhaft 2-Fluor-4-cyanophenylboronsäure, 2-Fluor-5-cyanophenylboronsäure, 2-Fluor-6-cyanophenylboronsäure, 3-Fluor-4-cyano-phenylboronsäure, 3-Fluor-5-cyanophenylboronsäure, 3-Fluor-6-cyanophenylboronsäure, 4-Fluor-3-cyanophenylboronsäure und 4-Fluor-2-cyanophenylboronsäure eingesetzt werden. Das Produkt wird durch Deprotonierung des entsprechenden Amins und anschließender nukleophiler Substitution der Fluorgruppe erhalten. Hierbei wird ein Stickstoffheterozyklus im Sinne einer nukleophilen aromatischen Substitution mit einem Edukt E1 umgesetzt. Typische Bedingungen beinhalten die Verwendung einer Base wie beispielweise tribasisches Kaliumphosphat oder Natriumhydrid in einem aprotischen polaren Lösungsmittel wie beispielweise Dimetylsulfoxid (DMSO) oder N,N-Dimethylformamid (DMF).

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und
(b) mindestens ein, d. h. ein oder mehrere Emitter- und/oder Hostmaterialien, die von dem erfindungsgemäßen

organischen Molekül verschiedenen ist bzw. sind und
(c) optional einem oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.
In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.
Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.
Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.
Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)
6. Emissionsschicht bzw. Emitterschicht (emitting layer, EML)
7. Elektronenblockierschicht (electron blocking layer, EBL)
8. Lochtransportschicht (hole transport layer, HTL)
9. Lochinjektionsschicht (hole injection layer, HIL)
10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen. Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Desweiteren können kleine Moleküle verwendet werden (z. B. Kupfer-Phthalocyanin (CuPc, z. B. 10 nm dick)) oder Metalloxide wie etwa $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N*,*N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis 100 nm.

Die HTL kann eine p-dotierte Schicht aufweisen, die einen anorganischen oder organischen Dotierstoff in einer

organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan), 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol, 9-[3,5-Bis(2-dibenzothiophenyl)phenyl]-9H-carbazol, T2T (2,4,6-Tris(biphenyl-3-yl)-1,3,5-triazin), T3T (2,4,6-Tris(triphenyl-3-yl)-1,3,5-triazin) TST (2,4,6-Tris(9,9'-spirobifluorene-2-yl)-1,3,5-triazin) und/oderDPEPO (Bis[2-((oxo)diphenylphosphino)phenyl]ether). In einer Ausführungsform enthält die EML 50-80 Gewichts-%, bevorzugt 60-75 Gewichts-% eines Hostmaterials ausgewählt aus der Gruppe bestehend aus CBP, mCP, mCBP, 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol und 9-[3,5-bis(2-Dibenzothiophenyl)phenyl]-9H-carbazol; 5-45 Gewichts-%, bevorzugt 10-30 Gewichts-% T2T und 5-40 Gewichts-%, bevorzugt 10-30 Gewichts-% einer erfindungsgemäßen organischen Moleküls als Emitter. Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), NBphen (2,9-Bis(naphthalen-2-yl)-4,7-diphenyl-1,10-phenanthrolin), Alq3 (Aluminium-tris(8-hydroxychinolin)), T2T, TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, NBphen, 1,3,5-tri(1-phenyl-1H-benzo[d]imidazol-2-yl)phenyl (TPBi), BPyTP2 (2,7-Di(2,2'-bipyridin-5-yl)triphenyl), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Eine Ausführungsform der Erfindung betrifft organische optoelektronische Vorrichtungen, welche eine externe Quanteneffizienz (EQE) bei 1000 cd/$m^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/$m^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht

ist.

Die lichtemittierende Schicht kann in einer Ausführungsform ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierender Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)- und Singulett ($S_1$)-Energieniveaus energetisch höher liegen als die Triplett ($T_1$)- und Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierender Schicht aufgebracht ist, und die lichtemittierende Schicht zwischen löcher- und elektronentransportierender Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED (AMOLED) Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

## Beispiele

Allgemeines Syntheseschema I:

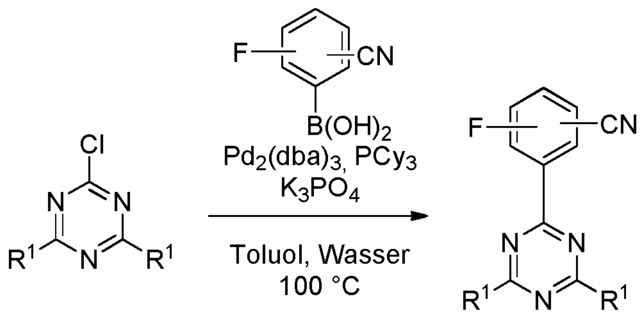


**E1**

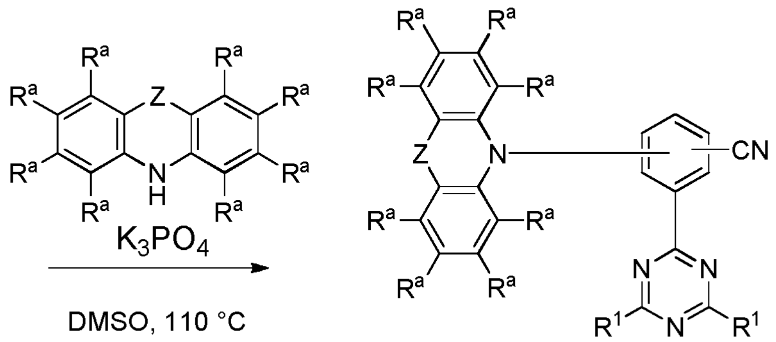


*Allgemeine Synthesevorschrift **AAV1:***

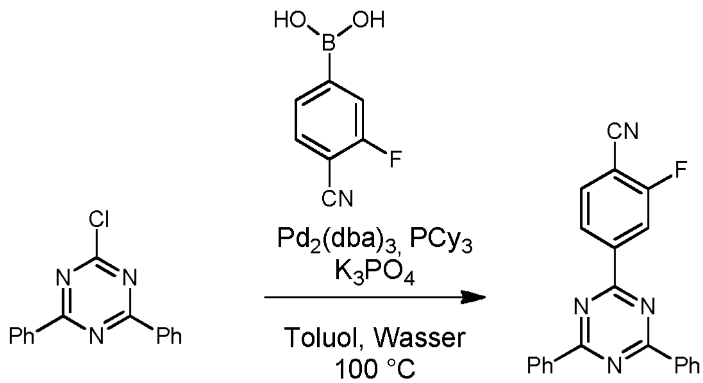


**Z1**

2-Chlor-4,6-diphenyl-1,3,5-triazin (1,00 Äquivalente), 3-Fluor-4-cyanophenylboronsäure (1,20 Äquivalente), $Pd_2(dba)_3$ (dba = Dibenzylideneaceton) (0,03 Äquivalente), Tricyclohexylphosphin ($PCy_3$) (0,07 Äquivalente) und tribasisches Kaliumphosphat (1,70 Äquivalente) werden unter Stickstoff in einem Dioxan/Toluol/Wasser-Gemisch (Verhältnis 10:3:2) bei 100 °C für 20 h gerührt. Anschließend wird die Reaktionsmischung in 200 mL gesättigte Natriumchlorid-Lösung gegeben und mit Ethylacetat (2 x 200 mL) extrahiert. Die vereinigten organischen Phasen werden mit gesättigte Natriumchlorid-Lösung gewaschen, über MgSO4 getrocknet und das Lösemittel entfernt. Das erhaltene Rohprodukt wird durch Flashchromatographie gereinigt und das Produkt als Feststoff erhalten.

Erfindungsgemäß kann statt einer Boronsäure auch ein entsprechender Boronsäureester verwendet werden.

*Allgemeine Synthesevorschrift **AAV2**:*

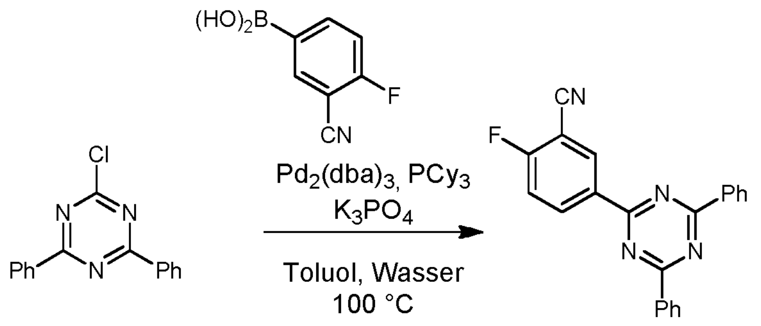


**Z2**

Die Synthese von **Z2** erfolgt analog ***AAV1***, wobei 2-Chlor-4,6-diphenyl-1,3,5-triazin mit 4-Fluor-3-cyanophenylboronsäure umgesetzt wird.

*Allgemeine Synthesevorschrift* ***AAV3*:**

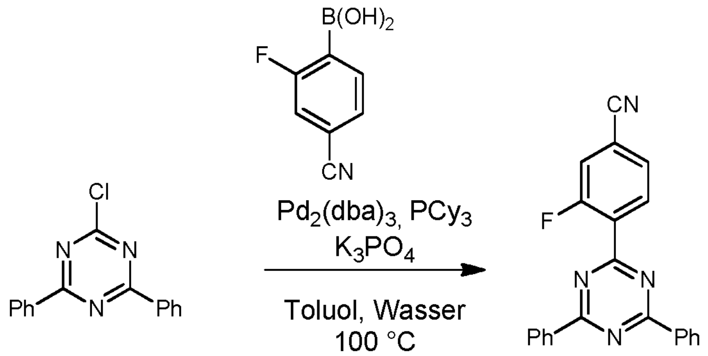


**Z3**

Die Synthese von **Z3** erfolgt analog ***AAV1***, wobei 2-Chlor-4,6-diphenyl-1,3,5-triazin mit 2-Fluor-4-cyanophenylboronsäure umgesetzt wird.

*Allgemeine Synthesevorschrift* ***AAV4*:**

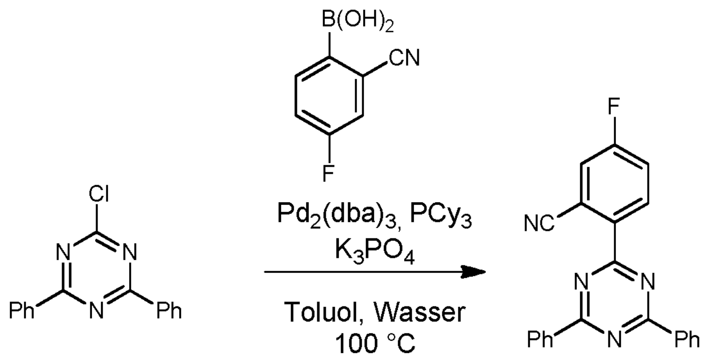


**Z4**

Die Synthese von **Z4** erfolgt analog ***AAV1***, wobei 2-Chlor-4,6-diphenyl-1,3,5-triazin mit 4-Fluor-2-cyanophenylboronsäure umgesetzt wird.

*Allgemeine Synthesevorschrift* ***AAV5*****:**

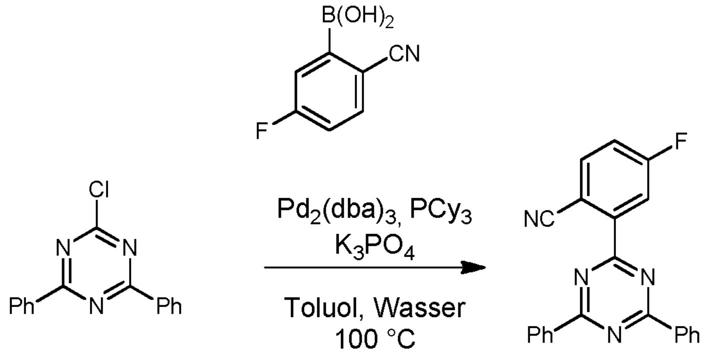


**Z5**

Die Synthese von **Z5** erfolgt analog ***AAV1***, wobei 2-Chlor-4,6-diphenyl-1,3,5-triazin mit 3-Fluor-6-cyanophenylboronsäure umgesetzt wird.

*Allgemeine Synthesevorschrift* ***AAV6*****:**

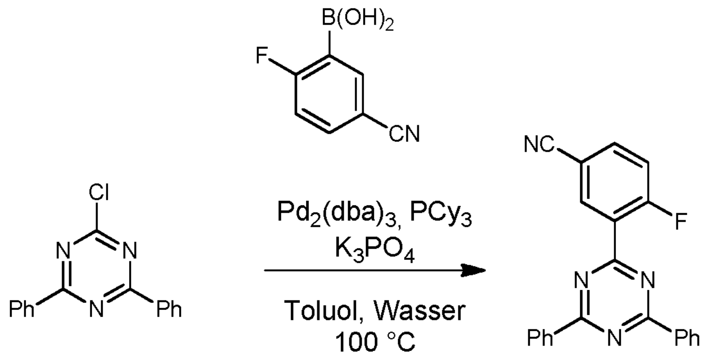


**Z6**

Die Synthese von **Z6** erfolgt analog ***AAV1***, wobei 2-Chlor-4,6-diphenyl-1,3,5-triazin mit 2-Fluor-5-cyanophenylboronsäure umgesetzt wird.

*Allgemeine Synthesevorschrift* ***AAV7:***

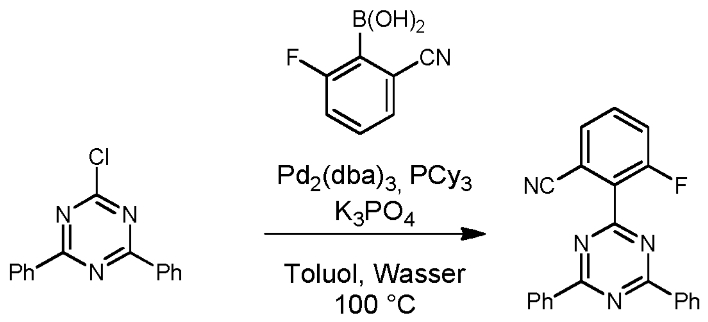


**Z7**

Die Synthese von **Z7** erfolgt analog ***AAV1***, wobei 2-Chlor-4,6-diphenyl-1,3,5-triazin mit 2-Fluor-6-cyanophenylboronsäure umgesetzt wird.

*Allgemeine Synthesevorschrift* ***AAV8*****:**

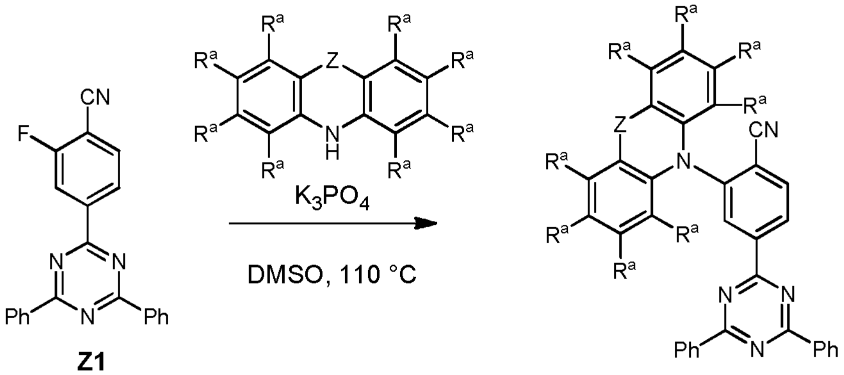


**Z1**

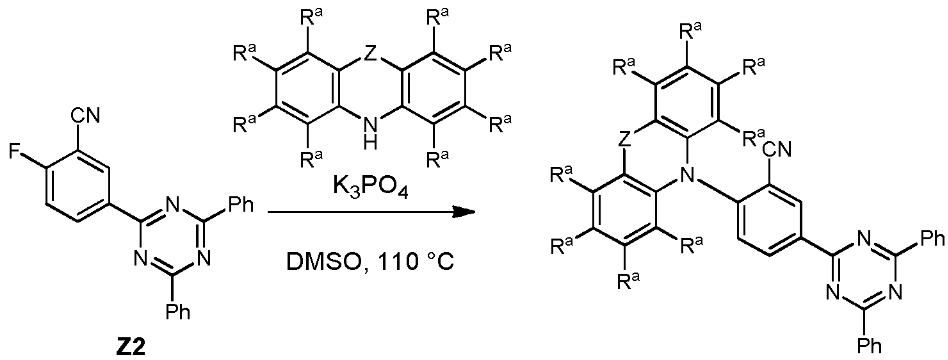


**Z2**

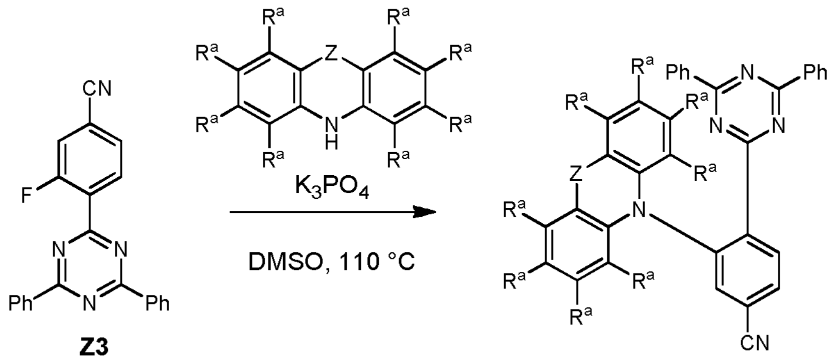


**Z3**

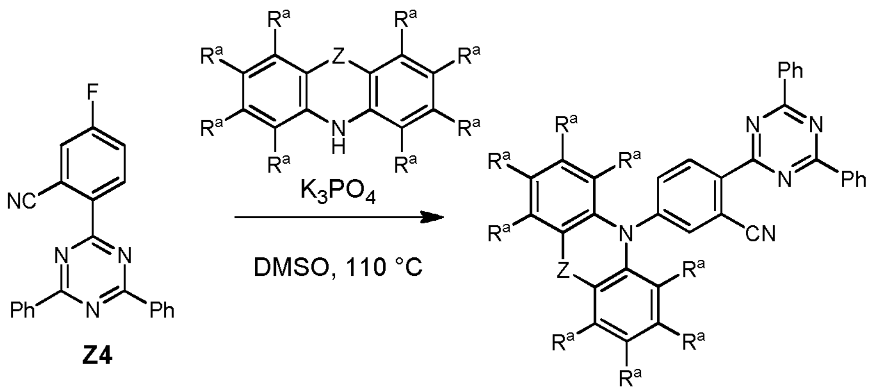


Z4

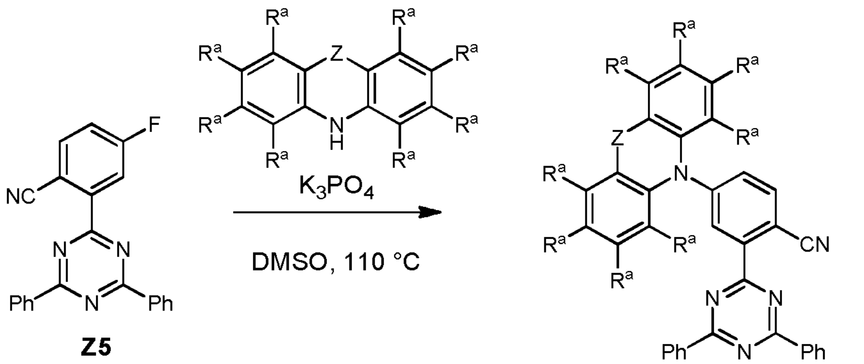


Z5

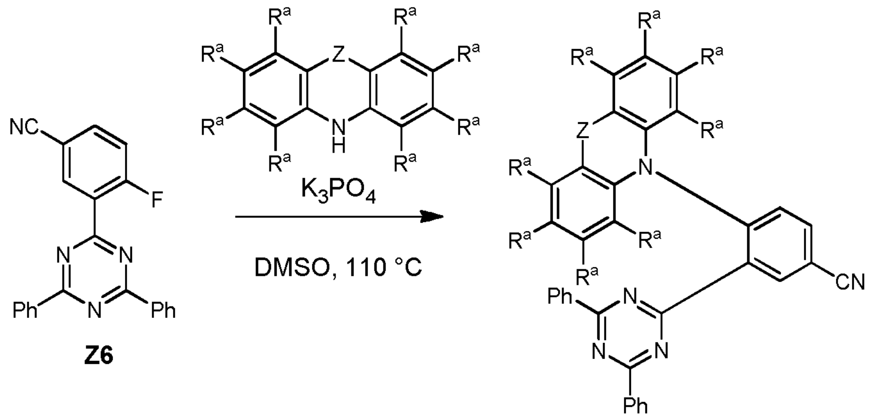


Z6

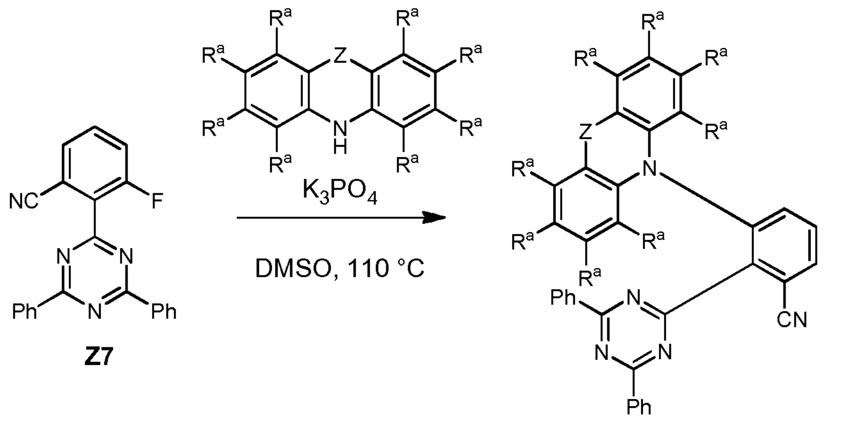


Z7

**Z1**, **Z2**, **Z3**, **Z4**, **Z5**, **Z6** oder **Z7** (jeweils 1,00 Äquivalent), das entsprechende Donor-Molekül D-H (1,00 Äquivalent) und

tribasisches Kaliumphosphat (2,00 Äquivalente) werden unter Stickstoff in DMSO suspendiert und bei 110 °C gerührt (16 h). Anschließend wird die Reaktionsmischung in gesättigte Natriumchlorid-Lösung gegeben und dreimal mit Dichlormethan extrahiert. Die vereinigten organischen Phasen werden zweimal mit gesättigter Natriumchlorid-Lösung gewaschen, getrocknet über Magnesiumsulfat und das Lösemittel anschließend entfernt. Das Rohprodukt wurde schließlich durch Umkristallisation aus Toluol oder durch Flashchromatographie gereinigt. Das Produkt wird als Feststoff erhalten.

Um die entsprechenden $R^1$ substituierten Verbindungen zu erhalten, wird statt 2-Chlor-4,6-diphenyl-1,3,5-triazin das entsprechende in 4- und 6-Position $R^1$-substituierte 2-Chlor-1,3,5-triazin eingesetzt, beispielsweise 2-Chlor-4,6-methyl-1,3,5-triazin.

Im speziellen entspricht D-H einem 3,6-substituierten Carbazol (z. B. 3,6-Dimethylcarbazol, 3,6-Diphenylcarbazol, 3,6-Di-tert-butylcarbazol), einem 2,7-substituierten Carbazol (z. B. 2,7-Dimethylcarbazol, 2,7-Diphenylcarbazol, 2,7-Di-tert-butylcarbazol), einem 1,8-substituierten Carbazol (z. B. 1,8-Dimethylcarbazol, 1,8-Diphenylcarbazol, 1,8-Di-tert-butylcarbazol), einem 1-substituierten Carbazol (z. B. 1-Methylcarbazol, 1-Phenylcarbazol, 1-tert-Butylcarbazol), einem 2-substituierten Carbazol (z. B. 2-Methylcarbazol, 2-Phenylcarbazol, 2-tert-Butylcarbazol) oder einem 3-substituierten Carbazol (z. B. 3-Methylcarbazol, 3-Phenylcarbazol, 3-tert-Butylcarbazol). Insbesondere kann ein Halogencarbazol, insbesondere 3-Bromcarbazol oder 3,6-Dibromcarbazol, als D-H eingesetzt werden, welches in einer Folgereaktion beispielsweise in eine entsprechende Boronsäure, beispielsweise (Carbazol-3-yl)boronsäure, oder in einen entsprechenden Boronsäureester, beispielsweise (Carbazol-3-yl)boronsäureester, beispielhaft durch Reaktion mit Bis(pinacol)boronsäureester (CAS-Nr. 73183-34-3), umgesetzt wird. In einer Folgereaktion können ein oder mehrere Reste $R^a$, welche als halogeniertes Edukt $R^a$-Hal, bevorzugt $R^a$-Cl und $R^a$-Br, eingesetzt werden, über eine Kupplungsreaktion an Stelle der Boronsäuregruppe oder der Boronsäureestergruppe eingeführt werden. Alternativ können ein oder mehrere Reste $R^a$ durch Reaktion des zuvor eingeführten Halogencarbazols mit Boronsäuren des Restes $R^a$ ($R^a$-$B(OH)_2$) oder entsprechenden Boronsäureestern eingeführt werden.

**Photophysikalische Messungen**

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

*Probenvorbereitung, Film: Spin-Coating*

Gerät: Spin150, SPS euro.
Die Probenkonzentration entsprach 10 mg/ml, angesetzt in Toluol oder Chlorbenzol. Programm: 1) 3 s bei 400 U/min; 2) 20 s bei 1000 U/min bei 1000 Upm/ s. 3) 10 s bei 4000 U/min bei 1000 Upm/s. Die Filme wurden nach dem Beschichten für 1 min bei 70 °C an Luft auf einer Präzisionsheizplatte von LHG getrocknet.

Photolumineszenzspektroskopie und TCSPC

Steady-state Emissionsspektroskopie wurde mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" (*Time-correlated single-photon counting,* TCSPC)-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen:

NanoLED 370 (Wellenlänge: 371 nm, Pulsdauer: 1,1 ns)
NanoLED 290 (Wellenlänge: 294 nm, Pulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: gemessenen Größe.

Quanteneffizienzbestimmung

Die Messung der Photolumineszenzquantenausbeute (PLQY) erfolgte mittels eines *Absolute PL Quantum Yield Measurement* C9920-03G-Systems der *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 $\mu$m) verbunden ist. Die Auswertung der Quanteneffizienz und der CIE-Koordinaten erfolgte mit Hilfe der Software U6039-05 Version 3.6.0.
Das Emissionsmaximum wird in nm, die Quantenausbeute $\Phi$ in % und die CIE-Farbkoordinaten als x,y-Werte angegeben.
Die Photolumineszenzquantenausbeute wurde nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung:
Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

Herstellung und Charakterisierung von organischen Elektrolumineszenzvorrichtungen aus der Gasphase

Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.
Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.
Die Werte ergeben sich aus dem Durchschnitt der verschiedenen Pixel einer OLED.

HPLC-MS:

HPLC-MS Spektroskopie wurde mit einer HPLC-Anlage der Firma Agilent (1100er Serie) mit einem angeschlossenen MS-Detektor (Thermo LTQ XL) gemessen. Für die HPLC wurde eine Eclipse Plus C18 Säule von Agilent mit einer Partikelgröße von 3,5 $\mu$m, einer Länge von 150 mm und einem Innendurchmesser von 4,6 mm eingesetzt. Es wurde ohne Vorsäule und bei Raumtemperatur mit den Lösemitteln Acetonitril, Wasser und Tetrahydrofuran in diesen Konzentrationen gearbeitet:

| | | |
|---|---|---|
| Lösemittel A: | $H_2O$ (90%) | MeCN (10%) |
| Lösemittel B: | $H_2O$ (10%) | MeCN (90%) |

(fortgesetzt)

Lösemittel C: THF (100%)

Es wurde mit einem Einspritzvolumen von 15 μL und einer Konzentration von 10 μg/mL mit diesem Gradienten gearbeitet:

| Fluss [ml/min] | Zeit [min] | A[%] | B[%] | C[%] | Druck [bar] |
|---|---|---|---|---|---|
| 0,3 | 0 | 80 | 20 | - | 115 |
| 0,3 | 5 | 80 | 20 | - | 115 |
| 0,3 | 14 | 0 | 90 | 10 | 65 |
| 0,3 | 25 | 0 | 90 | 10 | 65 |
| 0,3 | 26 | 80 | 20 | - | 115 |
| 0,3 | 33 | 80 | 20 | - | 115 |

Die Ionisation der Probe erfolgt durch APCI (atmospheric pressure chemical ionization).

**Beispiel 1**

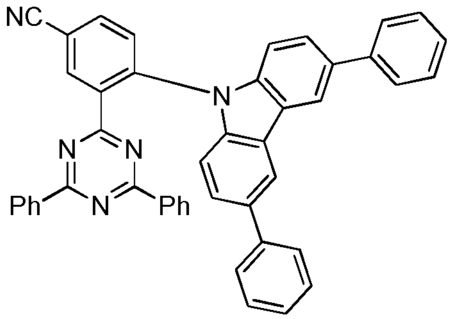

Beispiel 1 wurde nach AAV6 (Ausbeute 43%) und AAV8 hergestellt (Ausbeute 58%). $^1$H-NMR (500 MHz, $CDCl_3$): d = 8.91 (d, 1 H), 8.29 (d, 2 H), 8.15 (dd, 1 H), 8.06 (d, 4 H), 7.95 (d, 1 H), 7.68 (d, 4 H), 7.61 (dd, 2 H), 7.49 (t, 6 H), 7.26-7.38 (m, 8 H) ppm. Figur 1 zeigt das Emissionsspektrum von Beispiel 1 (10 % in PMMA). Das Emissionsmaximum liegt bei 499 nm. Die Photolumineszenzquantenausbeute (PLQY) beträgt 78% und die Halbwertsbreite beträgt 0,43 eV. Die Emissionslebensdauer beträgt 7 μs.

**Beispiel 2**

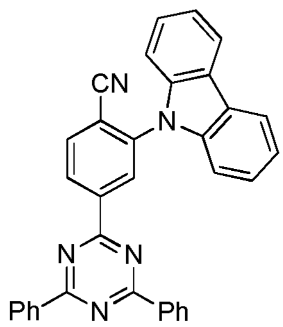

Beispiel 2 wurde nach AAV1 (Ausbeute 37%) und AAV8 hergestellt (Ausbeute 74%). $^1$H-NMR (500 MHz, $CDCl_3$): d = 9.03-9.06 (m, 2 H), 8.75 (d, 4 H), 8.23 (d, 2 H), 8.18 (d, 1 H), 7.64-7.67 (m, 2 H), 7.59 (t, 4 H), 7.50 (t, 2 H),

7.41 (t, 2 H), 7.31 (d, 2 H) ppm.
Figur 2 zeigt das Emissionsspektrum von Beispiel 2 (10 % in PMMA). Das Emissionsmaximum liegt bei 481 nm. Die Photolumineszenzquantenausbeute (PLQY) beträgt 74% und die Halbwertsbreite beträgt 0,46 eV.

**Beispiel 3**

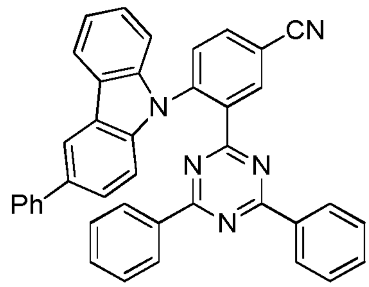

Beispiel 3 wurde nach AAV6 mit einem Boronester (Ausbeute 80%) und AAV8 hergestellt (Ausbeute 37%). $^1$H-NMR (500 MHz, CDCl3): d = 8.89 (m, 1 H), 8.25 (d, 1 H), 8.14-8.19 (m, 2 H), 8.04 (d, 4 H), 7.91 (dd, 1 H), 7.62-7.70 (m, 3 H), 7.48-7.53 (m, 4 H), 7.32-7.39 (m, 6 H), 7.23-7.26 (m, 2 H), 7.17 (d, 1 H), 7.12 (t, 1 H) ppm.
Figur 3 zeigt das Emissionsspektrum von Beispiel 3 (10 % in PMMA). Das Emissionsmaximum liegt bei 489 nm. Die Photolumineszenzquantenausbeute (PLQY) beträgt 72% und die Halbwertsbreite beträgt 0,44 eV. Die Emissionslebensdauer beträgt 8 μs.

**Beispiel 4**

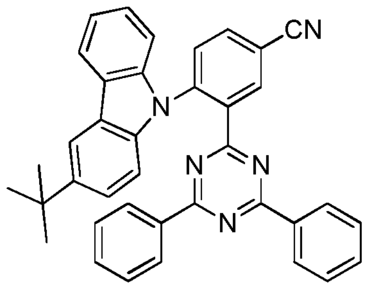

Beispiel **4** wurde nach AAV6 mit einem Boronester (Ausbeute 80%) und AAV8 hergestellt (Ausbeute 35%). $^1$H-NMR (500 MHz, CDCl3): d = 8.88 (d, 1 H), 8.10 (dd, 1 H), 8.00-8.07 (m, 6 H), 7.89 (d, 1 H), 7.52 (t, 2 H), 7.42 (dd, 1 H), 7.36 (t, 4 H), 7.25-7.28 (m, 1 H), 7.16-7.21 (m, 2 H), 7.12 (d, 1 H), 1.43 (s, 9 H) ppm.
Figur 4 zeigt das Emissionsspektrum von Beispiel **4**(10 % in PMMA). Das Emissionsmaximum liegt bei 487 nm. Die Photolumineszenzquantenausbeute (PLQY) beträgt 73% und die Halbwertsbreite beträgt 0,43 eV. Die Emissionslebensdauer beträgt 11 μs.

**Beispiel 5**

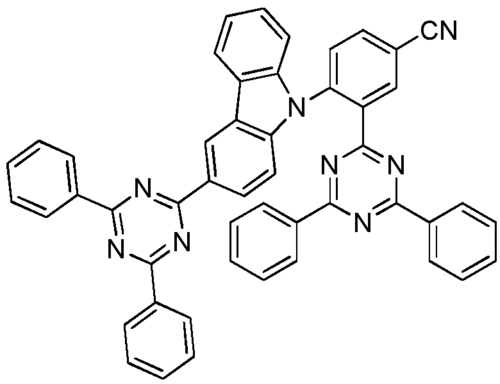

Beispiel **5** wurde nach AAV6 mit einem Boronester (Ausbeute 80%), nach AAV8 mit 3-Bromcarbazol (Ausbeute 80%), anschließender Folgeumsetzung mit Bis(pinacol)boronsäureester und anschließender Folgeumsetzung mit 2-Chlor-4,6-diphenyl-1,3,5-triazin hergestellt.
$^1$H-NMR (500 MHz, CDCl3): d = 9.49 (s, 1 H), 8.95 (d, 1 H), 8.81-8.83 (m, 5 H), 8.27 (d, 1 H), 8.17 (dd, 1 H), 8.04 (d, 4 H), 7.96 (d, 1 H), 7.60-7.67 (m, 6 H), 7.30-7.50 (m, 9 H), 7.25 (d, 1 H) ppm.
Figur 5 zeigt das Emissionsspektrum von Beispiel **5** (10 % in PMMA). Das Emissionsmaximum liegt bei 468 nm. Die Photolumineszenzquantenausbeute (PLQY) beträgt 80% und die Halbwertsbreite beträgt 0,41 eV. Die Emissionslebensdauer beträgt 24 µs.

**Beispiel 6**

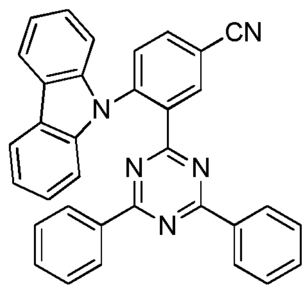

Beispiel 6 wurde nach AAV6 mit einem Boronester (Ausbeute 80%) und AAV8 hergestellt (Ausbeute 69%).
$^1$H-NMR (500 MHz, CDCl3): d = 8.89 (d, 1 H), 8.11 (dd, 1 H), 8.02 (d, 6 H), 7.91 (d, 1 H), 7.50-7.53 (m, 2 H), 7.33-7.37 (m, 6 H), 7.27 (t, 2 H), 7.19 (d, 2 H) ppm.
Figur 6 zeigt das Emissionsspektrum von Beispiel **6** (10 % in PMMA). Das Emissionsmaximum liegt bei 480 nm. Die Photolumineszenzquantenausbeute (PLQY) beträgt 83% und die Halbwertsbreite beträgt 0,43 eV. Die Emissionslebensdauer beträgt 15 µs.

**Beispiel 7**

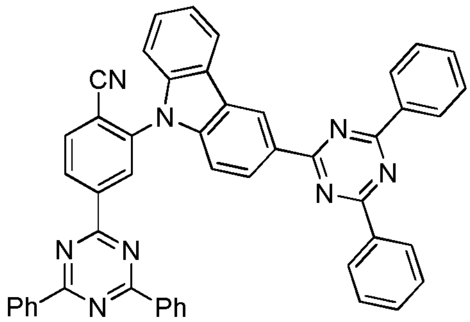

Beispiel 7 wurde nach AAV1 (Ausbeute 37%) und AAV8 (Ausbeute 98%), anschließender Folgeumsetzung mit Bis(pinacol)boronsäureester und anschließender Folgeumsetzung mit 2-Chlor-4,6-diphenyl-1,3,5-triazin hergestellt. $^1$H-NMR (500 MHz, $CDCl_3$): d = 9.68 (s, 1 H), 9.11 (t, 2 H), 8.98 (d, 1 H), 8.86 (d, 4 H), 8.76 (d, 4 H), 8.46 (d, 1 H), 8.22 (d, 1 H), 7.57-7.67 (m, 13 H), 7.51 (t, 1 H), 7.45 (d, 1 H), 7.35 (d, 1 H) ppm.
Figur 7 zeigt das Emissionsspektrum von Beispiel **7** (10 % in PMMA). Das Emissionsmaximum liegt bei 483 nm. Die Photolumineszenzquantenausbeute (PLQY) beträgt 68% und die Halbwertsbreite beträgt 0,48 eV.

**Beispiel 8**

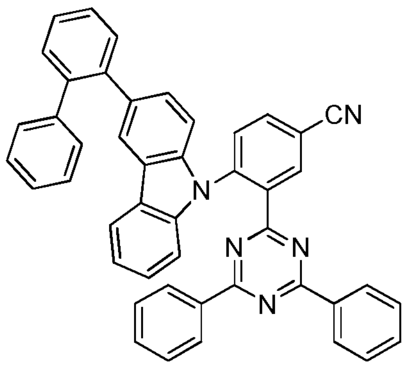

Beispiel **8** wurde nach AAV6 mit einem Boronester (Ausbeute 80%), nach AAV8 mit 3-Bromcarbazol (Ausbeute 65%), und in anschließender Folgeumsetzung mit 2-Biphenylboronsäure hergestellt (Ausbeute 93%).
$^1$H-NMR (500 MHz, CDCl3): d = 8.87 (d, 1 H), 8.08- 8.10 (m, 5 H), 7.99 (d, 1 H), 7.90-7.92 (m, 2 H), 7.52-7.56 (m, 2 H), 7.47-7.49 (m, 1 H), 7.37-7.42 (9 H), 7.30 (s, 1 H), 6.94-6.99 (m, 4 H), 6.86-6.87 (m, 1 H), 6.78 (t, 2 H) ppm.
Figur 8 zeigt das Emissionsspektrum von Beispiel **8** (10 % in PMMA). Das Emissionsmaximum liegt bei 490 nm. Die Photolumineszenzquantenausbeute (PLQY) beträgt 64% und die Halbwertsbreite beträgt 0,44 eV. Die Emissionslebensdauer beträgt 9 µs.

**Beispiel 9**

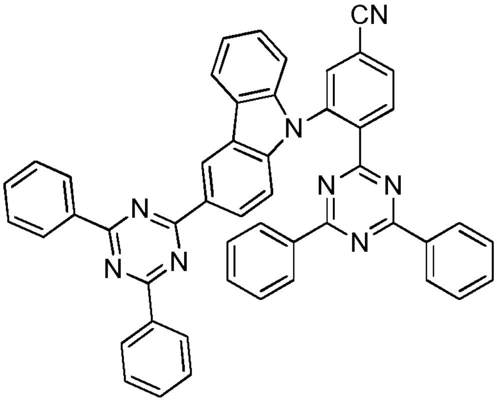

Beispiel **9** wurde nach AAV3 (Ausbeute 79%) nach AAV8 mit 3-Bromcarbazol (Ausbeute 87%), anschließender Folgeumsetzung mit Bis(pinacol)boronsäureester und anschließender Folgeumsetzung mit 2-Chlor-4,6-diphenyl-1,3,5-triazin hergestellt.
$^1$H-NMR (500 MHz, $CDCl_3$): d = 9.48 (s, 1 H), 8.81-8.83 (d, 5 H), 8.72 (d, 1 H), 8.27 (d, 1 H), 8.11-8.14 (m, 2 H), 8.03 (d, 4 H), 7.60-7.65 (m, 6 H), 7.30-7.50 (9 H), 7.25-7.26 (m, 1 H) ppm.
Figur 9 zeigt das Emissionsspektrum von Beispiel 9 (10 % in PMMA). Das Emissionsmaximum liegt bei 495 nm. Die Photolumineszenzquantenausbeute (PLQY) beträgt 71% und die Halbwertsbreite beträgt 0,43 eV. Die Emissionslebensdauer beträgt 20 µs.

**Beispiel 10**

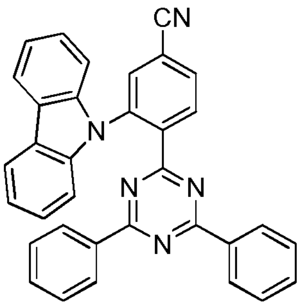

Beispiel **10** wurde nach AAV3 (Ausbeute 79%) und AAV8 hergestellt (Ausbeute 56%). $^1$H-NMR (500 MHz, $CDCl_3$): d = 8.65 (d, 1 H), 8.00-8.08 (m, 8 H), 7.51 (t, 2 H), 7.33-7.37 (m, 6 H), 7.26 (t, 2 H), 7.17 (d, 2 H) ppm.
Figur 10 zeigt das Emissionsspektrum von Beispiel **10** (10% in PMMA). Das Emissionsmaximum liegt bei 501 nm. Die Photolumineszenzquantenausbeute (PLQY) beträgt 68% und die Halbwertsbreite beträgt 0,44 eV. Die Emissionslebensdauer beträgt 18 µs.

**Beispiel 11**

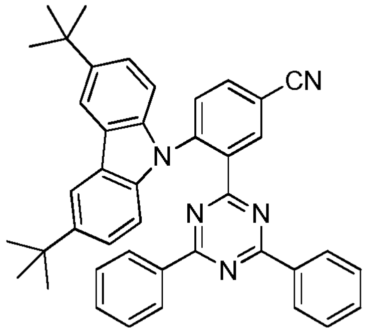

Beispiel **11** wurde nach AAV6 mit einem Boronester (Ausbeute 80%) und AAV8 hergestellt (Ausbeute 73%). MS (HPLC-MS), m/z (Retentionszeit): 611 (10,55 min).
Figur 11 zeigt das Emissionsspektrum von Beispiel **11** (10% in PMMA). Das Emissionsmaximum liegt bei 498 nm. Die Photolumineszenzquantenausbeute (PLQY) beträgt 71% und die Halbwertsbreite beträgt 0,42 eV. Die Emissionslebensdauer beträgt 9 μs.

**Beispiel 12**

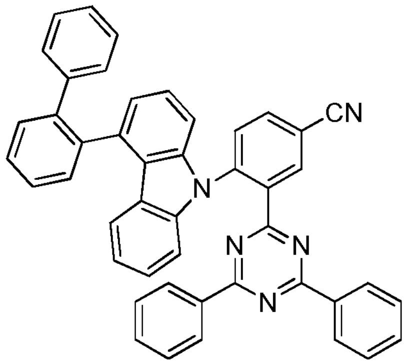

Beispiel **12** wurde nach AAV6 mit einem Boronester (Ausbeute 80%) und AAV8 hergestellt (Ausbeute 91%). MS (HPLC-MS), m/z (Retentionszeit): 651 (9,15 min). Figur 12 zeigt das Emissionsspektrum von Beispiel **12** (10 % in PMMA). Das Emissionsmaximum liegt bei 476 nm. Die Photolumineszenzquantenausbeute (PLQY) beträgt 80% und die Halbwertsbreite beträgt 0,42 eV. Die Emissionslebensdauer beträgt 26 μs.

**Beispiel 13**

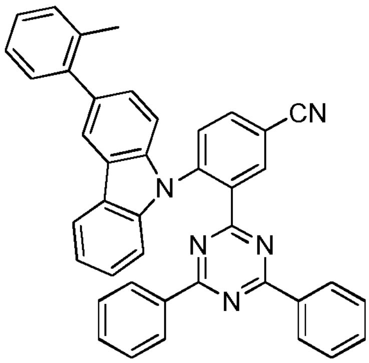

Beispiel **13** wurde nach AAV6 mit einem Boronester (Ausbeute 80%) und AAV8 hergestellt (Ausbeute 44%).

MS (HPLC-MS), m/z (Retentionszeit): 589 (9,14 min). Figur 13 zeigt das Emissionsspektrum von Beispiel **13** (10% in PMMA). Das Emissionsmaximum liegt bei 485 nm. Die Photolumineszenzquantenausbeute (PLQY) beträgt 78% und die Halbwertsbreite beträgt 0,43 eV. Die Emissionslebensdauer beträgt 12 µs.

**Beispiel 14**

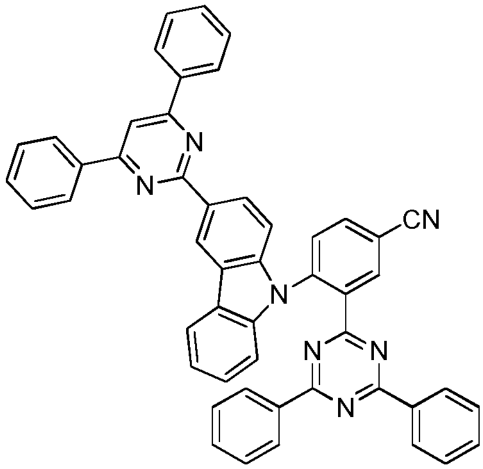

Beispiel **14** wurde nach AAV6 (Ausbeute 80%), nach AAV8 mit 3-Bromcarbazol (Ausbeute 65%), anschließender Folgeumsetzung mit Bis(pinacol)boronsäure und anschließender Folgeumsetzung mit 2-Chlor-4,6-diphenyl-1,3-pyrimidin hergestellt.
MS (HPLC-MS), m/z (Retentionszeit): 729 (10,60 min). Figur 14 zeigt das Emissionsspektrum von Beispiel **14** (10 % in PMMA). Das Emissionsmaximum liegt bei 484 nm. Die Photolumineszenzquantenausbeute (PLQY) beträgt 73% und die Halbwertsbreite beträgt 0,44 eV. Die Emissionslebensdauer beträgt 25 µs.

**Beispiel 15**

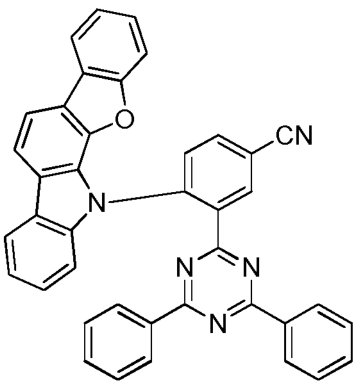

Beispiel **15** wurde nach AAV6 (Ausbeute 80%) und AAV8 (Ausbeute 65%) hergestellt.
MS (HPLC-MS), m/z (Retentionszeit): 589 (9.03 min).
Figur 15 zeigt das Emissionsspektrum von Beispiel **15** (10 % in PMMA). Das Emissionsmaximum liegt bei 472 nm. Die Photolumineszenzquantenausbeute (PLQY) beträgt 63% und die Halbwertsbreite beträgt 0,44 eV. Die Emissionslebensdauer beträgt 5 µs.

**Beispiel 16**

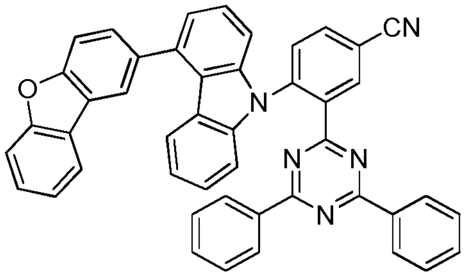

Beispiel **16** wurde nach AAV6 (Ausbeute 80%) und AAV8 (Ausbeute 100%) hergestellt.
MS (HPLC-MS), m/z (Retentionszeit): 666 (10,67 min).
Figur 16 zeigt das Emissionsspektrum von Beispiel **16** (10 % in PMMA). Das Emissionsmaximum liegt bei 479 nm. Die Photolumineszenzquantenausbeute (PLQY) beträgt 81% und die Halbwertsbreite beträgt 0,42 eV. Die Emissionslebensdauer beträgt 6 µs.

**Beispiel 17**

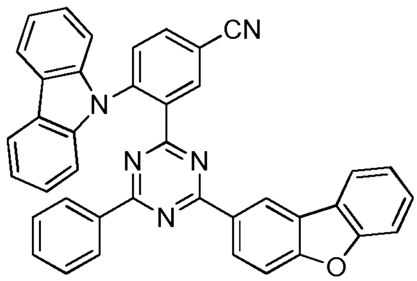

Beispiel **17** wurde nach AAV6, wobei statt 2-Chlor-4,6-diphenyl-1,3,5-triazin 2-Chlor-4-(2-dibenzofuranyl)-6-phenyl-1,3,5-triazin eingesetzt wird (Ausbeute 85%), und AAV8 (Ausbeute 10%) hergestellt.
Figur 17 zeigt das Emissionsspektrum von Beispiel **17** (10 % in PMMA). Das Emissionsmaximum liegt bei 474 nm. Die Photolumineszenzquantenausbeute (PLQY) beträgt 74% und die Halbwertsbreite beträgt 0,42 eV. Die Emissionslebensdauer beträgt 7 µs.

**OLED-Bauteil: Beispiele**

Es wurden beschleunigte Lebensdauermessungen durchgeführt (z. B. durch Anlegen einer erhöhten Stromdichte). Beispielhaft werden LT80 Werte bei 500 cd/m$^2$ nach folgender Gleichung bestimmt:

$$\mathrm{LT80}\left(500\,\frac{cd^2}{m^2}\right) = \mathrm{LT80}(L_0)\left(\frac{500\,\frac{cd^2}{m^2}}{L_0}\right)^{-1.6}$$

wobei $L_0$ der Startleuchtdichte bei der eingesetzten Stromdichte entspricht.

**Beispiele D1 und D2**

Beispiel 5 wurde in den OLED-Bauteilen **D1** und **D2** mit folgenden Aufbauten getestet (der Anteil des erfindungsgemäßen Moleküls an der Emissionsschicht ist in Massenprozent angegeben):

| Schicht | Dicke | D1 |
|---|---|---|
| **10** | 100 nm | Al |

(fortgesetzt)

| Schicht | Dicke | D1 |
|---|---|---|
| **9** | 2 nm | Liq |
| **8** | 30 nm | TPBi |
| **7** | 10 nm | DPEPO |
| **6** | 20 nm | 5 (20%):DPEPO |
| **5** | 10 nm | CzSi |
| **4** | 20 nm | TCTA |
| **3** | 70 nm | NPB |
| **2** | 20 nm | m-MTDATA |
| **1** | 130 nm | ITO |
| **Substrat** | | Glass |

Für das Bauteil **D1** wurde eine externe Quanteneffizienz bei 1000 cd/$m^2$ von 12,7% $\pm$ 0,4 bestimmt. Das Emissionsmaximum liegt bei 479 nm, CIEx wurde mit 0,32 und die CIEy: 0,17 bei 6 V bestimmt.

| Schicht | Dicke | D2 |
|---|---|---|
| **7** | 100 nm | Al |
| **6** | 2 nm | Liq |
| **5** | 40 nm | NBPhen |
| **4** | 20 nm | 5 (10%):mCBP |
| **3** | 10 nm | TCTA |
| **2** | 80 nm | NPB |
| **1** | 130 nm | ITO |
| **Substrat** | | Glass |

Für das Bauteil **D2** wurde eine externe Quanteneffizienz bei 1000 cd/$m^2$ von 8,2% $\pm$ 0,1 und ein LT80-Wert bei 500 cd/$m^2$ von 10 h aus den beschleunigten Lebensdauermessungen bestimmt. Das Emissionsmaximum liegt bei 463 nm, CIEx wurde mit 0,16 und die CIEy: 0,20 bei 6 V bestimmt.

**Beispiele D3, D4, D5, D6 und D7**

Beispiel 3 wurde in den OLED-Bauteilen **D3, D4, D5, D6** und **D7** mit folgenden Aufbauten getestet (der Anteil des erfindungsgemäßen Moleküls an der Emissionsschicht ist in Massenprozent angegeben):

| Schicht | Dicke | D3 | D4 | D5 | D6 |
|---|---|---|---|---|---|
| **8** | 100 nm | Al | Al | Al | Al |
| **7** | 2 nm | Liq | Liq | Liq | Liq |
| **6** | 40 nm | NBPhen | NBPhen | NBPhen | NBPhen |
| **5** | 20 nm | 3 (20%): mCBP | 3 (10%): mCBP | 3 (10%): T2T (15%): mCBP (75%) | 3 (20%): T2T (15%): mCBP (65%) |
| **4** | 5 nm | mCBP | mCBP | mCBP | mCBP |
| **3** | 10 nm | TCTA | TCTA | TCTA | TCTA |

(fortgesetzt)

| Schicht | Dicke | D3 | D4 | D5 | D6 |
|---|---|---|---|---|---|
| **2** | 75 nm | NPB | NPB | NPB | NPB |
| **1** | 130 nm | ITO | ITO | ITO | ITO |
| **Substrat** | | Glass | Glass | Glass | Glass |

Für das Bauteil **D3** wurde eine externe Quanteneffizienz bei 1000 cd/m$^2$ von 14,8% ± 0,1 und ein LT80-Wert bei 500 cd/m$^2$ von 1277 h aus den beschleunigten Lebensdauermessungen bestimmt. Das Emissionsmaximum liegt bei 488 nm, CIEx wurde mit 0,23 und die CIEy: 0,41 bei 6 V bestimmt.
Für das Bauteil **D4** wurde eine externe Quanteneffizienz bei 1000 cd/m$^2$ von 12,5% ± 0,1 und ein LT80-Wert bei 500 cd/m$^2$ von 817 h aus den beschleunigten Lebensdauermessungen bestimmt. Das Emissionsmaximum liegt bei 480 nm, CIEx wurde mit 0,21 und die CIEy: 0,36 bei 6 V bestimmt.
Für das Bauteil **D5** wurde eine externe Quanteneffizienz bei 1000 cd/m$^2$ von 13,9% ± 0,1 und ein LT80-Wert bei 500 cd/m$^2$ von 553 h aus den beschleunigten Lebensdauermessungen bestimmt. Das Emissionsmaximum liegt bei 487 nm, CIEx wurde mit 0,21 und die CIEy: 0,38 bei 6 V bestimmt.
Für das Bauteil **D6** wurde eine externe Quanteneffizienz bei 1000 cd/m$^2$ von 13,3% ± 0,2 und ein LT80-Wert bei 500 cd/m$^2$ von 840 h aus den beschleunigten Lebensdauermessungen bestimmt. Das Emissionsmaximum liegt bei 493 nm, CIEx wurde mit 0,23 und die CIEy: 0,42 bei 6 V bestimmt.

Beispiel **D7:**

| Schicht | Dicke | D7 |
|---|---|---|
| **9** | 100 nm | Al |
| **8** | 2 nm | Liq |
| **7** | 30 nm | NBPhen |
| **6** | 10 nm | T2T |
| **5** | 20 nm | 3 (20%) : mCBP (65%) : T2T (15%) |
| **4** | 5 nm | mCBP |
| **3** | 10nm | TCTA |
| **2** | 75 nm | NPB |
| **1** | 130 nm | ITO |
| **Substrat** | | Glass |

Für das Bauteil **D7** wurde eine externe Quanteneffizienz bei 1000 cd/m$^2$ von 17,2% ± 0,1 und ein LT80-Wert bei 500 cd/m$^2$ von 640 h aus den beschleunigten Lebensdauermessungen bestimmt. Das Emissionsmaximum liegt bei 493 nm, CIEx wurde mit 0,23 und die CIEy: 0,45 bei 6 V bestimmt.

Beispiele **D8** und **D9**

Beispiel **6** wurde in den OLED-Bauteilen **D8** und **D9** mit folgenden Aufbauten getestet (der Anteil des erfindungsgemäßen Moleküls an der Emissionsschicht ist in Massenprozent angegeben):

| Schicht | | Dicke D1 | Dicke D2 |
|---|---|---|---|
| **8** | Al | 100 nm | 100 nm |
| **7** | Liq | 2 nm | 2 nm |

(fortgesetzt)

| Schicht | | Dicke D1 | Dicke D2 |
|---|---|---|---|
| **6** | NBPhen | 40 nm | 40 nm |
| **5** | 6 (20%):mCBP | 50 nm | 60 nm |
| **4** | mCBP | 10nm | 10 nm |
| **3** | TCTA | 10 nm | 10 nm |
| **2** | NPB | 115 nm | 30 nm |
| **1** | ITO | 130 nm | 130 nm |
| **Substrat** | Glass | | |

Für das Bauteil **D8** wurde eine externe Quanteneffizienz bei 1000 cd/m$^2$ von 17,7% $\pm$ 0,2 und ein LT80-Wert bei 500 cd/m$^2$ von 539 h aus den beschleunigten Lebensdauermessungen bestimmt. Das Emissionsmaximum liegt bei 482 nm, CIEx wurde mit 0,17 und die CIEy: 0,34 bei 6 V bestimmt.
Für das Bauteil **D9** wurde eine externe Quanteneffizienz bei 1000 cd/m$^2$ von 14,9% $\pm$ 0,2 und ein LT80-Wert bei 500 cd/m$^2$ von 480 h aus den beschleunigten Lebensdauermessungen bestimmt. Das Emissionsmaximum liegt bei 475 nm, CIEx wurde mit 0,19 und die CIEy: 0,33 bei 6 V bestimmt.

Beispiel **D10**

Beispiel **13** wurde in dem OLED-Bauteil **D10** mit folgendem Aufbau getestet (der Anteil des erfindungsgemäßen Moleküls an der Emissionsschicht ist in Massenprozent angegeben):

| Schicht | Dicke | |
|---|---|---|
| **8** | 100 nm | Al |
| **7** | 2 nm | Liq |
| **6** | 40 nm | NBPhen |
| **5** | 30 nm | **13** (20%):9-[3,5-Bis(2-dibenzofuranyl)phenyl]-9H-carbazol |
| **4** | 8 nm | 9-[3,5-Bis(2-dibenzofuranyl)phenyl]-9H-carbazol |
| **3** | 10 nm | TCTA |
| **2** | 62 nm | NPB |
| **1** | 130 nm | ITO |
| **Substrat** | | Glass |

Für das Bauteil **D10** wurde eine externe Quanteneffizienz bei 1000 cd/m$^2$ von 14,5% $\pm$ 0,8 und ein LT80-Wert bei 500 cd/m$^2$ von 91 h aus den beschleunigten Lebensdauermessungen bestimmt. Das Emissionsmaximum liegt bei 480 nm, CIEx wurde mit 0,20 und die CIEy: 0,37 bei 6 V bestimmt.

Beispiele **D11** und **D12**

Beispiel **5** und Beispiel **6** wurden in den OLED-Bauteilen **D11** und **D12** mit folgenden Aufbauten getestet (der Anteil des erfindungsgemäßen Moleküls an der Emissionsschicht ist in Massenprozent angegeben):

| | | D11 | D12 |
|---|---|---|---|
| **Schicht** | **Dicke** | | |
| **9** | 100 nm | Al | Al |
| **8** | 2 nm | Liq | Liq |

(fortgesetzt)

| | | D11 | D12 |
|---|---|---|---|
| **Schicht** | **Dicke** | | |
| **7** | 30 nm | NBPhen | NBPhen |
| **6** | 40 nm | **5** (30%):mCBP | **6** (30%):mCBP |
| **5** | 10 nm | **5** (10%):mCBP | **6** (10%):mCBP |
| **4** | 10 nm | mCBP | mCBP |
| **3** | 10 nm | TCTA | TCTA |
| **2** | 100 nm | NPB | NPB |
| **1** | 130 nm | ITO | ITO |
| **Substrat** | | Glass | Glass |

Für das Bauteil **D11** wurde eine externe Quanteneffizienz bei 1000 cd/m$^2$ von 15,2% ± 0,1 und ein LT80-Wert bei 500 cd/m$^2$ von 110 h aus den beschleunigten Lebensdauermessungen bestimmt. Das Emissionsmaximum liegt bei 474 nm, CIEx wurde mit 0,15 und die CIEy: 0,24 bei 6 V bestimmt.
Für das Bauteil **D12** wurde eine externe Quanteneffizienz bei 1000 cd/m$^2$ von 16,3% ± 0,1 und ein LT80-Wert bei 500 cd/m$^2$ von 431 h aus den beschleunigten Lebensdauermessungen bestimmt. Das Emissionsmaximum liegt bei 480 nm, CIEx wurde mit 0,17 und die CIEy: 0,33 bei 6 V bestimmt.

**Weitere Beispiele erfindungsgemäßer Moleküle:**

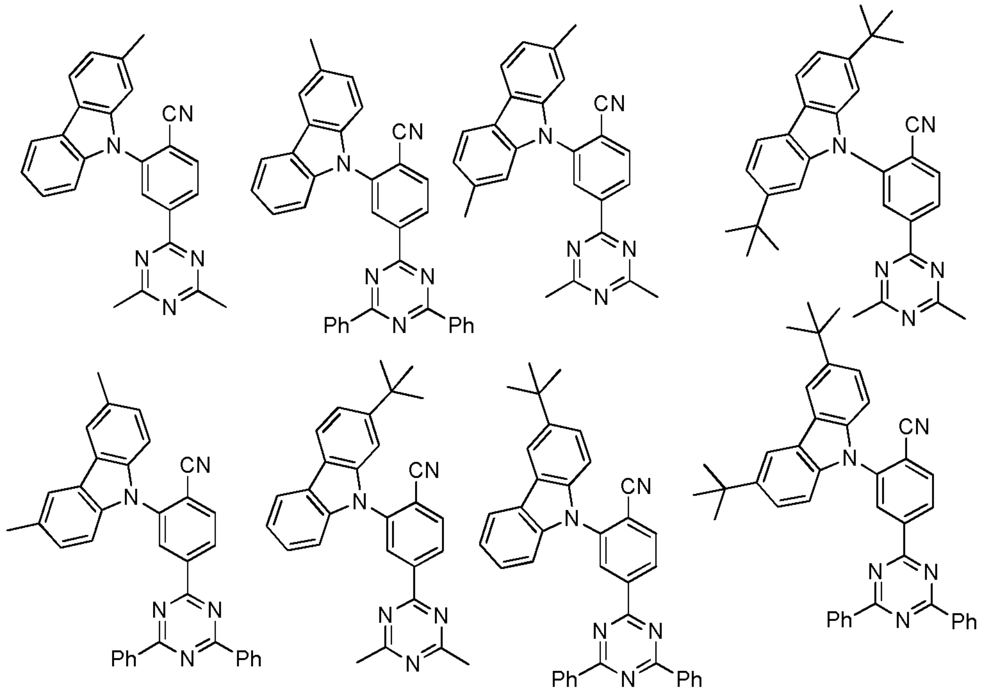

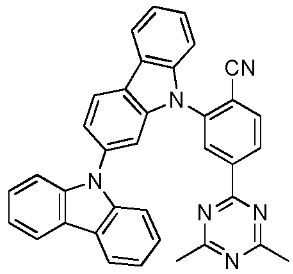 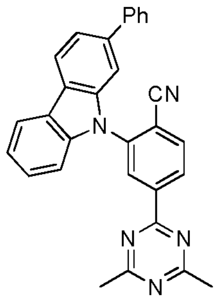 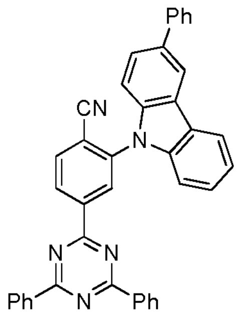 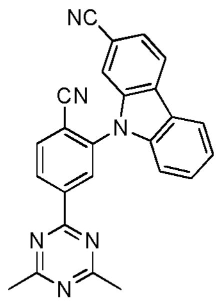

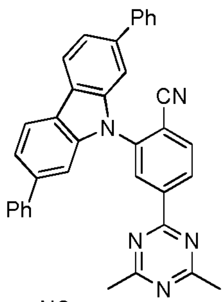 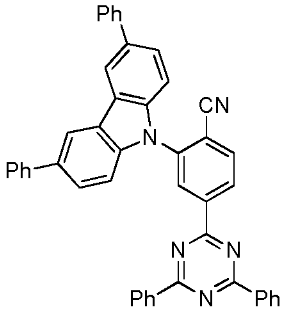 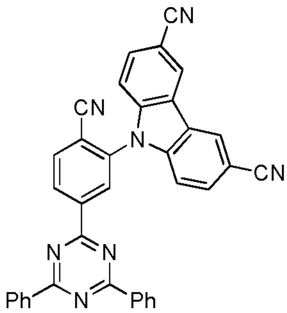 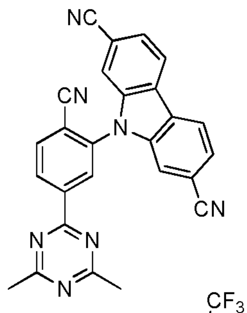

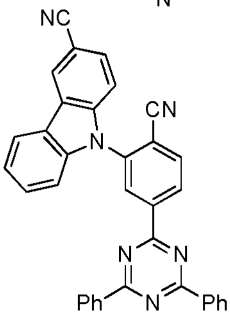 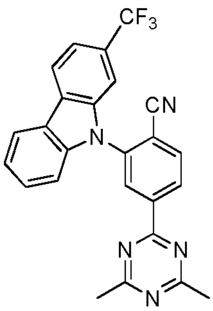 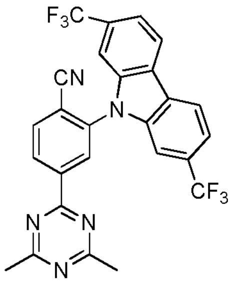 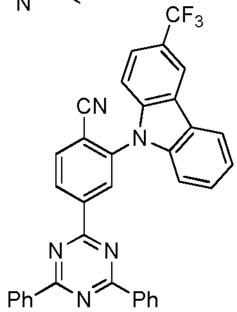

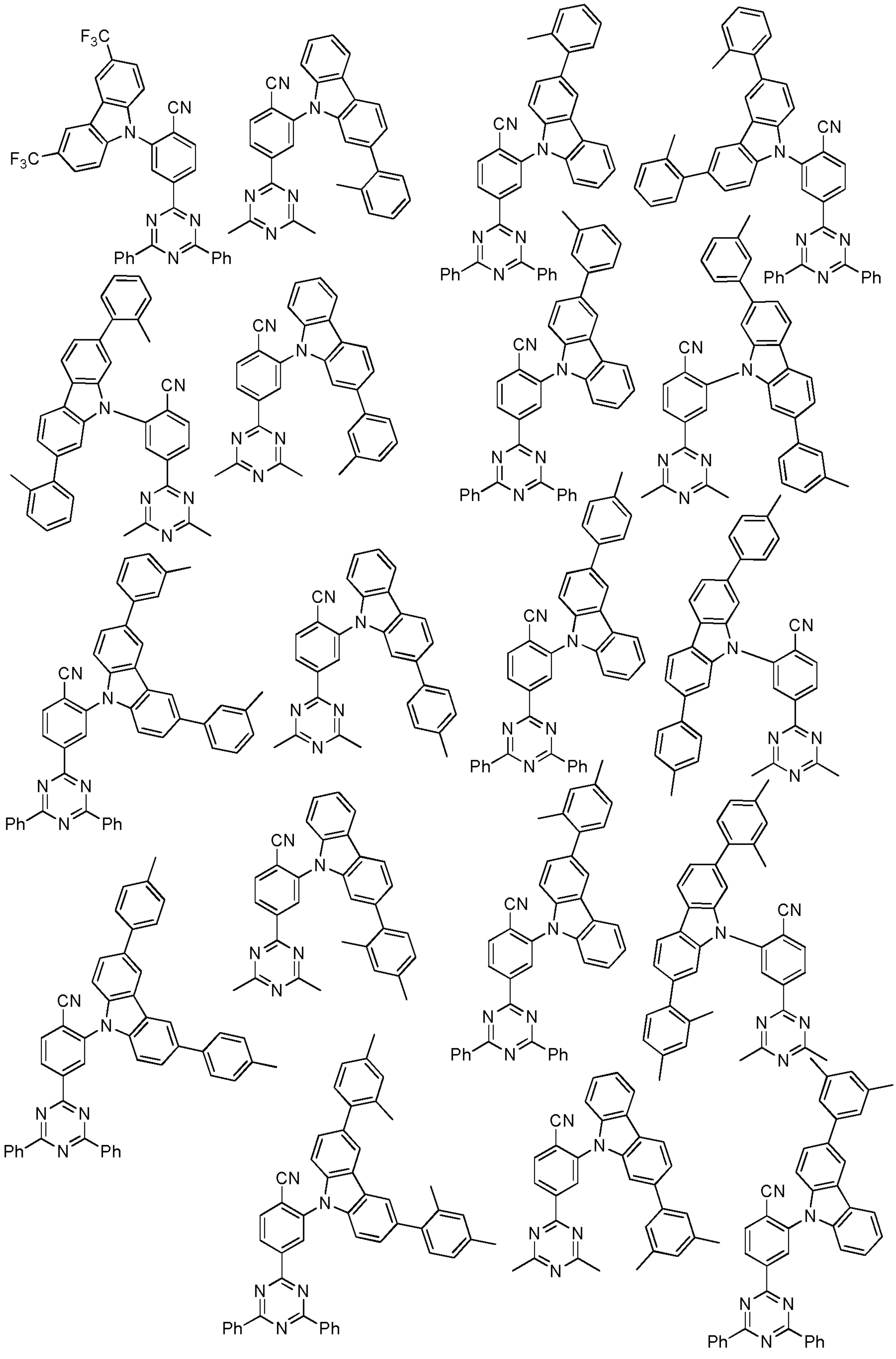

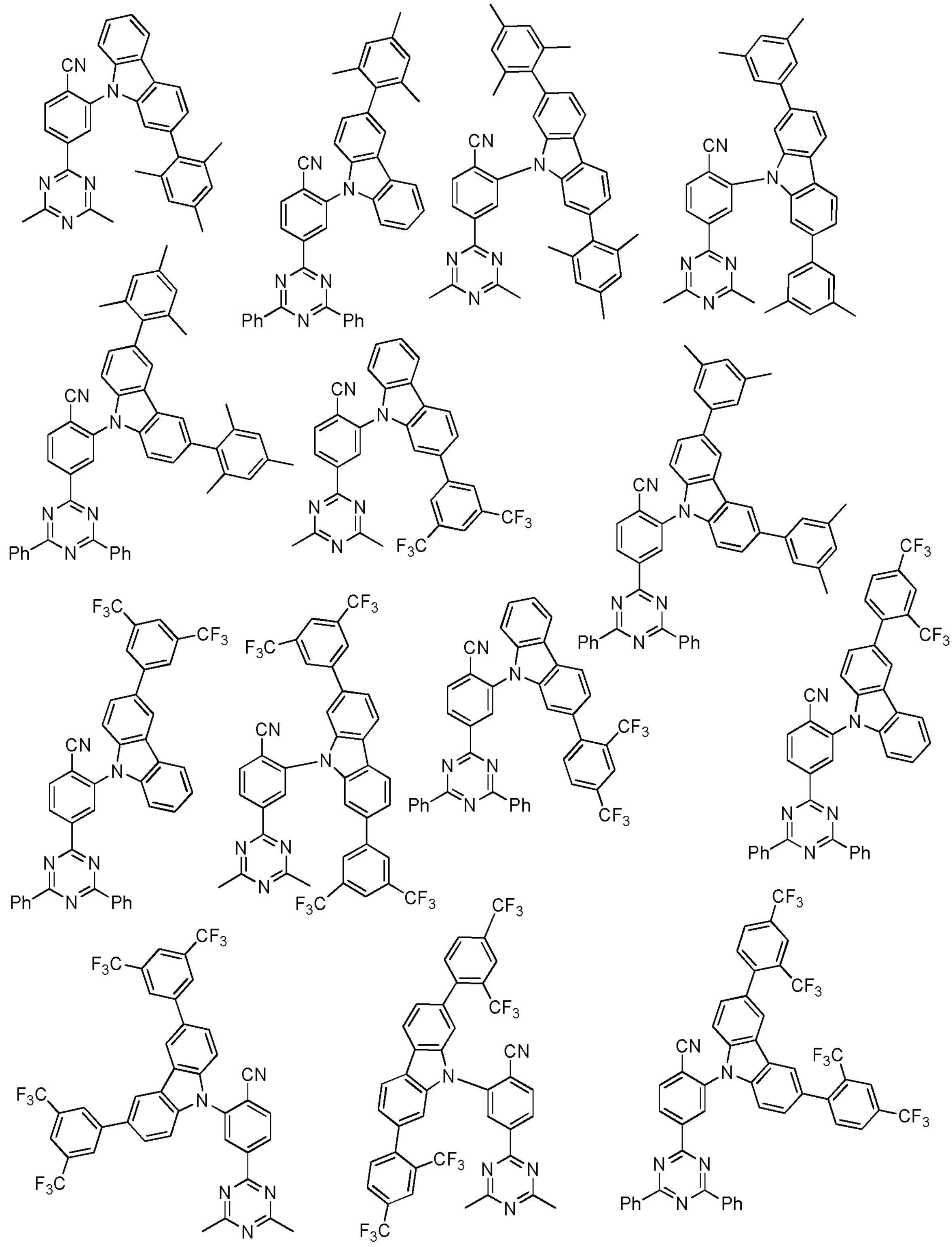

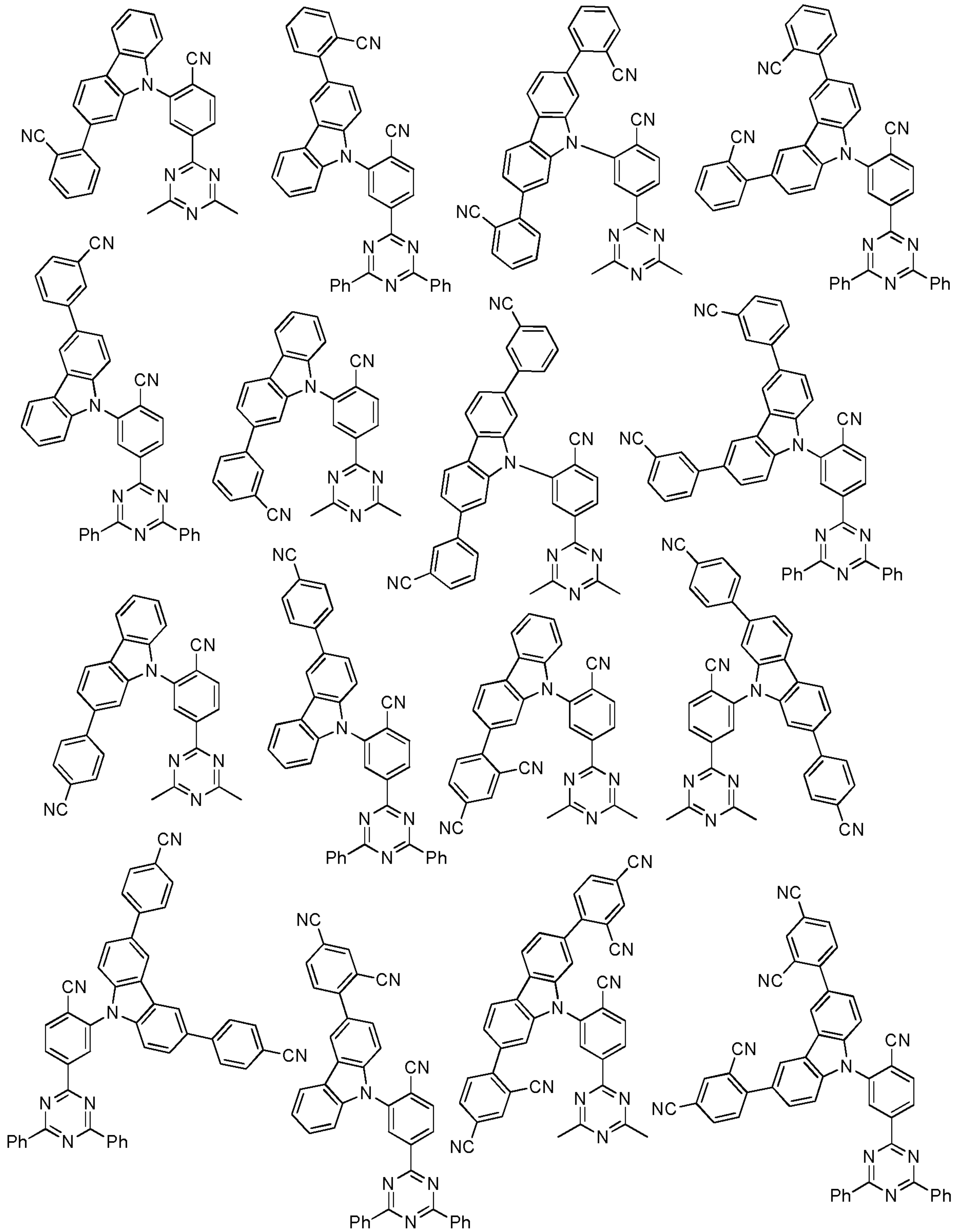

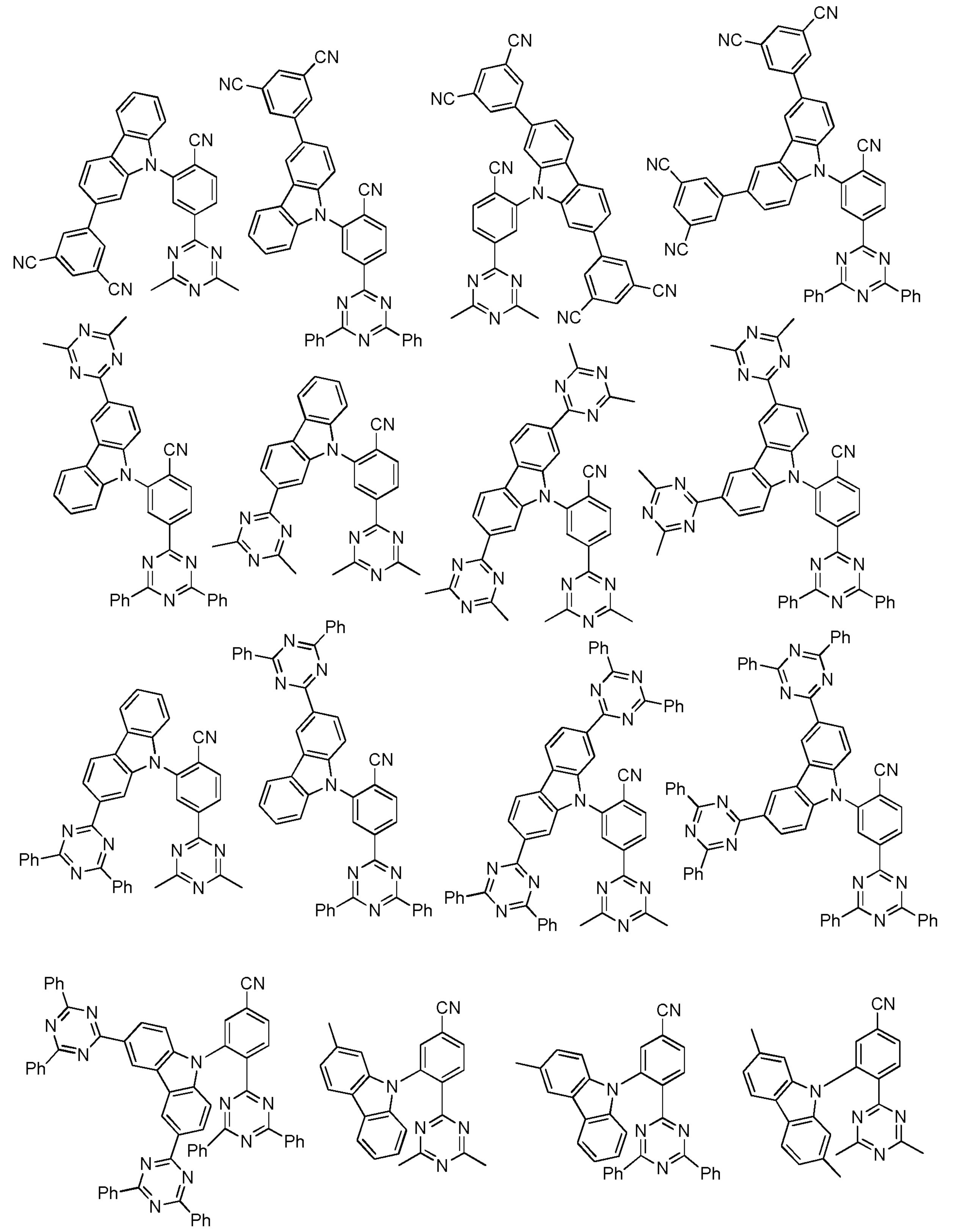

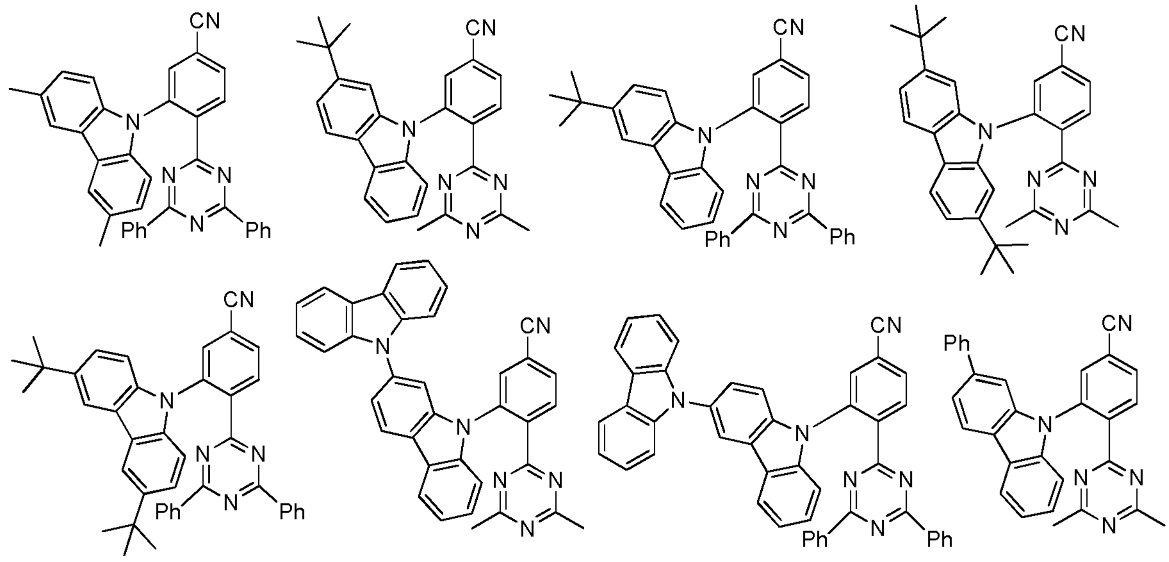

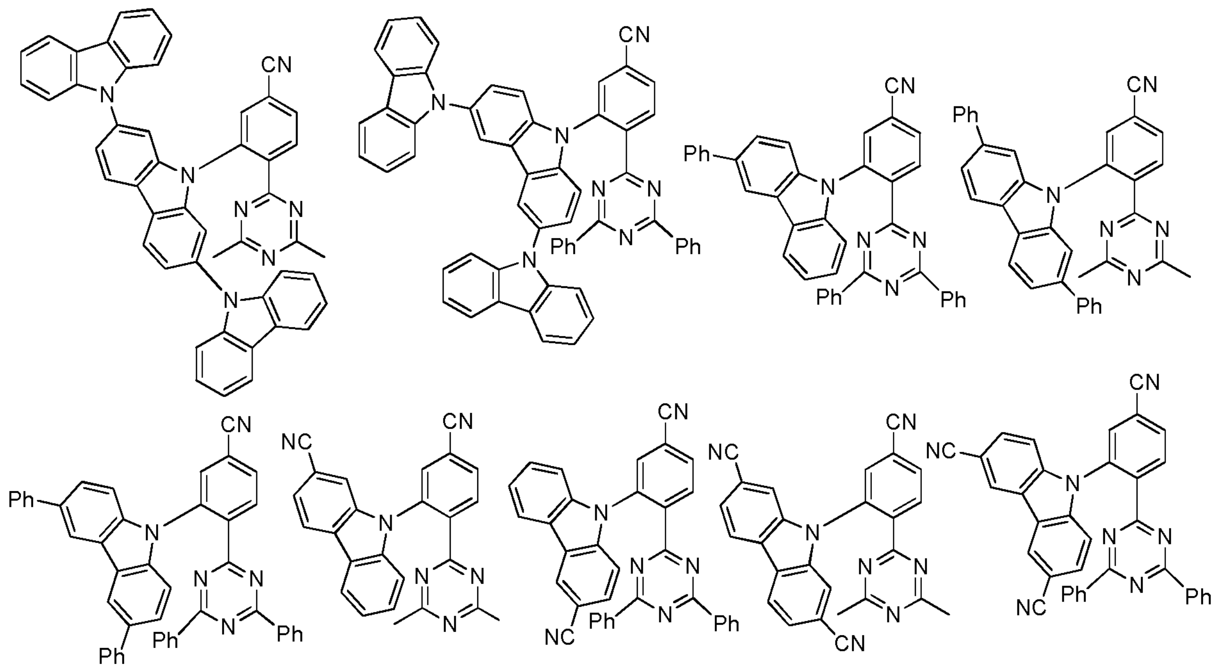

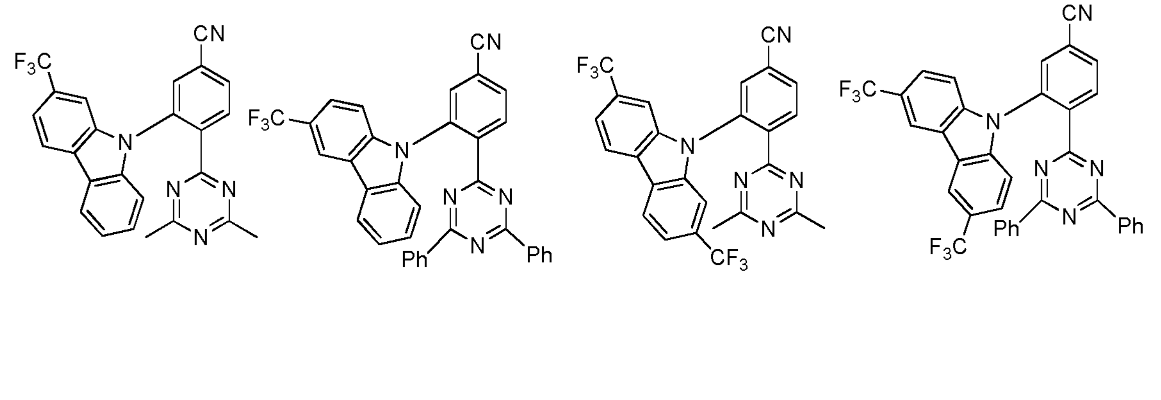

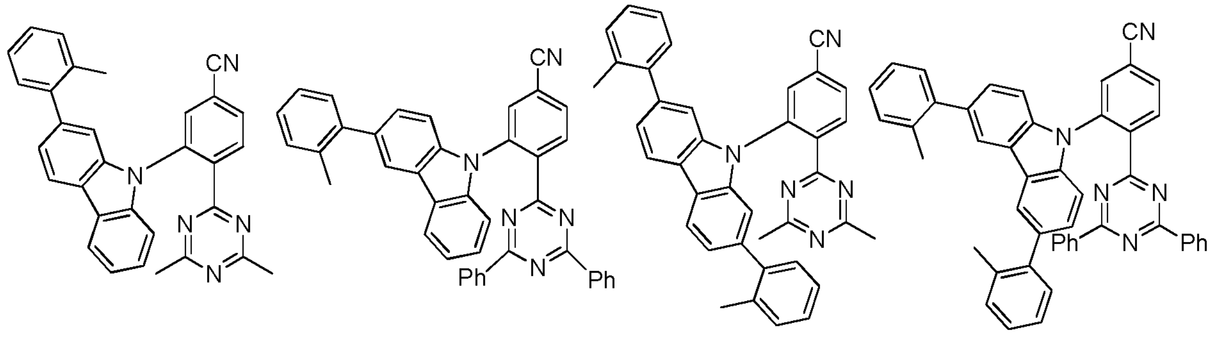

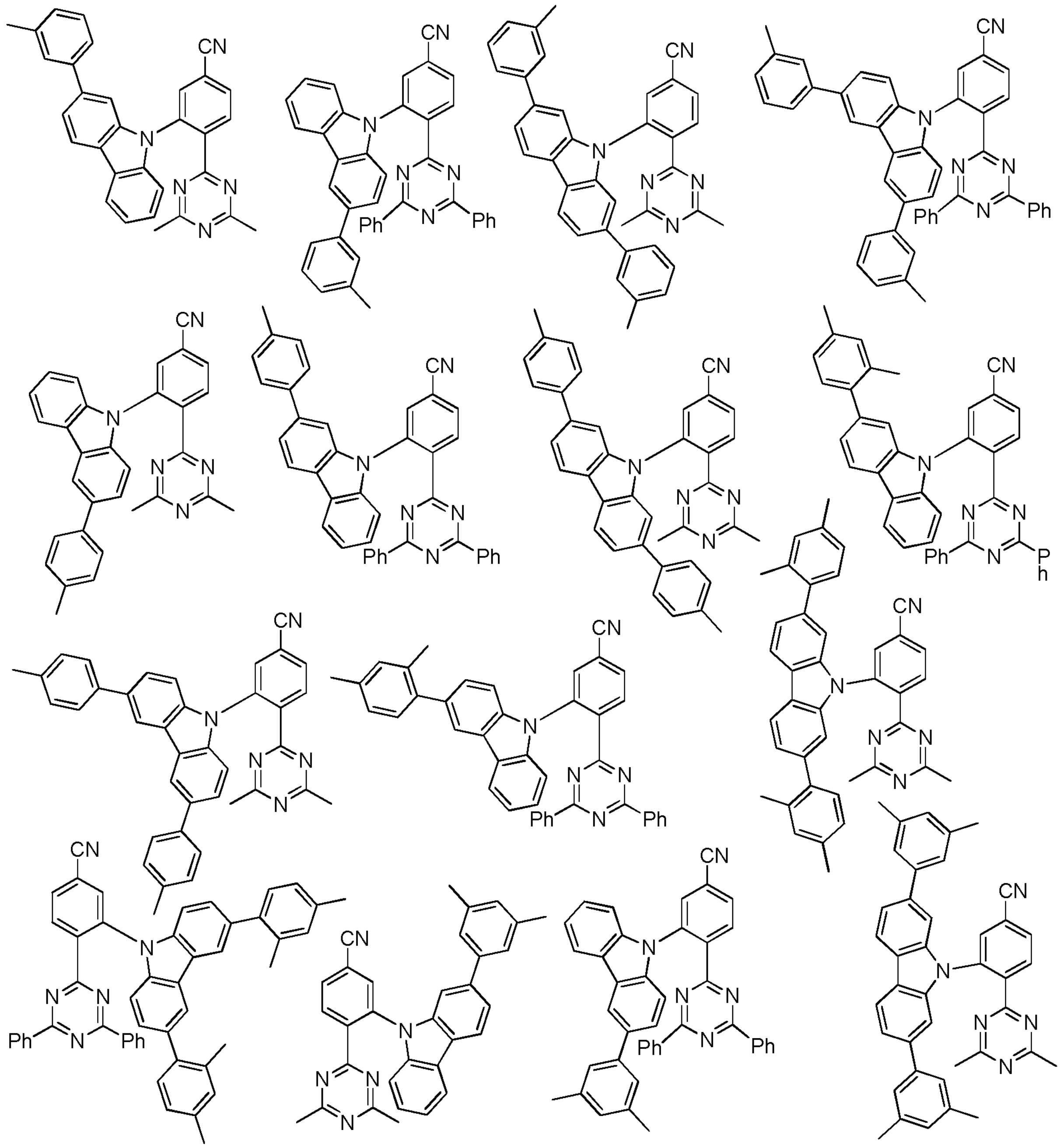

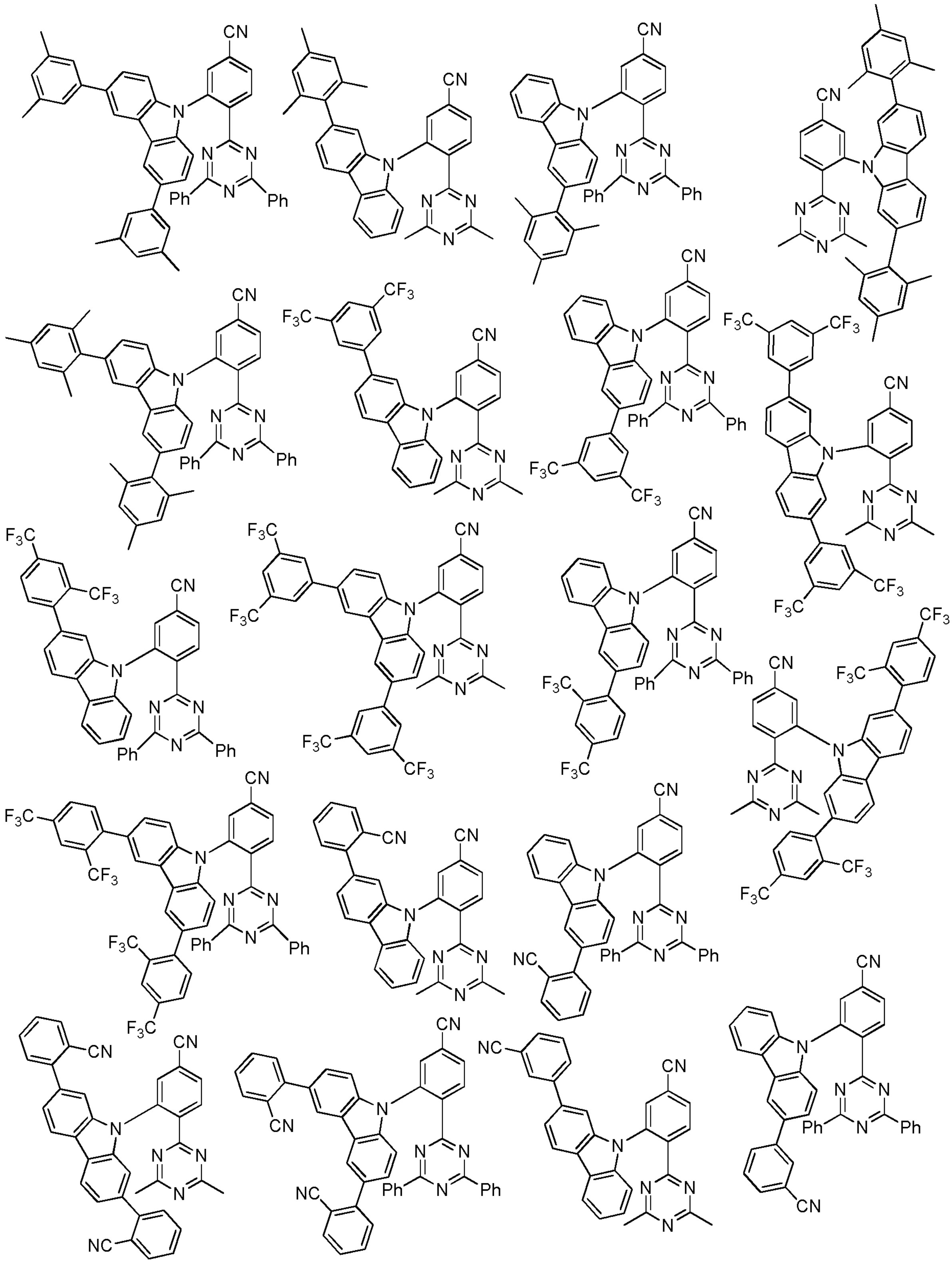

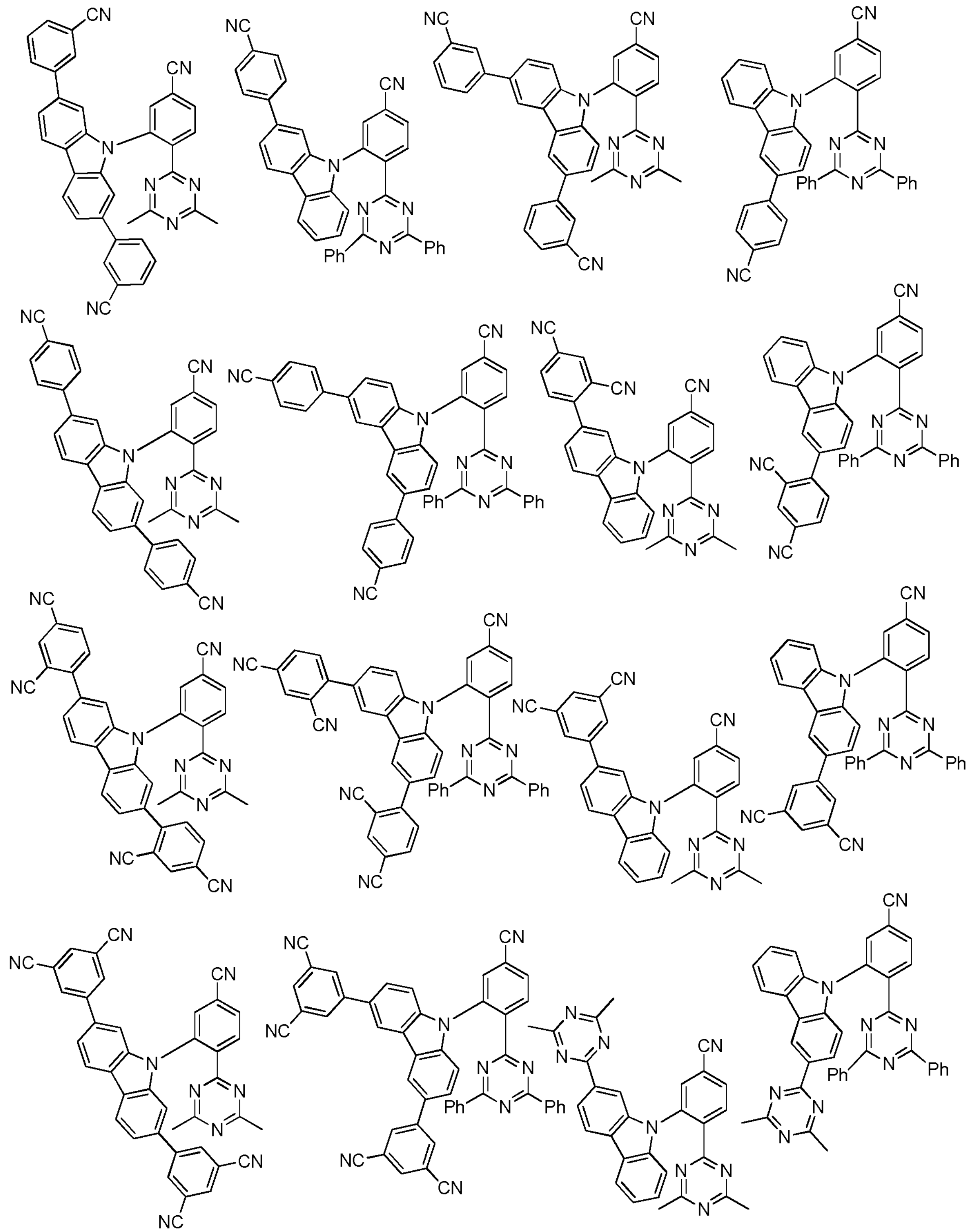

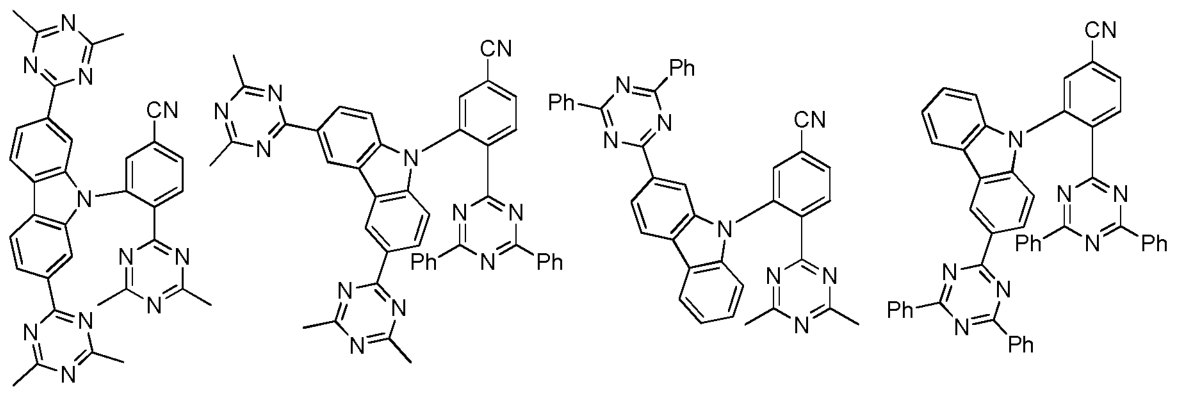

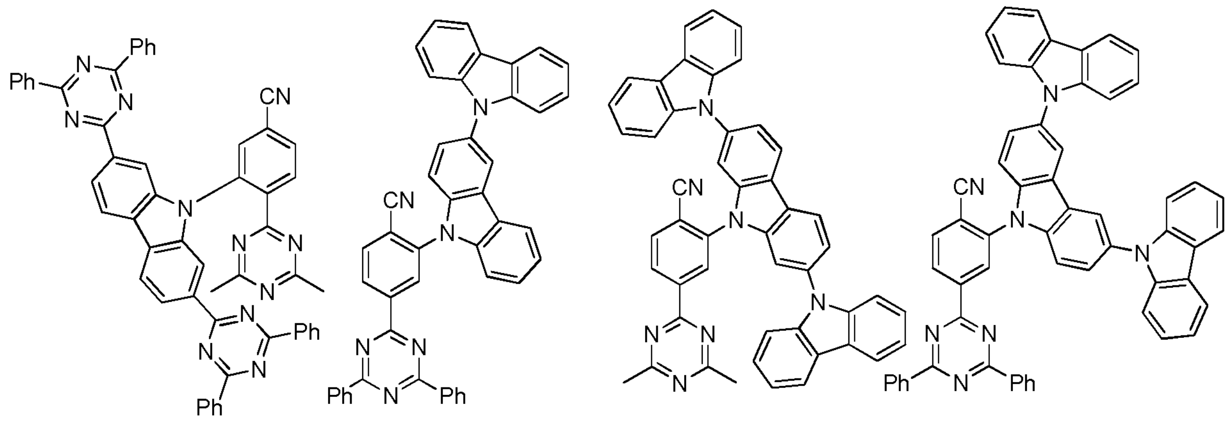

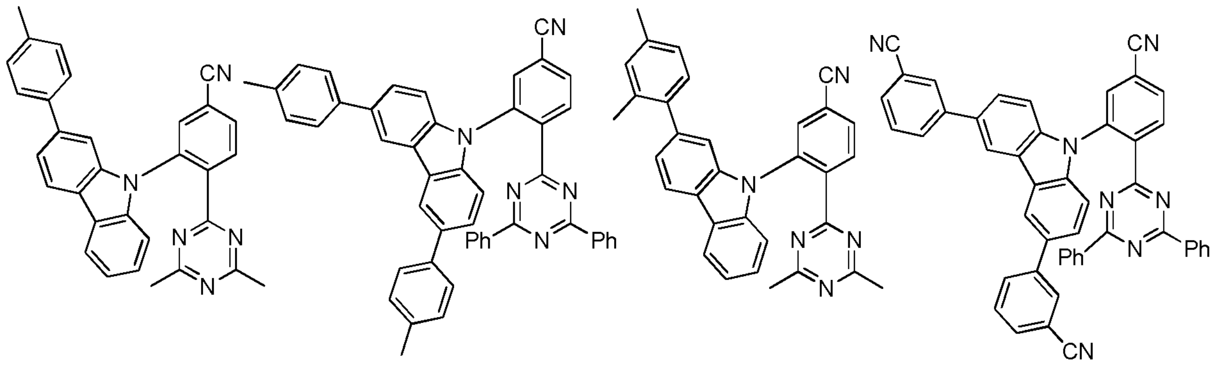

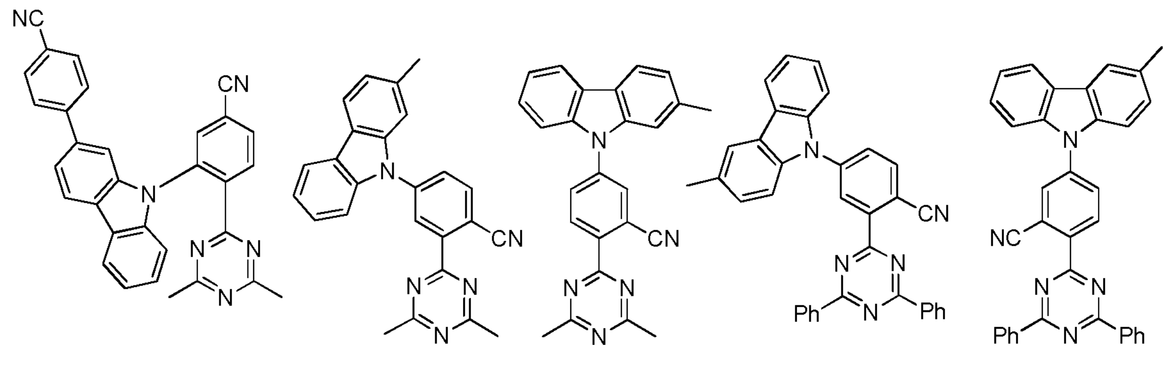

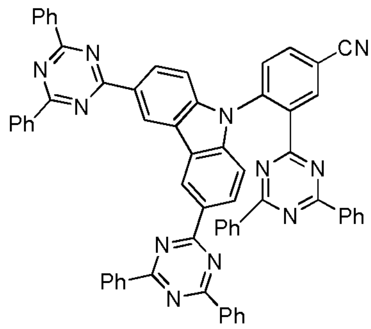
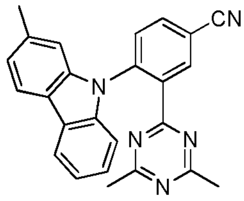
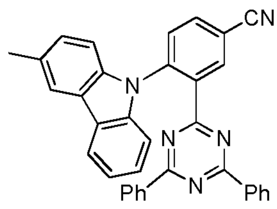
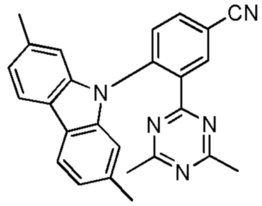

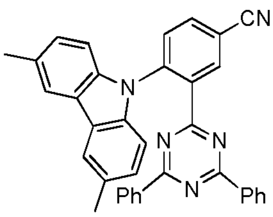
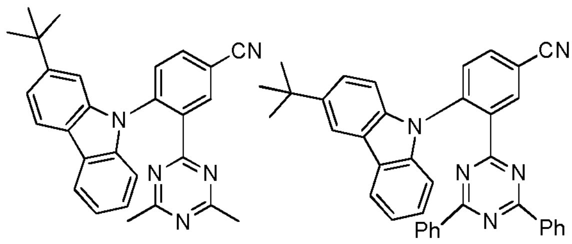
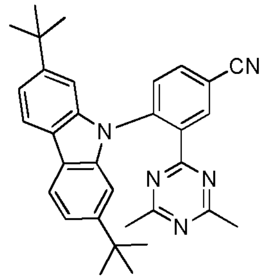

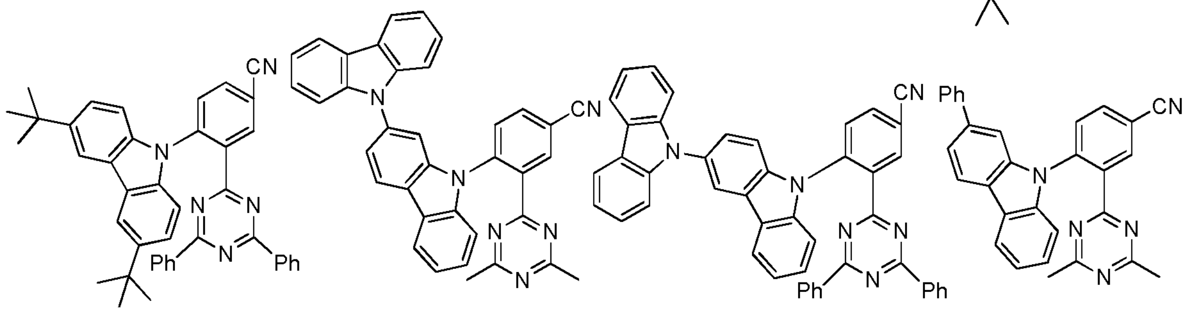

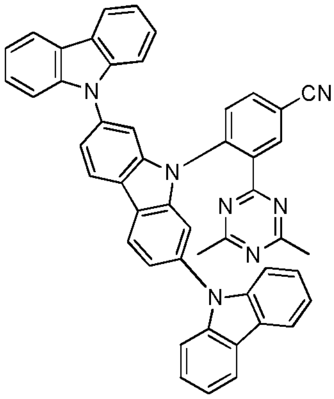
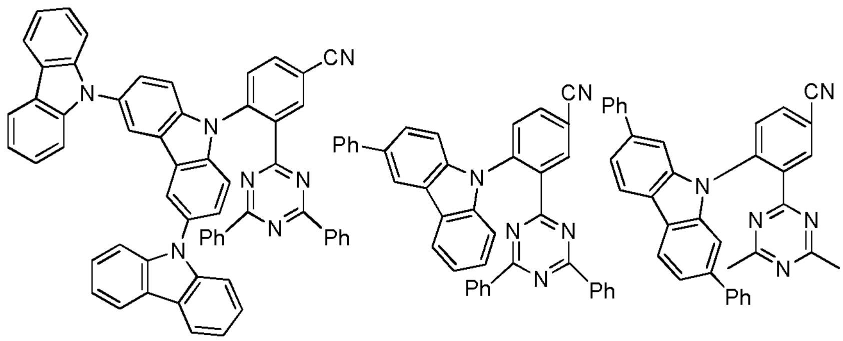

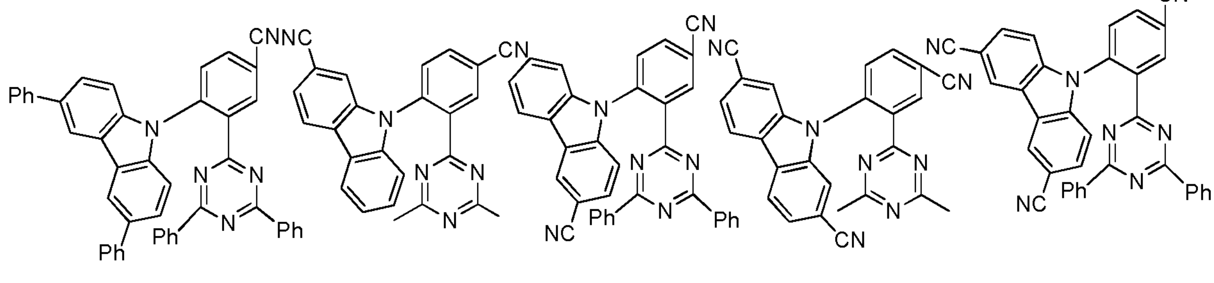

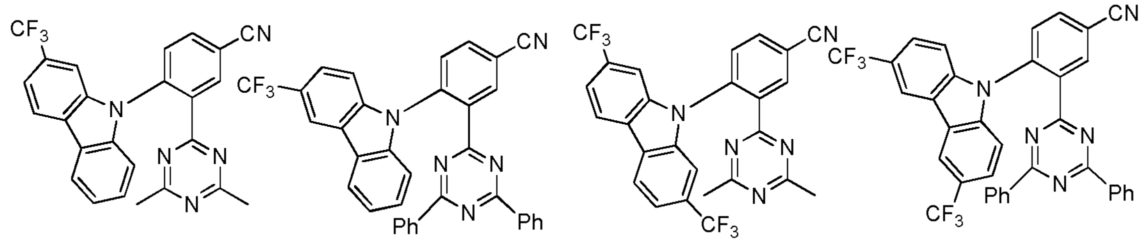

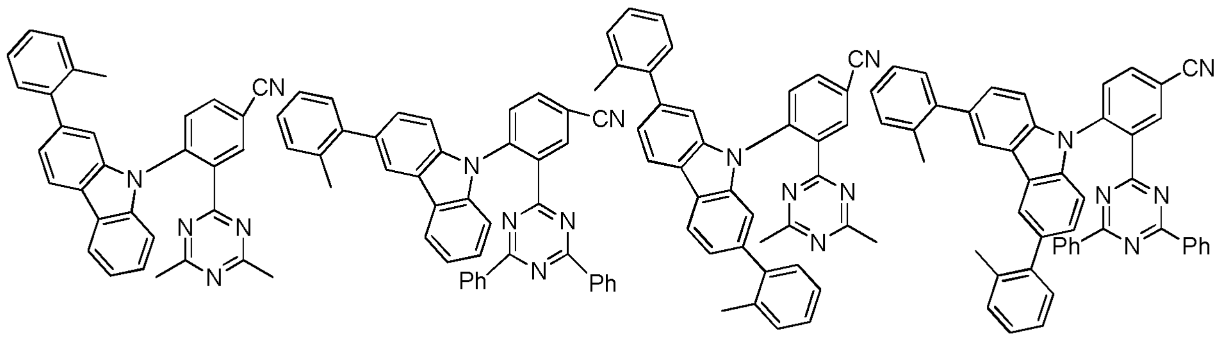

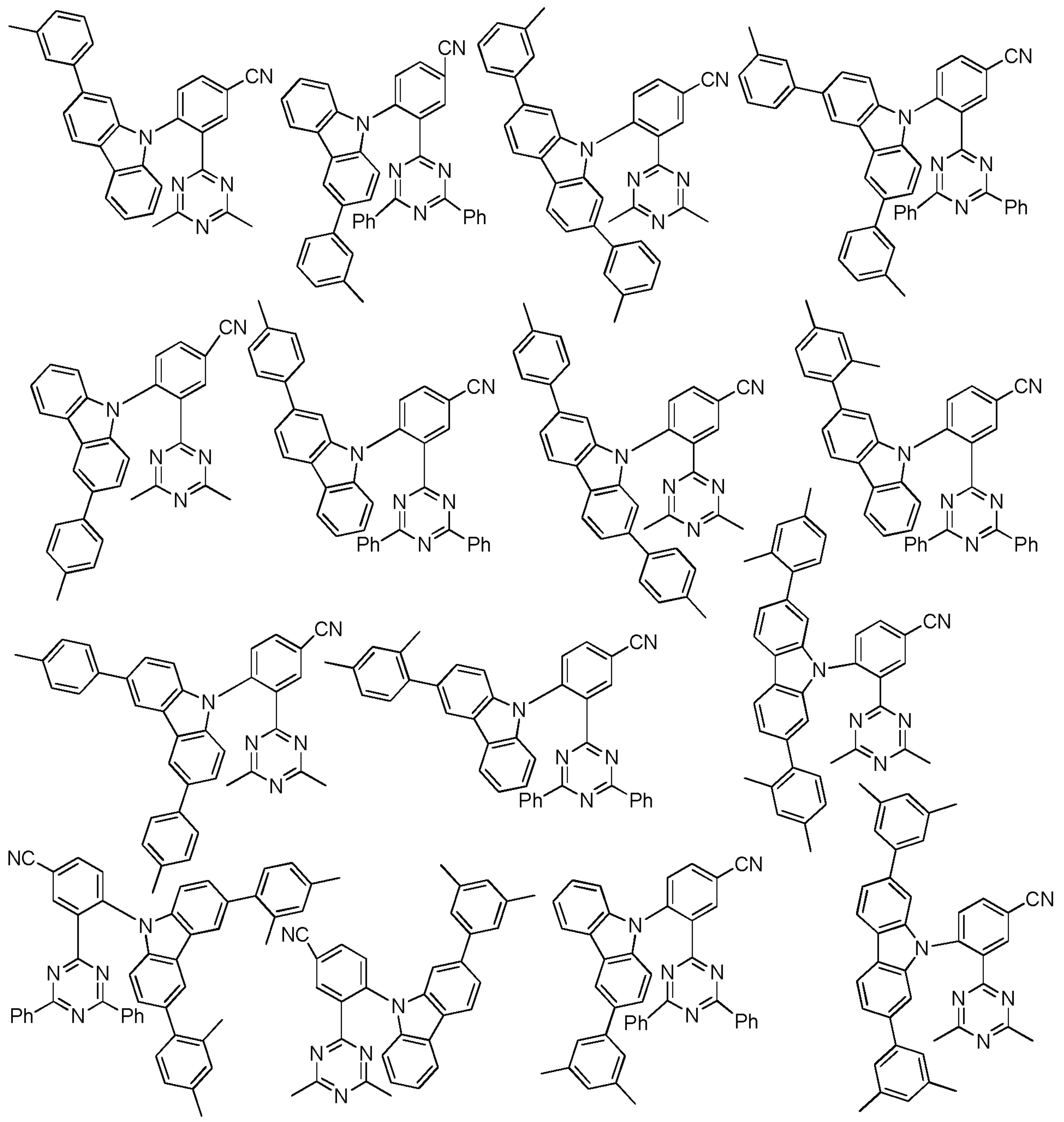

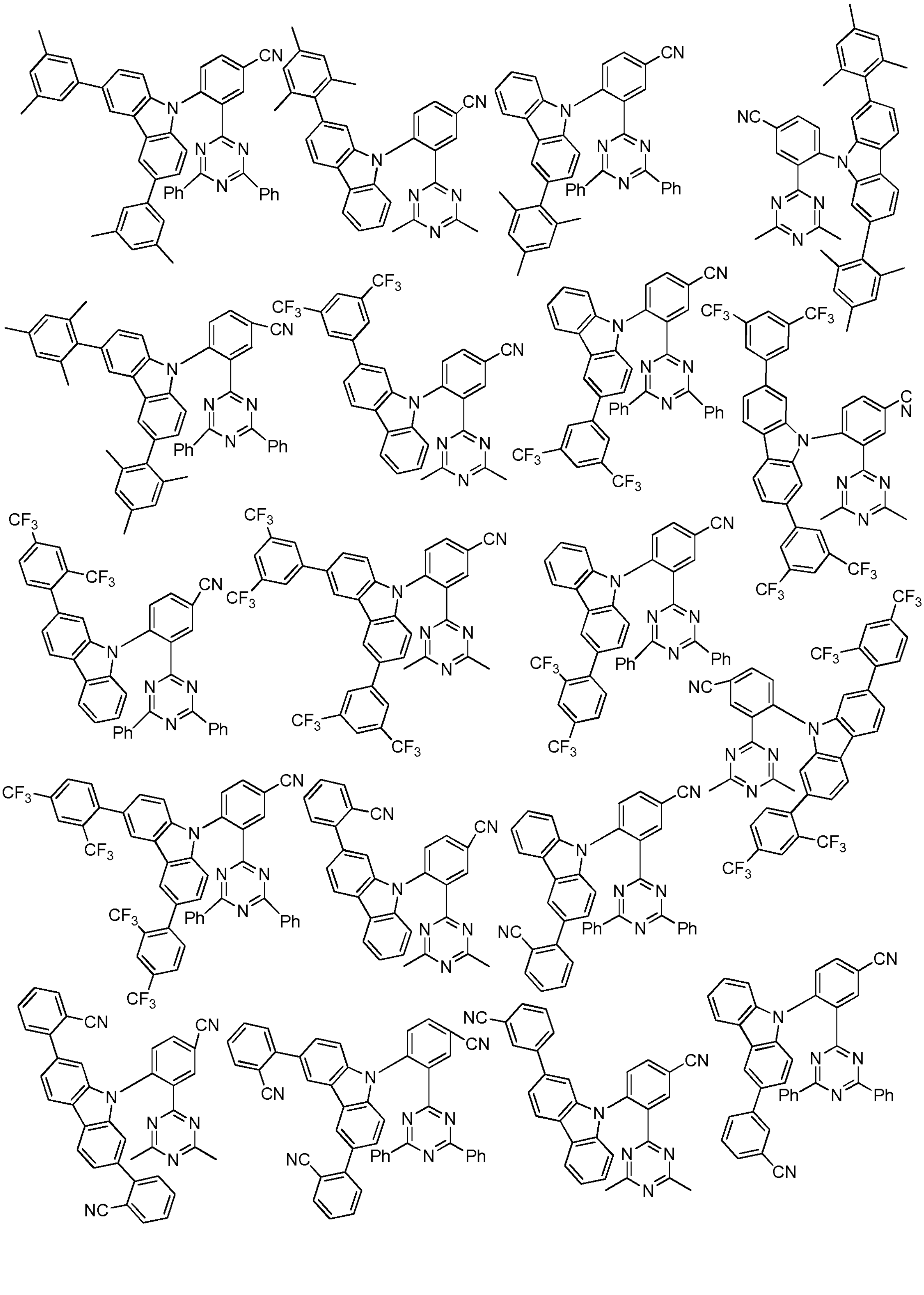

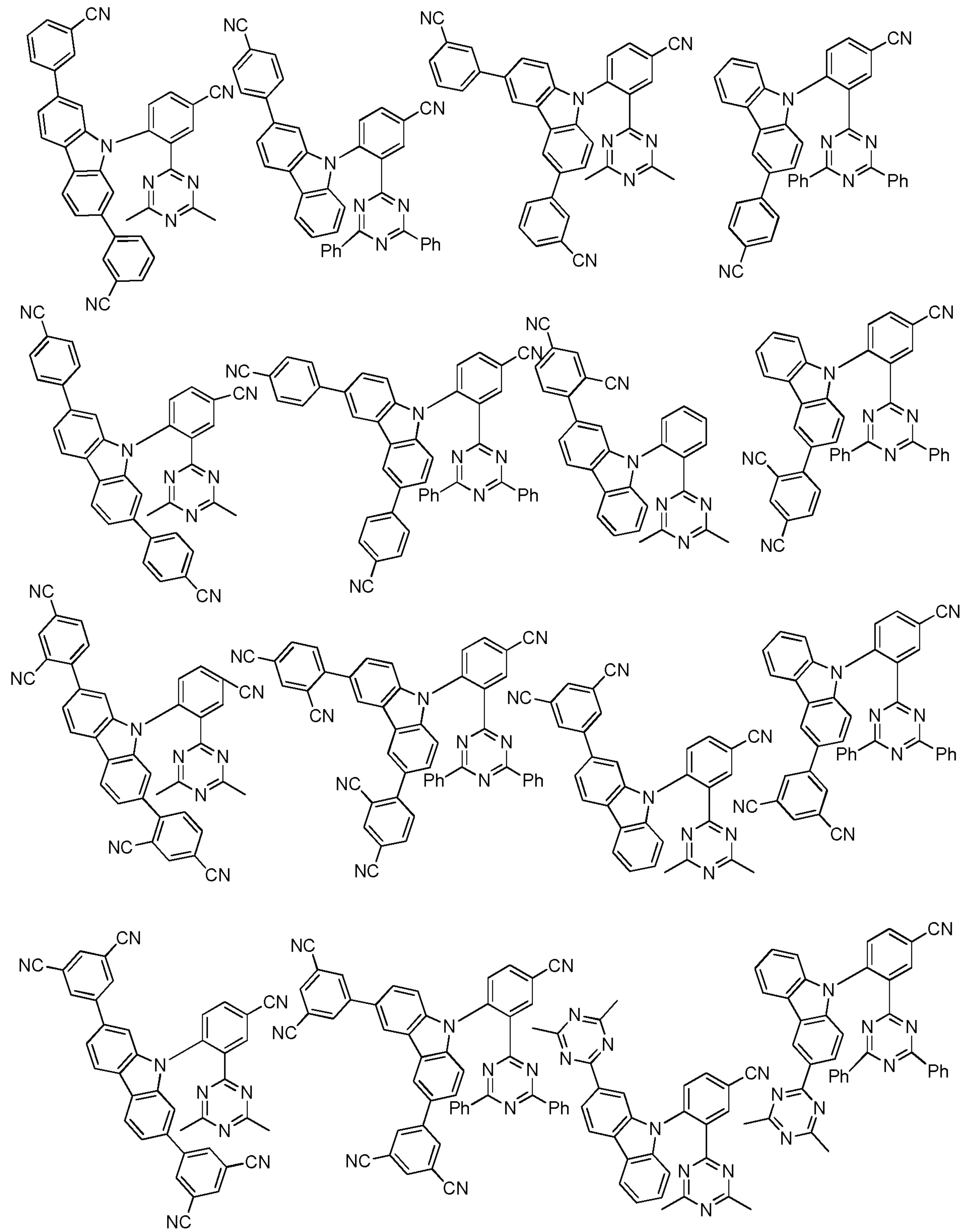

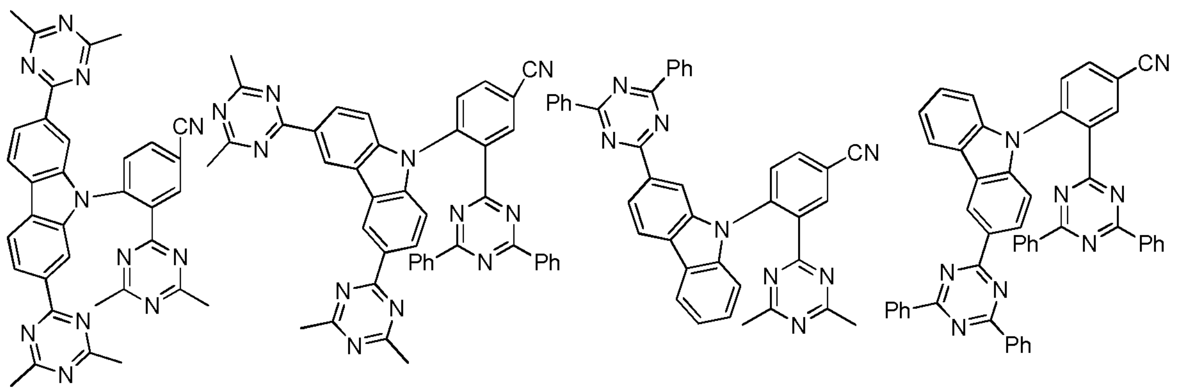

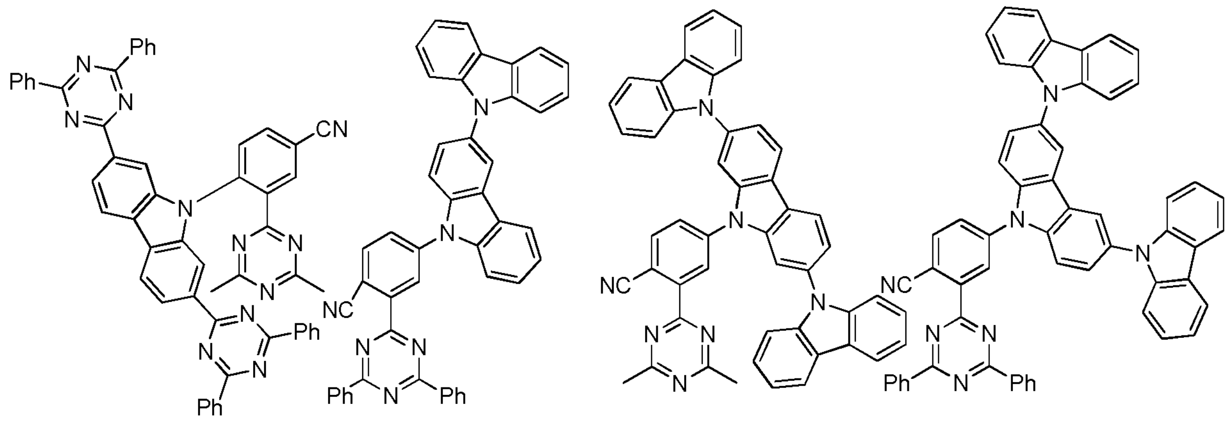

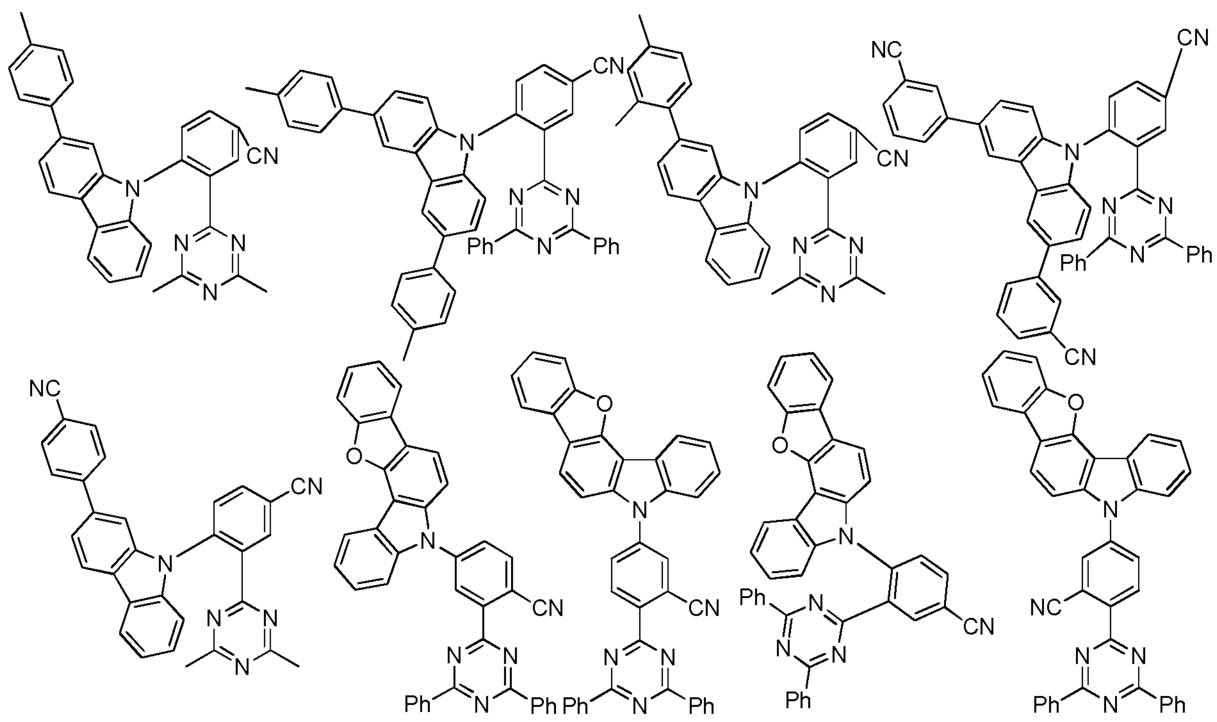

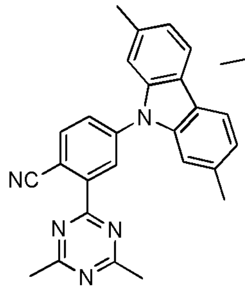
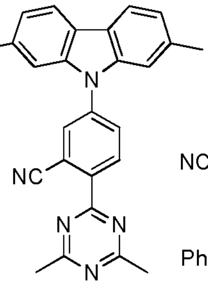
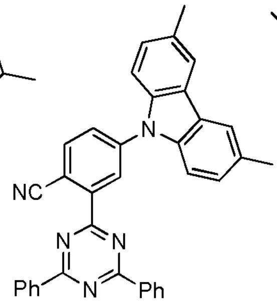
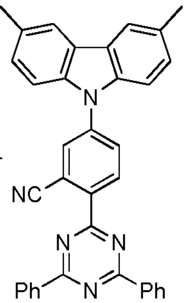
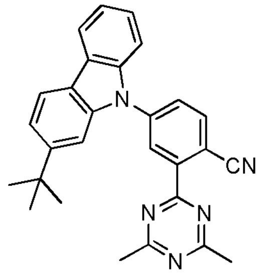

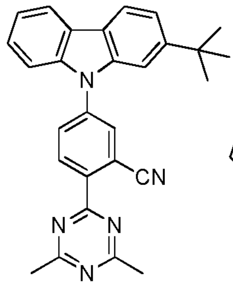
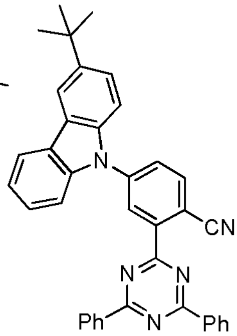
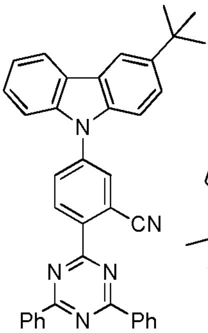
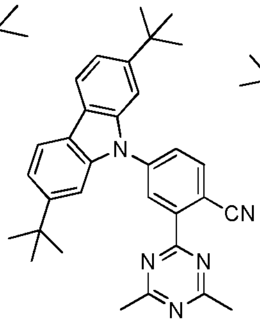
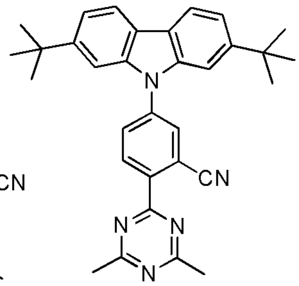

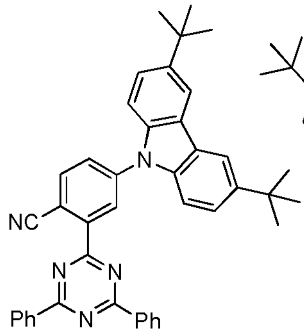
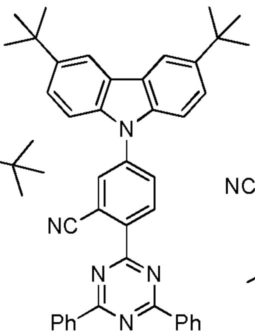
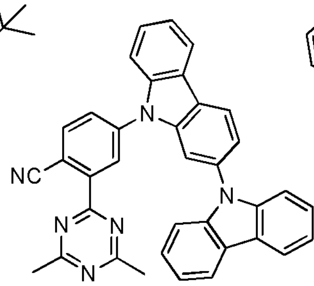
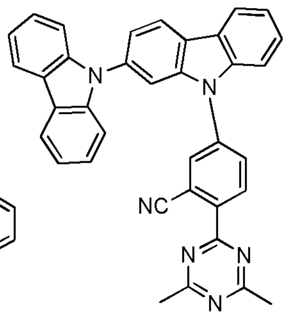

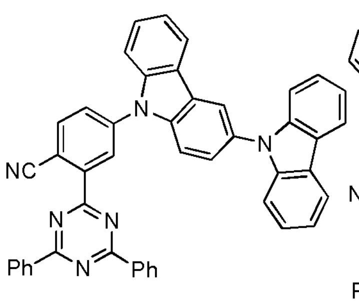
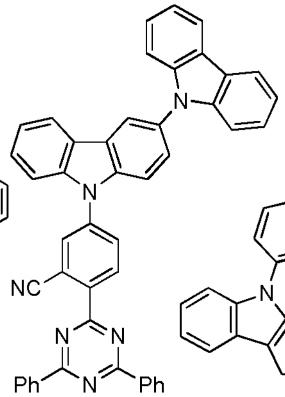
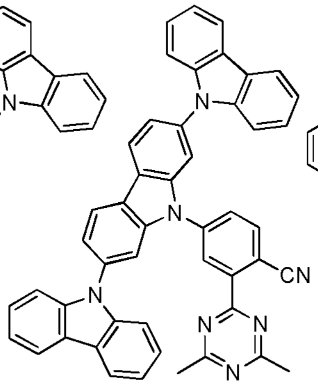
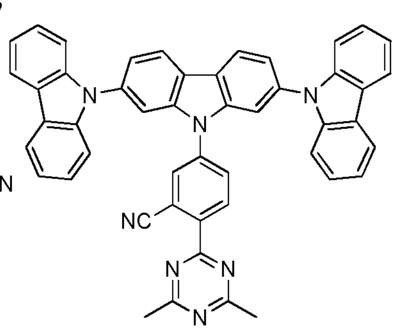

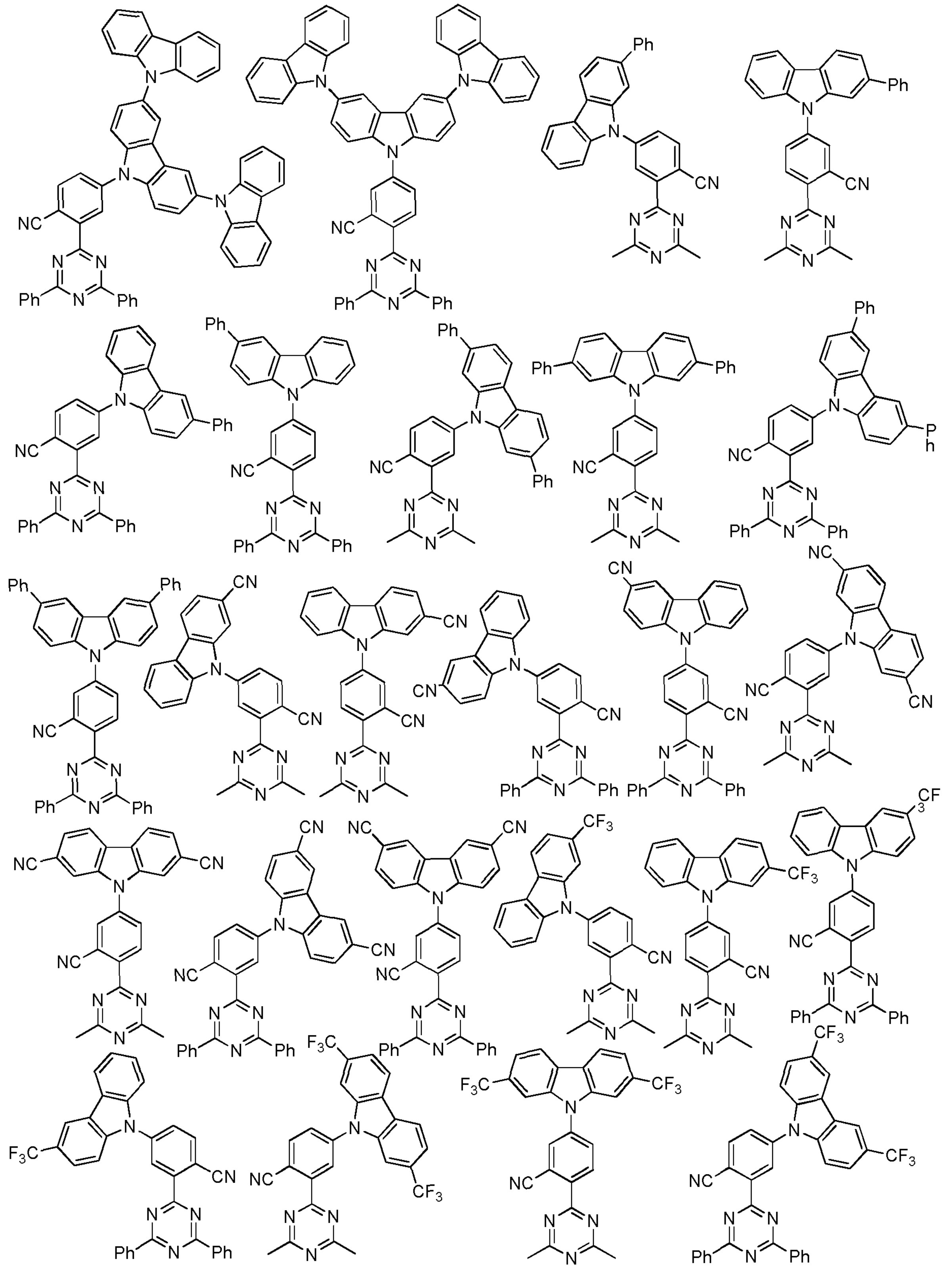

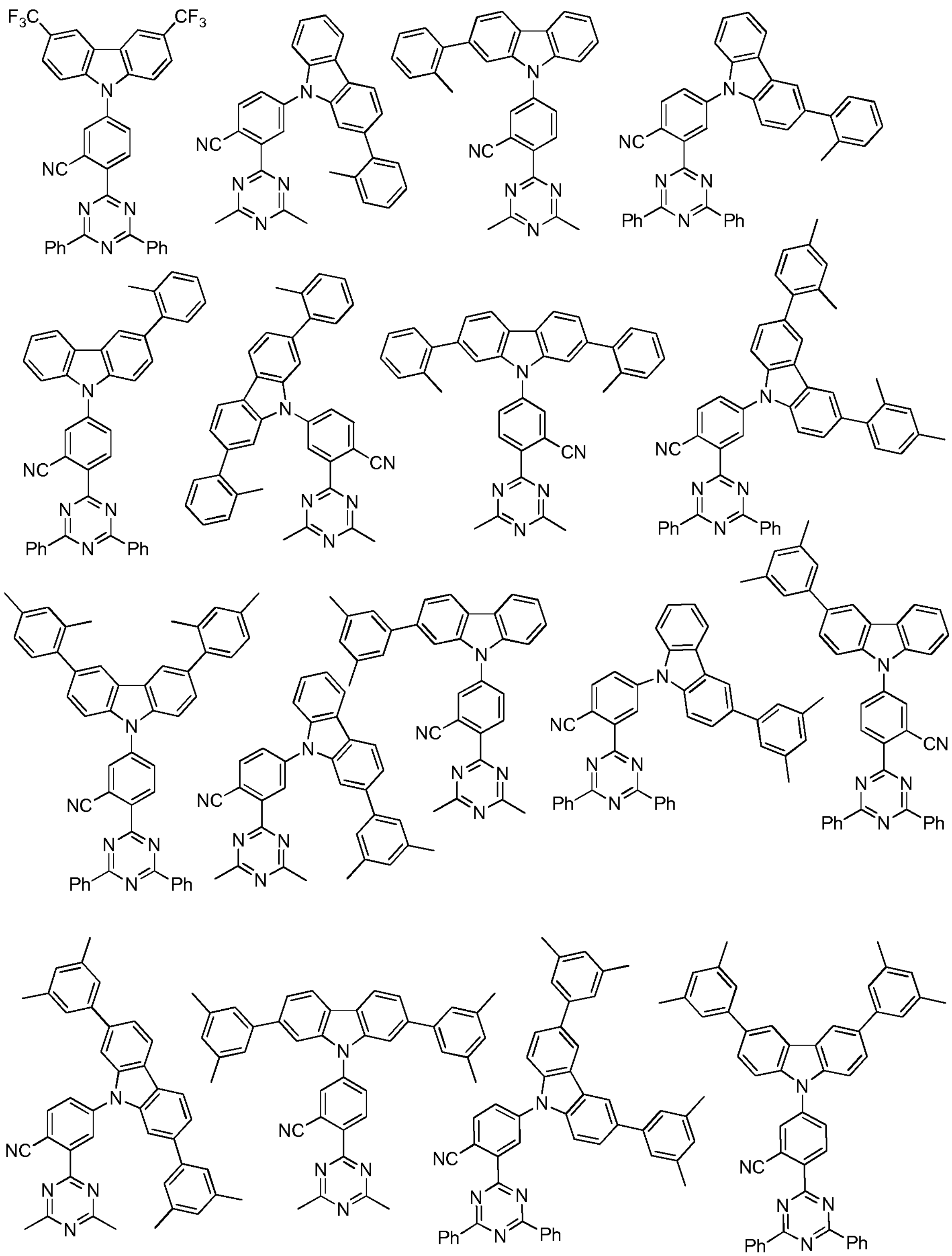

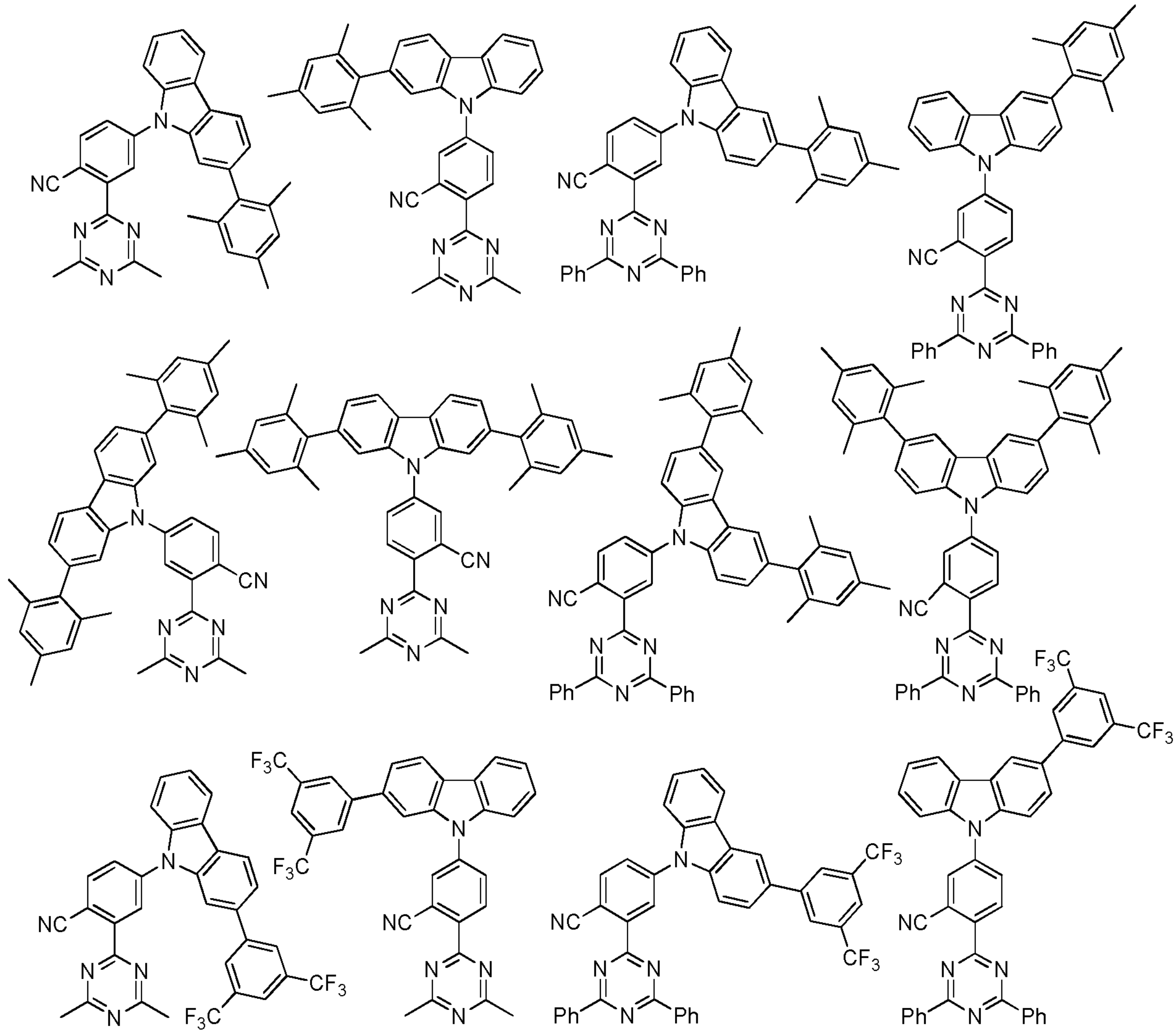

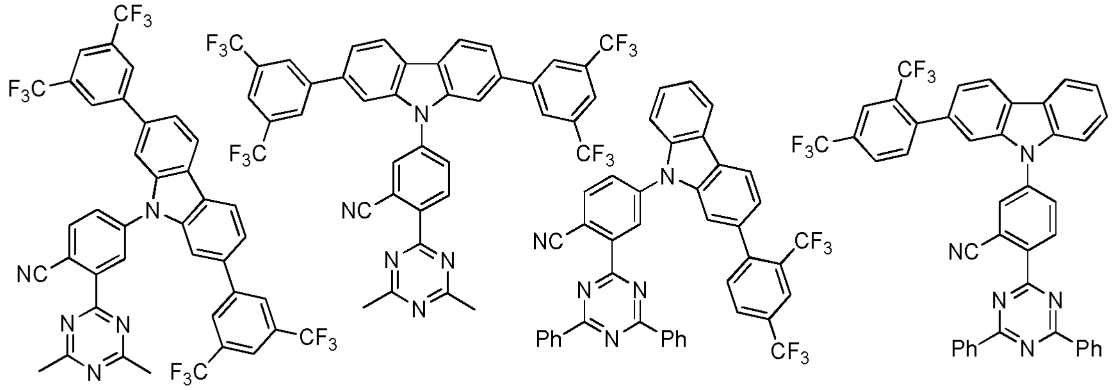

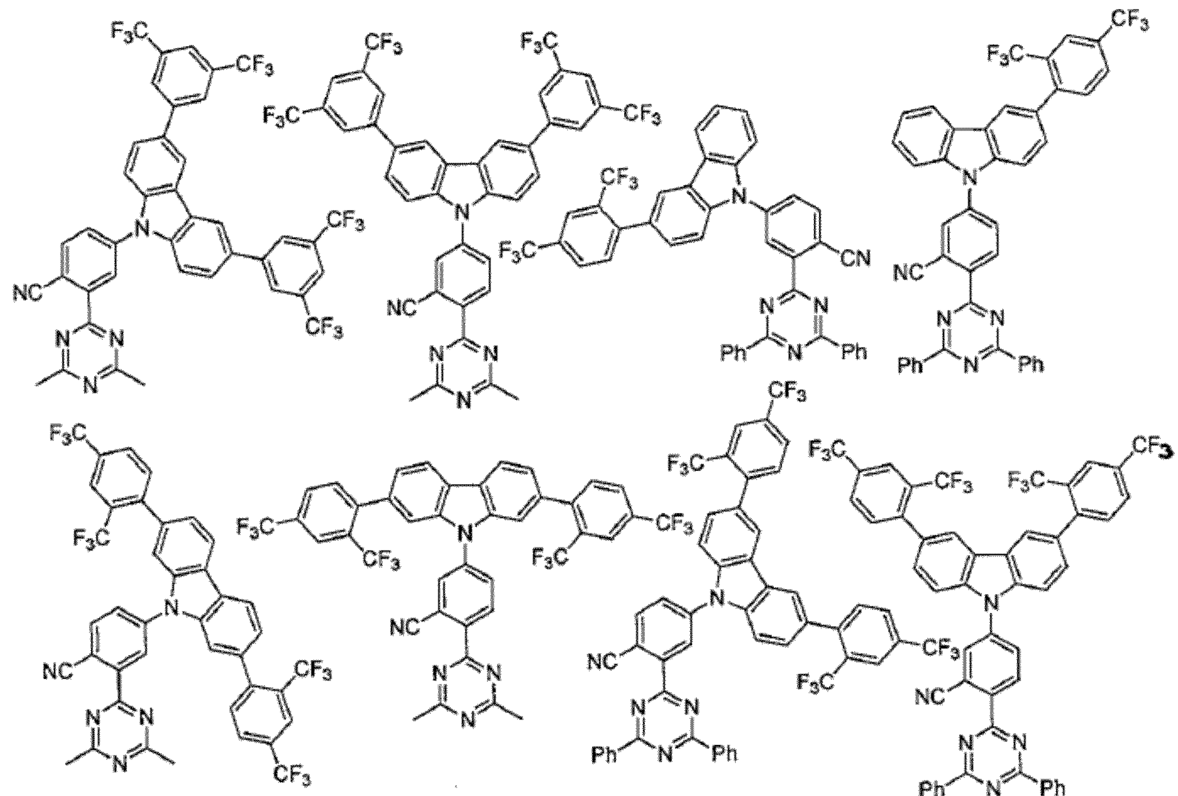

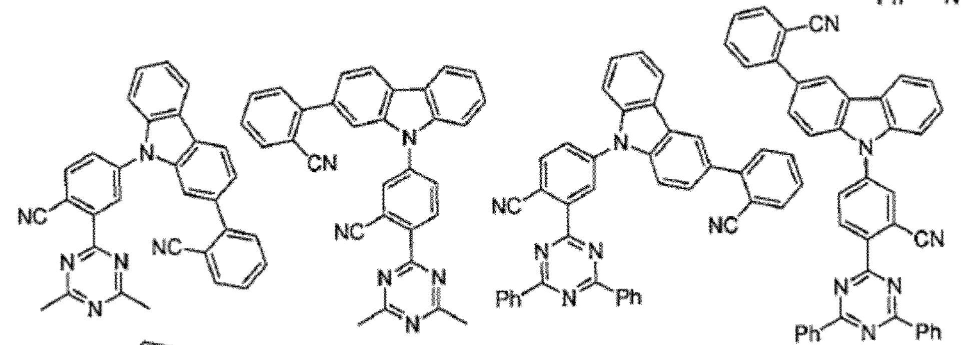

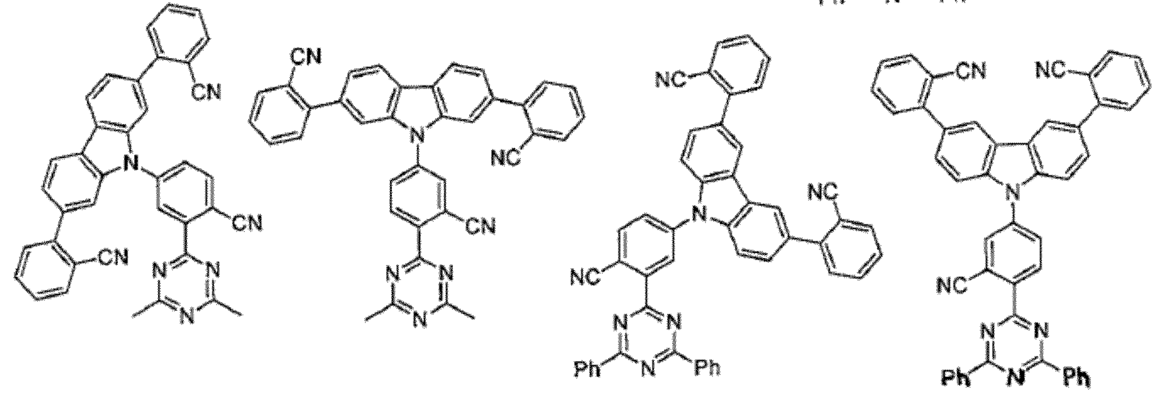

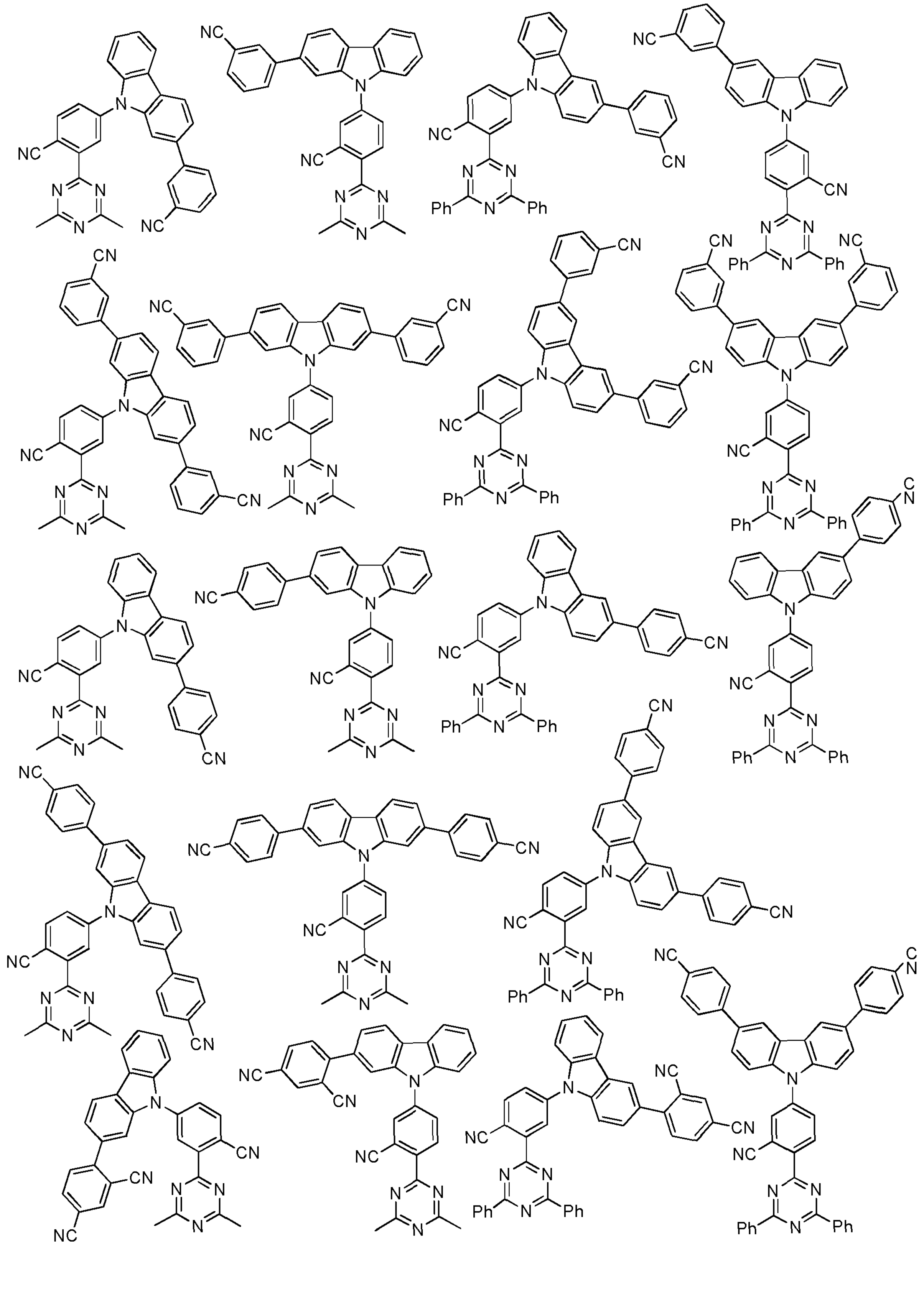

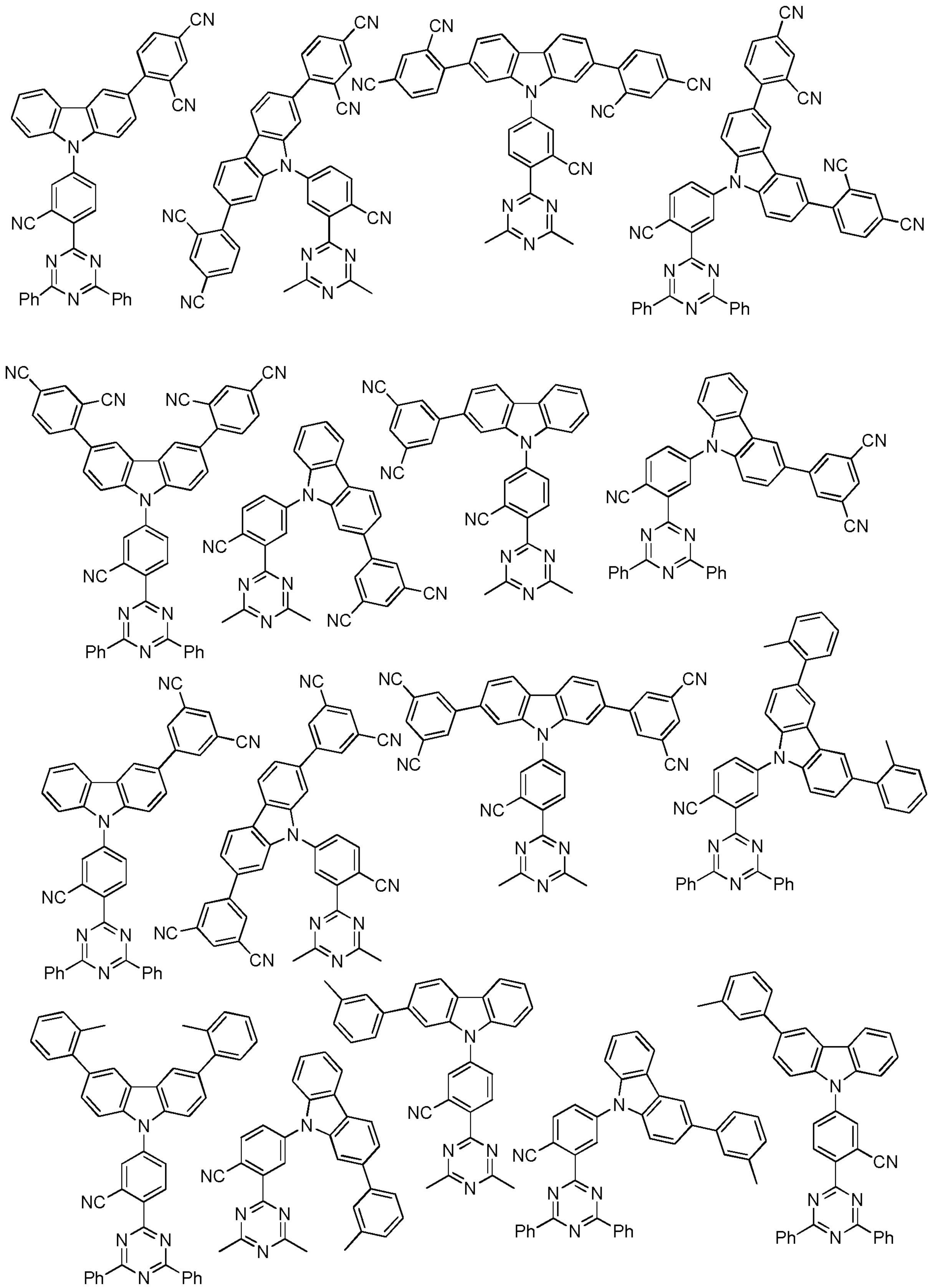

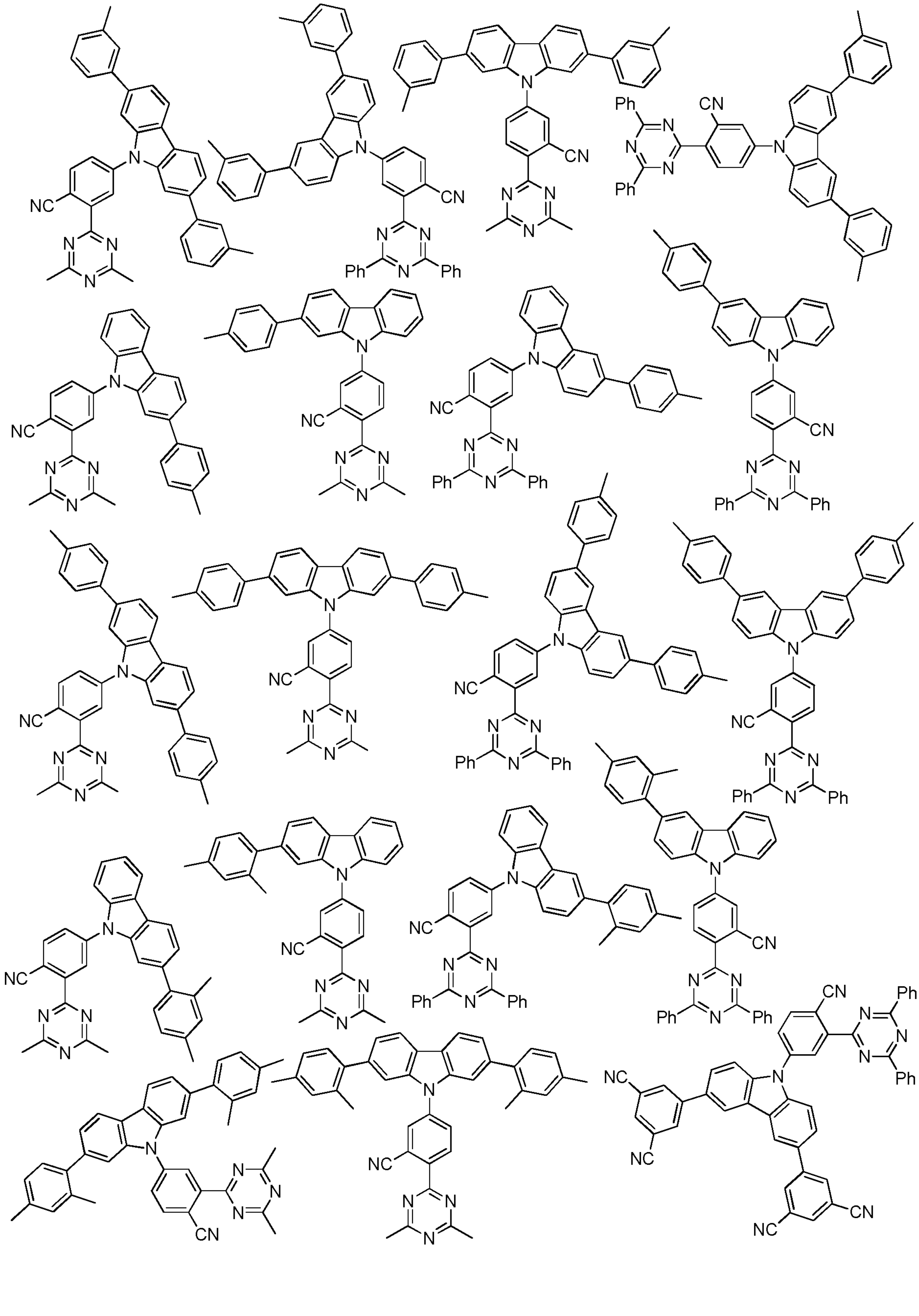

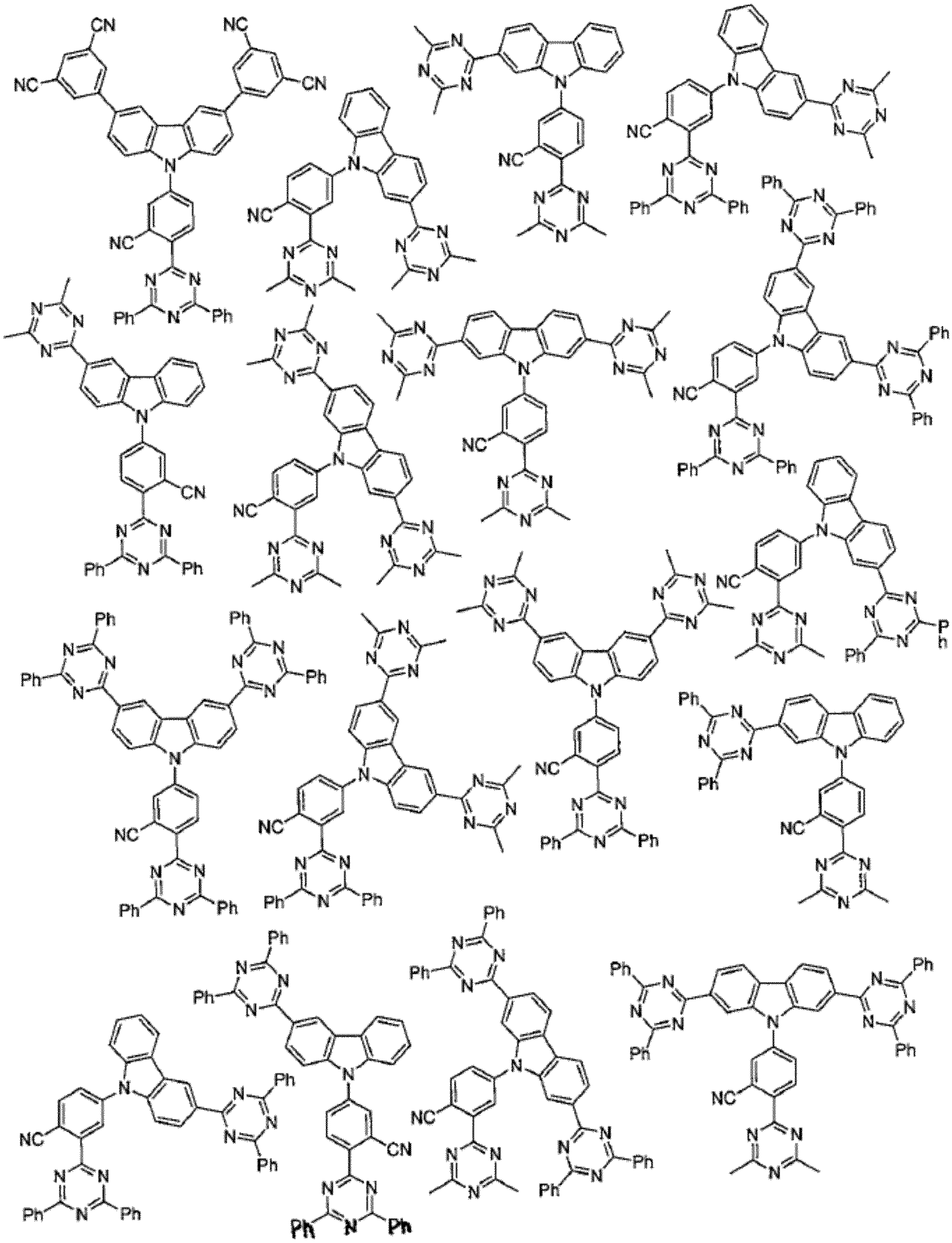

**Figuren**

Es zeigen:

Figur 1 Emissionsspektrum von Beispiel **1** (10 % in PMMA).
Figur 2 Emissionsspektrum von Beispiel **2** (10 % in PMMA).
Figur 3 Emissionsspektrum von Beispiel **3** (10 % in PMMA).
Figur 4 Emissionsspektrum von Beispiel **4** (10 % in PMMA).

| | |
|---|---|
| Figur 5 | Emissionsspektrum von Beispiel **5** (10 % in PMMA). |
| Figur 6 | Emissionsspektrum von Beispiel **6** (10 % in PMMA). |
| Figur 7 | Emissionsspektrum von Beispiel **7** (10 % in PMMA). |
| Figur 8 | Emissionsspektrum von Beispiel **8** (10 % in PMMA). |
| Figur 9 | Emissionsspektrum von Beispiel **9** (10 % in PMMA). |
| Figur 10 | Emissionsspektrum von Beispiel **10** (10 % in PMMA). |
| Figur 11 | Emissionsspektrum von Beispiel **11** (10 % in PMMA). |
| Figur 12 | Emissionsspektrum von Beispiel **12** (10 % in PMMA). |
| Figur 13 | Emissionsspektrum von Beispiel **13** (10 % in PMMA). |
| Figur 14 | Emissionsspektrum von Beispiel **14** (10 % in PMMA). |
| Figur 15 | Emissionsspektrum von Beispiel **15** (10 % in PMMA). |
| Figur 16 | Emissionsspektrum von Beispiel **16** (10 % in PMMA). |
| Figur 17 | Emissionsspektrum von Beispiel **17** (10 % in PMMA). |

**Patentansprüche**

1. Organisches Molekül, aufweisend

- eine erste chemische Einheit aufweisend eine oder bestehend aus einer Struktur gemäß Formel I

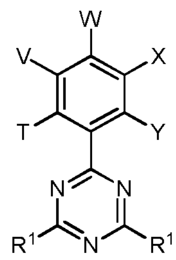

Formel I

und

- eine zweite chemische Einheit aufweisend eine oder bestehend aus einer Struktur gemäß Formel II,

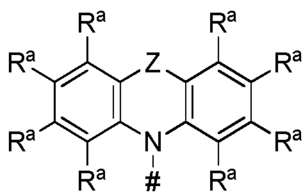

Formel II

wobei die erste chemische Einheit über eine Einfachbindung mit der zweiten chemischen Einheit verknüpft ist;

mit

T ist der Anknüpfungspunkt der Einfachbindung zwischen der ersten chemischen Einheit und der zweiten chemischen Einheit oder ist ausgewählt aus der Gruppe bestehend aus $R^2$ und CN;

V ist H oder ist der Anknüpfungspunkt der Einfachbindung zwischen der ersten chemischen Einheit und der zweiten chemischen Einheit;

W ist Anknüpfungspunkt der Einfachbindung zwischen der ersten chemischen Einheit und der zweiten chemischen Einheit oder ist ausgewählt aus der Gruppe bestehend aus $R^2$ und CN;

X ist ausgewählt aus der Gruppe bestehend aus $R^2$ und CN;

Y ist ausgewählt aus der Gruppe bestehend aus $R^2$ und CN;

# kennzeichnet den Anknüpfungspunkt der Einfachbindung zwischen der zweiten chemischen Einheit und der ersten chemischen Einheit;

Z ist bei jedem Auftreten gleich oder verschieden eine direkte Bindung oder ist ausgewählt aus der Gruppe

bestehend aus $CR^3R^4$, $C=CR^3R^4$, C=O, $C=NR^3$, $NR^3$, O, $SiR^3R^4$, S, S(O) und $S(O)_2$;
$R^1$ ist bei jedem Auftreten gleich oder verschieden ausgewählt aus der Gruppe bestehend aus:

- H, Deuterium,
- einer linearen Alkylgruppe mit 1 bis 5 C-Atomen, einer linearen Alkenyl- oder Alkinylgruppe mit 2 bis 8 C-Atomen, einer verzweigten oder cyclischen Alkyl-, Alkenyl- oder Alkinylgruppe mit 3 bis 10 C-Atomen, wobei in den vorgenannten Gruppen ein oder mehrere H-Atome durch Deuterium ersetzt sein können; und
- einem aromatischen oder heteroaromatischen Ringsystem mit 5 bis 15 aromatischen Ringatomen, das jeweils mit einem oder mehreren Resten $R^6$ substituiert sein kann;

$R^2$ ist bei jedem Auftreten gleich oder verschieden ausgewählt aus der Gruppe bestehend aus:

- H, Deuterium,
- einer linearen Alkylgruppe mit 1 bis 5 C-Atomen, einer linearen Alkenyl- oder Alkinylgruppe mit 2 bis 8 C-Atomen, einer verzweigten oder cyclischen Alkyl-, Alkenyl- oder Alkinylgruppe mit 3 bis 10 C-Atomen, wobei in den vorgenannten Gruppen ein oder mehrere H-Atome durch Deuterium ersetzt sein können; und
- einem aromatischen oder heteroaromatischen Ringsystem mit 5 bis 15 aromatischen Ringatomen, das jeweils mit einem oder mehreren Resten $R^6$ substituiert sein kann;

$R^a$, $R^3$ und $R^4$ ist bei jedem Auftreten gleich oder verschieden ausgewählt aus der Gruppe bestehend aus:

- H, Deuterium, $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

$R^5$ ist bei jedem Auftreten gleich oder verschieden ausgewählt aus der Gruppe bestehend aus:

- H, Deuterium, $N(R^6)_2$, OH, $Si(R^6)_3$, $B(OR^6)_2$, $OSO_2R^6$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;

- einem aromatischen oder heteroaromatischen Ringsystem mit 5 bis 60 aromatischen Ringatomen, das jeweils mit einem oder mehreren Resten $R^6$ substituiert sein kann;
- einer Aryloxy- oder Heteroaryloxygruppe mit 5 bis 60 aromatischen Ringatomen, die jeweils mit einem oder mehreren Resten $R^6$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^6$ substituiert sein kann;

$R^6$ ist bei jedem Auftreten gleich oder verschieden ausgewählt aus der Gruppe bestehend aus:

- H, Deuterium, OH, $CF_3$, CN, F;
- einer linearen Alkyl-, Alkoxy- oder Thioalkoxygruppe mit 1 bis 5 C-Atomen, wobei ein oder mehrere H-Atome durch Deuterium, CN, $CF_3$ oder $NO_2$ ersetzt sein können;
- einer linearen Alkenyl- oder Alkinylgruppe mit 2 bis 5 C-Atomen, wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen;
- einer Aryloxy- oder Heteroaryloxygruppe mit 5 bis 60 aromatischen Ringatomen; und
- einer Diarylaminogruppe, Diheteroarylaminogruppe oder Arylheteroarylaminogruppe mit 10 bis 40 aromatischen Ringatomen;

wobei jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden kann;
wobei genau ein Rest ausgewählt aus der Gruppe bestehend aus T, W, X und Y gleich CN ist und genau ein Rest ausgewählt aus der Gruppe bestehend aus T, V und W gleich dem Anknüpfungspunkt der Einfachbindung zwischen der ersten chemischen Einheit gemäß Formel I und der zweiten chemischen Einheit ist und
weiterhin W gleich H ist, wenn T gleich CN und V gleich dem Anknüpfungspunkt der Einfachbindung zwischen der ersten chemischen Einheit und der zweiten chemischen Einheit ist;
wobei eine Verbindung der Formel

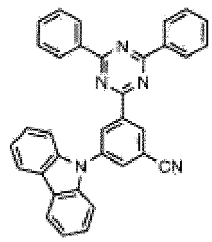

ausgeschlossen ist.

2. Organisches Molekül nach Anspruch 1, wobei $R^1$ bei jedem Auftreten gleich oder verschieden gleich Methyl oder Phenyl ist.

3. Organisches Molekül nach Anspruch 1 oder 2, wobei $R^2$ bei jedem Auftreten gleich oder verschieden gleich H, Methyl oder Phenyl ist.

4. Organisches Molekül nach Anspruch 1 bis 3, wobei W gleich CN ist.

5. Organisches Molekül nach Anspruch 1 bis 4, wobei die zweite chemische Einheit eine Struktur der Formel IIa aufweist:

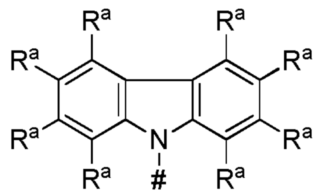

Formel IIa

wobei für # und $R^a$ die in Anspruch 1 genannten Definitionen gelten.

**6.** Organisches Molekül nach Anspruch 1 bis 5, wobei die zweite chemische Einheit eine Struktur der Formel IIb aufweist:

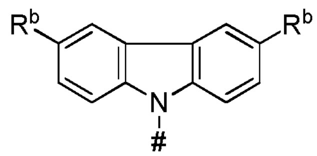

Formel IIb

wobei

$R^b$ bei jedem Auftreten gleich oder verschieden ausgewählt ist aus der Gruppe bestehen aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

und für # und $R^5$ die in Anspruch 1 genannten Definitionen gelten.

**7.** Organisches Molekül nach Anspruch 1 bis 5, wobei die zweite chemische Einheit eine Struktur der Formel IIc aufweist:

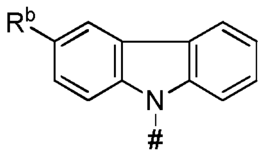

Formel IIc

wobei

$R^b$ bei jedem Auftreten gleich oder verschieden ausgewählt ist aus der Gruppe bestehen aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

und im Übrigen die in Anspruch 1 genannte Definitionen gelten.

**8.** Organisches Molekül nach Anspruch 6 oder 7, wobei $R^b$ bei jedem Auftreten gleich oder verschieden
Me, $^i$Pr, $^t$Bu, CN, $CF_3$,
Ph, das jeweils mit einem oder mehreren Resten ausgewählt aus Me, $^i$Pr, $^t$Bu, CN, $CF_3$ oder Ph substituiert sein kann,
Pyridinyl, das jeweils mit einem oder mehreren Resten ausgewählt aus Me, $^i$Pr, $^t$Bu, CN, $CF_3$ oder Ph substituiert sein kann,
Pyrimidinyl, das jeweils mit einem oder mehreren Resten ausgewählt aus Me, $^i$Pr, $^t$Bu, CN, $CF_3$ oder Ph substituiert sein kann, oder
Triazinyl, das jeweils mit einem oder mehreren Resten ausgewählt aus Me, $^i$Pr, $^t$Bu, CN, $CF_3$ oder Ph substituiert sein kann,
ist.

**9.** Verfahren zur Herstellung eines organischen Moleküls nach Anspruch 1 bis 8, wobei eine in 4- und 6-Position $R^1$-substituiertes 2-Halogen-1,3,5-triazin als Edukt eingesetzt wird.

**10.** Verwendung eines organischen Moleküls nach Anspruch 1 bis 8 als lumineszierender Emitter und/oder als Hostmaterial und/oder als Elektronentransportmaterial und/oder als Lochinjektionsmaterial und/oder als Lochblockiermaterial in einer organischen optoelektronischen Vorrichtung.

**11.** Verwendung nach Anspruch 10, wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**12.** Zusammensetzung, aufweisend oder bestehend aus:

(a) mindestens einem organischen Molekül nach einem der Ansprüche 1 bis 8, insbesondere in Form eines Emitters und/oder Hosts, und
(b) einem oder mehrerer Emitter- und/oder Hostmaterialien, die von dem Molekül nach Anspruch 1 bis 8 verschiedenen sind und
(c) optional einem oder mehreren Farbstoffen und/oder einem oder mehreren Lösungsmitteln.

**13.** Organische optoelektronische Vorrichtung, aufweisend ein organisches Molekül nach Anspruch 1 bis 8 oder eine Zusammensetzung nach Anspruch 12, insbesondere ausgeformt als eine Vorrichtung ausgewählt aus der Gruppe bestehend ausorganischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**14.** Organische optoelektronische Vorrichtung nach Anspruch 13, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein organisches Molekül nach Anspruch 1 bis 8 oder eine Zusammensetzung nach Anspruch 12 aufweist.

**15.** Verfahren zur Herstellung eines optoelektronischen Bauelements, wobei ein organisches Molekül nach Anspruch 1 bis 8 verwendet wird, insbesondere umfassend die Verarbeitung des organischen Moleküls mittels eines Vakuumverdampfungsverfahrens oder aus einer Lösung.

## Claims

**1.** Organic molecule, comprising

- a first chemical unit comprising or consisting of a structure of formula I

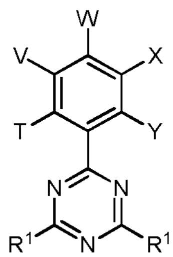

Formula I

and
- a second chemical unit comprising or consisting of a structure of formula II

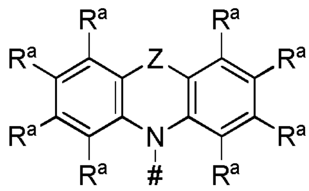

Formula II

wherein the first chemical unit is connected to the second chemical unit via a single bond; with

T is the attachment point of the single bond between the first chemical unit and the second chemical unit or is selected from the group consisting of $R^2$ and CN;

V is H or is the attachment point of the single bond between the first chemical unit and the second chemical unit;

W is an attachment point of the single bond between the first chemical unit and the second chemical unit or is selected from the group consisting of $R^2$ and CN;

X is selected from the group consisting of $R^2$ and CN;

Y is selected from the group consisting of $R^2$ and CN;

# indicates the attachment point of the single bond between the second chemical unit and the first chemical unit;

Z is the same or different at each instance and is a direct bond or is selected from the group consisting of $CR^3R^4$, $C=CR^3R^4$, C=O, $C=NR^3$, $NR^3$, O, $SiR^3R^4$, S, S(O) and $S(O)_2$;

$R^1$ is the same or different at each instance and is selected from the group consisting of:

- H, deuterium,
- a linear alkyl group having 1 to 5 carbon atoms, a linear alkenyl or alkynyl group having 2 to 8 carbon atoms, a branched or cyclic alkyl, alkenyl or alkynyl group having 3 to 10 carbon atoms, wherein one or more hydrogen atoms in the aforementioned groups may be replaced by deuterium; and
- an aromatic or heteroaromatic ring system which has 5 to 15 aromatic ring atoms and may be substituted in each case by one or more $R^6$ moieties;

$R^2$ is the same or different at each instance and is selected from the group consisting of:

- H, deuterium,
- a linear alkyl group having 1 to 5 carbon atoms, a linear alkenyl or alkynyl group having 2 to 8 carbon atoms, a branched or cyclic alkyl, alkenyl or alkynyl group having 3 to 10 carbon atoms, wherein one or more hydrogen atoms in the aforementioned groups may be replaced by deuterium; and
- an aromatic or heteroaromatic ring system which has 5 to 15 aromatic ring atoms and may be substituted in each case by one or more $R^6$ moieties;

$R^a$, $R^3$ and $R^4$ are the same or different at each instance and are selected from the group consisting of:

- H, deuterium, $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group which has 1 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- a linear alkenyl or alkynyl group which has 2 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^5$ moieties;

- an aryloxy or heteroaryloxy group which has 5 to 60 aromatic ring atoms and may be substituted in each case by one or more $R^5$ moieties; and
- a diarylamino group, diheteroarylamino group or arylheteroarylamino group which has 10 to 40 aromatic ring atoms and may be substituted in each case by one or more $R^5$ moieties;

$R^5$ is the same or different at each instance and is selected from the group consisting of:

- H, deuterium, $N(R^6)_2$, OH, $Si(R^6)_3$, $B(OR^6)_2$, $OSO_2R^6$, $CF_3$, CN, F, Br, I;
- a linear alkyl, alkoxy or thioalkoxy group which has 1 to 40 carbon atoms and may be substituted in each case by one or more $R^6$ moieties, wherein one or more nonadjacent $CH_2$ groups may be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more hydrogen atoms may be replaced by deuterium, CN, $CF_3$ or $NO_2$;
- a linear alkenyl or alkynyl group which has 2 to 40 carbon atoms and may be substituted in each case by one or more $R^6$ moieties, wherein one or more nonadjacent $CH_2$ groups may be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^6$ moieties, wherein one or more nonadjacent $CH_2$ groups may be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^6$ moieties;
- an aryloxy or heteroaryloxy group which has 5 to 60 aromatic ring atoms and may be substituted in each case by one or more $R^6$ moieties; and
- a diarylamino group, diheteroarylamino group or arylheteroarylamino group which has 10 to 40 aromatic ring atoms and may be substituted in each case by one or more $R^6$ moieties;

$R^6$ is the same or different at each instance and is selected from the group consisting of:

- H, deuterium, OH, $CF_3$, CN, F;
- a linear alkyl, alkoxy or thioalkoxy group having 1 to 5 carbon atoms, wherein one or more hydrogen atoms may be replaced by deuterium, CN, $CF_3$ or $NO_2$;
- a linear alkenyl or alkynyl group having 2 to 5 carbon atoms, wherein one or more hydrogen atoms may be replaced by deuterium, CN, $CF_3$ or $NO_2$;
- a branched or cyclic alkyl, alkenyl, alkynyl, alkoxy or thioalkoxy group having 3 to 5 carbon atoms, wherein one or more hydrogen atoms may be replaced by deuterium, CN, $CF_3$ or $NO_2$;
- an aromatic or heteroaromatic ring system having 5 to 60 aromatic ring atoms;
- an aryloxy or heteroaryloxy group having 5 to 60 aromatic ring atoms; and
- a diarylamino group, diheteroarylamino group or arylheteroarylamino group having 10 to 40 aromatic ring atoms;

wherein each of the $R^a$, $R^3$, $R^4$ or $R^5$ moieties together with one or more further $R^a$, $R^3$, $R^4$ or $R^5$ moieties may form a mono- or polycyclic, aliphatic, aromatic and/or benzofused ring system; wherein
exactly one moiety selected from the group consisting of T, W, X and Y is CN and exactly one moiety selected from the group consisting of T, V and W is the attachment point of the single bond between the first chemical unit of formula I and the second chemical unit, and
W is also H when T is CN and V is the attachment point of the single bond between the first chemical unit and the second chemical unit,
wherein a compound of the formula

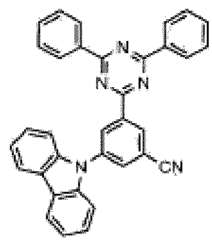

is excluded.

**2.** Organic molecule according to claim 1, wherein $R^1$ is the same or different at each instance and is methyl or phenyl.

**3.** Organic molecule according to claim 1 or 2, wherein $R^2$ is the same or different at each instance and is H, methyl or phenyl.

**4.** Organic molecule according to claim 1 to 3, wherein W is CN.

**5.** Organic molecule according to claims 1 to 4, wherein the second chemical unit comprises a structure of the formula IIa:

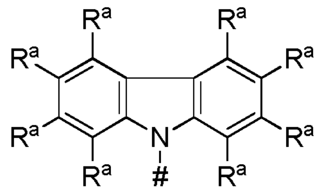

Formula IIa

wherein the definitions given in claim 1 are applicable to # and $R^a$.

**6.** Organic molecule according to claims 1 to 5, wherein the second chemical unit comprises a structure of the formula IIb:

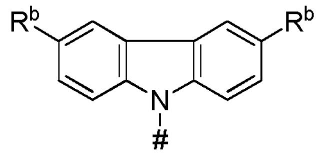

Formula IIb

wherein

$R^b$ is the same or different at each instance and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group which has 1 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- a linear alkenyl or alkynyl group which has 2 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^5$ moieties;
- an aryloxy or heteroaryloxy group which has 5 to 60 aromatic ring atoms and may be substituted in each case by one or more $R^5$ moieties; and
- a diarylamino group, diheteroarylamino group or arylheteroarylamino group which has 10 to 40 aromatic ring atoms and may be substituted in each case by one or more $R^5$ moieties;

and the definitions given in claim 1 are applicable to # and $R^5$.

**7.** Organic molecule according to claims 1 to 5, wherein the second chemical unit comprises a structure of the formula IIc:

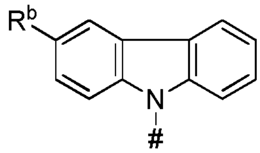

Formula IIc

wherein

$R^b$ is the same or different at each instance and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group which has 1 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- a linear alkenyl or alkynyl group which has 2 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^5$ moieties, wherein one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^5$ moieties;
- an aryloxy or heteroaryloxy group which has 5 to 60 aromatic ring atoms and may be substituted in each case by one or more $R^5$ moieties; and
- a diarylamino group, diheteroarylamino group or arylheteroarylamino group which has 10 to 40 aromatic ring atoms and may be substituted in each case by one or more $R^5$ moieties;

and for the rest the definitions given in claim 1 are applicable.

**8.** Organic molecule according to claim 6 or 7, wherein $R^b$ is the same or different at each instance and is
Me, $^iPr$, $^tBu$, CN, $CF_3$,
Ph which may be substituted in each case by one or more moieties selected from Me, $^iPr$, $^tBu$, CN, $CF_3$ and Ph,
pyridinyl which may be substituted in each case by one or more moieties selected from Me, $^iPr$, $^tBu$, CN, $CF_3$ and Ph,
pyrimidinyl which may be substituted in each case by one or more moieties selected from Me, $^iPr$, $^tBu$, CN, $CF_3$ and Ph, or
triazinyl which may be substituted in each case by one or more moieties selected from Me, $^iPr$, $^tBu$, CN, $CF_3$ and Ph.

**9.** Method for preparing an organic molecule according to claims 1 to 8, wherein a 4- and 6-$R^1$-substituted 2-halo-1,3,5-triazine is provided as reactant.

**10.** Use of an organic molecule according to claims 1 to 8 as luminescent emitter and/or as host material and/or as electron transport material and/or as hole injection material and/or as hole blocker material in an organic optoelectronic device.

**11.** Use according to claim 10, wherein the organic optoelectronic device is selected from the group consisting of:

- organic light-emitting diodes (OLEDs),
- light-emitting electrochemical cells,
- OLED sensors, especially in gas and vapor sensors not hermetically shielded from the outside,
- organic diodes,

• organic solar cells,
• organic transistors,
• organic field-effect transistors,
• organic lasers and
• down-conversion elements.

**12.** Composition, comprising or consisting of:

(a) at least one organic molecule according to any of claims 1 to 8, especially in the form of an emitter and/or host, and
(b) one or more emitter and/or host materials other than the molecule according to claims 1 to 8, and
(c) optionally, one or more dyes and/or one or more solvents.

**13.** Organic optoelectronic device comprising an organic molecule according to claims 1 to 8 or a composition according to claim 12, especially in the form of a device selected from the group consisting of organic light-emitting diode (OLED), light-emitting electrochemical cell, OLED sensor, especially gas and vapor sensors that are not hermetically shielded from the outside, organic diode, organic solar cell, organic transistor, organic field effect transistor, organic laser and down-conversion element.

**14.** Organic optoelectronic device according to claim 13, comprising

- a substrate,
- an anode and
- a cathode, wherein the anode or the cathode has been applied to the substrate, and
- at least one light-emitting layer which is arranged between the anode and the cathode and comprises an organic molecule according to claims 1 to 8 or a composition according to claim 12.

**15.** Method for producing an optoelectronic device, wherein an organic molecule according to claims 1 to 8 is used, in particular comprising the processing of the organic molecule by means of a vacuum evaporation method or from a solution.

**Revendications**

**1.** Molécule organique, comprenant :

- une première unité chimique comprenant ou constituée par une structure selon la formule I

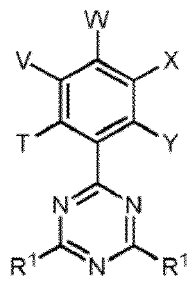

Formule I

et
- une deuxième unité chimique comprenant ou constituée par une structure selon la formule II

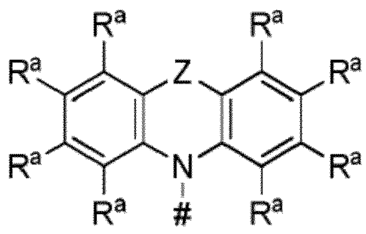

Formule II

la première unité chimique étant reliée par une simple liaison avec la deuxième unité chimique ;
T étant le point de liaison de la simple liaison entre la première unité chimique et la deuxième unité chimique, ou étant choisi dans le groupe constitué par $R^2$ et CN ;
V représentant H ou étant le point de liaison de la simple liaison entre la première unité chimique et la deuxième unité chimique ;
W étant le point de liaison de la simple liaison entre la première unité chimique et la deuxième unité chimique, ou étant choisi dans le groupe constitué par $R^2$ et CN ;
X étant choisi dans le groupe constitué par $R^2$ et CN ;
Y étant choisi dans le groupe constitué par $R^2$ et CN ;
# désignant le point de liaison de la simple liaison entre la deuxième unité chimique et la première unité chimique ;
les Z représentant à chaque occurrence de manière identique ou différente une liaison directe ou étant choisis dans le groupe constitué par $CR^3R^4$, $C=CR^3R^4$, C=O $C=NR^3$, $NR^3$, O, $SiR^3R^4$, S, S(O) et $S(O)_2$;
les $R^1$ étant choisis à chaque occurrence de manière identique ou différente dans le groupe constitué par :

- H, le deutérium,
- un groupe alkyle linéaire de 1 à 5 atomes C, un groupe alcényle ou alcynyle linéaire de 2 à 8 atomes C, un groupe alkyle, alcényle ou alcynyle ramifié ou cyclique de 3 à 10 atomes C, un ou plusieurs atomes H pouvant être remplacés par le deutérium dans les groupes susmentionnés ; et
- un système cyclique aromatique ou hétéroaromatique de 5 à 15 atomes de cycle aromatique, qui peut à chaque fois être substitué avec un ou plusieurs radicaux $R^6$ ;

les $R^2$ étant choisis à chaque occurrence de manière identique ou différente dans le groupe constitué par :

- H, le deutérium,
- un groupe alkyle linéaire de 1 à 5 atomes C, un groupe alcényle ou alcynyle linéaire de 2 à 8 atomes C, un groupe alkyle, alcényle ou alcynyle ramifié ou cyclique de 3 à 10 atomes C, un ou plusieurs atomes H pouvant être remplacés par le deutérium dans les groupes susmentionnés ; et
- un système cyclique aromatique ou hétéroaromatique de 5 à 15 atomes de cycle aromatique, qui peut à chaque fois être substitué avec un ou plusieurs radicaux $R^6$ ;

$R^a$, $R^3$ et $R^4$ étant choisis à chaque occurrence de manière identique ou différente dans le groupe constitué par :

- H, le deutérium, $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;

- un système cyclique aromatique ou hétéroaromatique de 5 à 60 atomes de cycle aromatique, qui peut à chaque fois être substitué avec un ou plusieurs radicaux $R^5$ ;
- un groupe aryloxy ou hétéroaryloxy de 5 à 60 atomes de cycle aromatique, qui peut à chaque fois être substitué avec un ou plusieurs radicaux $R^5$ ; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^5$;

les $R^5$ étant choisis à chaque occurrence de manière identique ou différente dans le groupe constitué par :

- H, le deutérium, $N(R^6)_2$, OH, $Si(R^6)_3$, $B(OR^6)_2$, $OSO_2R^6$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^6$, un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^6$, un ou plusieurs groupes $CH_2$ non voisins pouvant être remplacés par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^6$, un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$;
- un système cyclique aromatique ou hétéroaromatique de 5 à 60 atomes de cycle aromatique, qui peut à chaque fois être substitué avec un ou plusieurs radicaux $R^6$ ;
- un groupe aryloxy ou hétéroaryloxy de 5 à 60 atomes de cycle aromatique, qui peut à chaque fois être substitué avec un ou plusieurs radicaux $R^6$ ; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^6$;

les $R^6$ étant choisis à chaque occurrence de manière identique ou différente dans le groupe constitué par :

- H, le deutérium, OH, $CF_3$, CN, F ;
- un groupe alkyle, alcoxy ou thioalcoxy linéaire de 1 à 5 atomes C, un ou plusieurs atomes H pouvant être remplacés par le deutérium, CN, $CF_3$ ou $NO_2$ ;
- un groupe alcényle ou alcynyle linéaire de 2 à 5 atomes C, un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 5 atomes C, un ou plusieurs atomes H pouvant être remplacés par le deutérium, CN, $CF_3$ ou $NO_2$;
- un système cyclique aromatique ou hétéroaromatique de 5 à 60 atomes de cycle aromatique ;
- un groupe aryloxy ou hétéroaryloxy de 5 à 60 atomes de cycle aromatique ; et
- un groupe diarylamino, un groupe dihétéroarylamino ou un groupe arylhétéroarylamino de 10 à 40 atomes de cycle aromatique ;

les radicaux $R^a$, $R^3$, $R^4$ ou $R^5$ pouvant également former avec un ou plusieurs autres radicaux $R^a$, $R^3$, $R^4$ ou $R^5$ un système cyclique mono- ou polycyclique, aliphatique, aromatique et/ou benzoannelé ;
exactement un radical choisi dans le groupe constitué par T, W, X et Y représentant CN, et exactement un radical choisi dans le groupe constitué par T, V et W représentant le point de liaison de la simple liaison entre la première unité chimique selon la formule I et la deuxième unité chimique ; et
W représentant H lorsque T représente CN et V représente le point de liaison de la simple liaison entre la première unité chimique et la deuxième unité chimique ;
un composé de formule

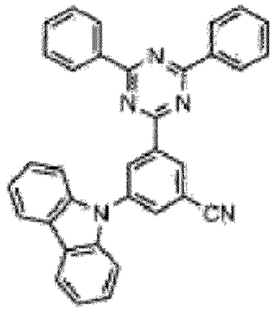

étant exclu.

**2.** Molécule organique selon la revendication 1, dans laquelle les $R^1$ représentent à chaque occurrence de manière identique ou différente méthyle ou phényle.

**3.** Molécule organique selon la revendication 1 ou 2, dans laquelle les $R^2$ représentent à chaque occurrence de manière identique ou différente H, méthyle ou phényle.

**4.** Molécule organique selon les revendications 1 à 3, dans laquelle W représente CN.

**5.** Molécule organique selon les revendications 1 à 4, dans laquelle la deuxième unité chimique présente une structure de la formule IIa :

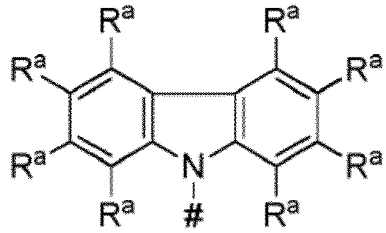

Formule IIa

dans laquelle # et $R^a$ ont les définitions indiquées dans la revendication 1.

**6.** Molécule organique selon les revendications 1 à 5, dans laquelle la deuxième unité chimique présente une structure de formule IIb :

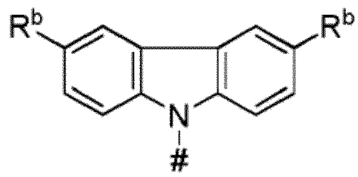

Formule IIb

dans laquelle

les $R^b$ sont choisis à chaque occurrence de manière identique ou différente dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^5$, un ou plusieurs groupes $CH_2$ non voisins pouvant être remplacés par

$R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$;
- un système cyclique aromatique ou hétéroaromatique de 5 à 60 atomes de cycle aromatique, qui peut à chaque fois être substitué avec un ou plusieurs radicaux $R^5$ ;
- un groupe aryloxy ou hétéroaryloxy de 5 à 60 atomes de cycle aromatique, qui peut à chaque fois être substitué avec un ou plusieurs radicaux $R^5$; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^5$;

# et $R^5$ ayant les définitions indiquées dans la revendication 1.

**7.** Molécule organique selon les revendications 1 à 5, dans laquelle la deuxième unité chimique présente une structure de formule IIc :

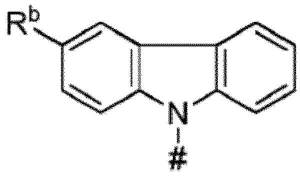

Formule IIc

dans laquelle

les $R^b$ sont choisis à chaque occurrence de manière identique ou différente dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$;
- un système cyclique aromatique ou hétéroaromatique de 5 à 60 atomes de cycle aromatique, qui peut à chaque fois être substitué avec un ou plusieurs radicaux $R^5$ ;
- un groupe aryloxy ou hétéroaryloxy de 5 à 60 atomes de cycle aromatique, qui peut à chaque fois être substitué avec un ou plusieurs radicaux $R^5$; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^5$;

les définitions indiquées dans la revendication 1 s'appliquant pour le reste.

**8.** Molécule organique selon la revendication 6 ou 7, dans laquelle les $R^b$ représentent à chaque occurrence de manière identique ou différente :

Me, $^{i}Pr$, $^{t}Bu$, CN, $CF_3$,
Ph, qui peut à chaque fois être substitué avec un ou plusieurs radicaux choisis parmi Me, $^{i}Pr$, $^{t}Bu$, CN, $CF_3$ ou Ph,
pyridinyle, qui peut à chaque fois être substitué avec un ou plusieurs radicaux choisis parmi Me, $^{i}Pr$, $^{t}Bu$, CN, $CF_3$ ou Ph,
pyrimidinyle, qui peut à chaque fois être substitué avec un ou plusieurs radicaux choisis parmi Me, $^{i}Pr$, $^{t}Bu$, CN, $CF_3$ ou Ph, ou
triazinyle, qui peut à chaque fois être substitué avec un ou plusieurs radicaux choisis parmi Me, $^{i}Pr$, $^{t}Bu$, CN, $CF_3$ ou Ph.

**9.** Procédé de fabrication d'une molécule organique selon les revendications 1 à 8, selon lequel une 2-halogéno-1,3,5-triazine substituée par $R^1$ en position 4 et 6 est utilisée en tant que réactif.

**10.** Utilisation d'une molécule organique selon les revendications 1 à 8 en tant qu'émetteur luminescent et/ou en tant que matériau hôte et/ou en tant que matériau de transport d'électrons et/ou en tant que matériau d'injection de trous et/ou en tant que matériau de blocage de trous dans un dispositif optoélectronique organique.

**11.** Utilisation selon la revendication 10, dans laquelle le dispositif optoélectronique organique est choisi dans le groupe constitué par :

- les diodes organiques électroluminescentes (OLED),
- les cellules électrochimiques électroluminescentes,
- les capteurs à OLED, notamment dans des capteurs de gaz et de vapeur non blindés hermétiquement vers l'extérieur,
- les diodes organiques,
- les cellules solaires organiques,
- les transistors organiques,
- les transistors à effet de champ organiques ;
- les lasers organiques et
- les éléments de conversion descendante.

**12.** Composition, comprenant ou constituée par :

(a) au moins une molécule organique selon l'une quelconque des revendications 1 à 8, notamment sous la forme d'un émetteur et/ou d'un hôte, et
(b) un ou plusieurs matériaux émetteurs et/ou hôtes, qui sont différents de la molécule selon les revendications 1 à 8 ; et
(c) éventuellement un ou plusieurs colorants et/ou un ou plusieurs solvants.

**13.** Dispositif optoélectronique organique, comprenant une molécule organique selon les revendications 1 à 8 ou une composition selon la revendication 12, notamment configurée sous la forme d'un dispositif choisi dans le groupe constitué par une diode organique électroluminescente (OLED), une cellule électrochimique électroluminescente, un capteur d'OLED, notamment des capteurs de gaz et de vapeur non blindés hermétiquement vers l'extérieur, une diode organique, une cellule solaire organique, un transistor organique, un transistor à effet de champ organique, un laser organique et un élément de conversion descendante.

**14.** Dispositif optoélectronique organique selon la revendication 13, comprenant :

- un substrat,
- une anode et
- une cathode, l'anode ou la cathode étant appliquée sur le substrat et
- au moins une couche électroluminescente, qui est agencée entre l'anode et la cathode, et comprend une molécule organique selon les revendications 1 à 8 ou une composition selon la revendication 12.

**15.** Procédé de fabrication d'un composant optoélectronique, selon lequel une molécule organique selon les revendications 1 à 8 est utilisée, notamment comprenant le traitement de la molécule organique par un procédé d'évaporation sous vide ou à partir d'une solution.

**Figur 1**

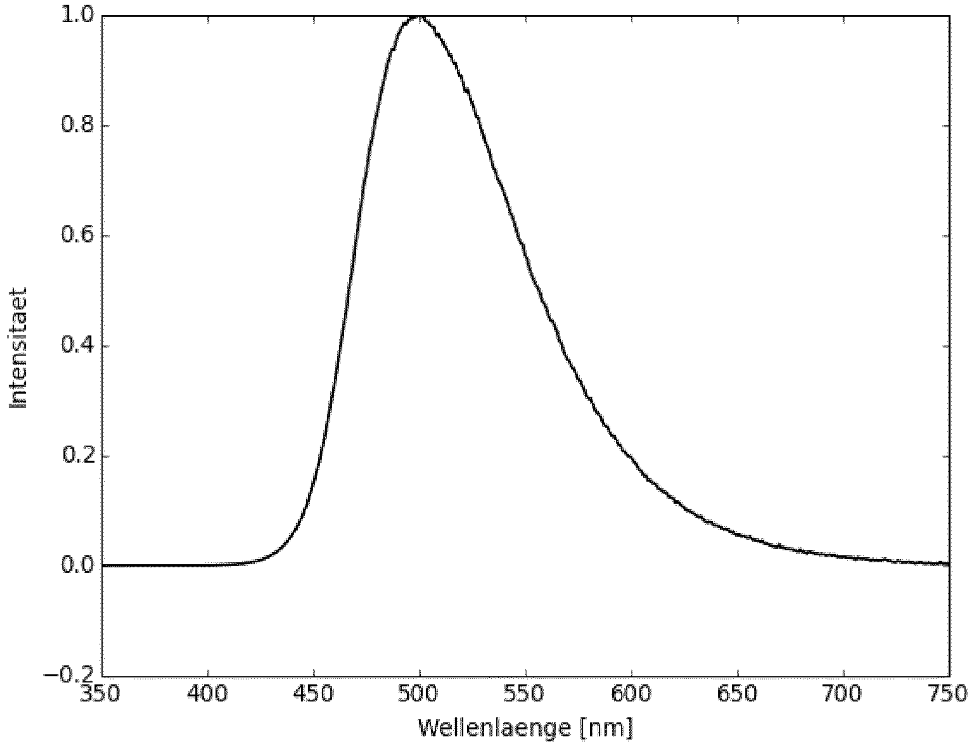


**Figur 2**

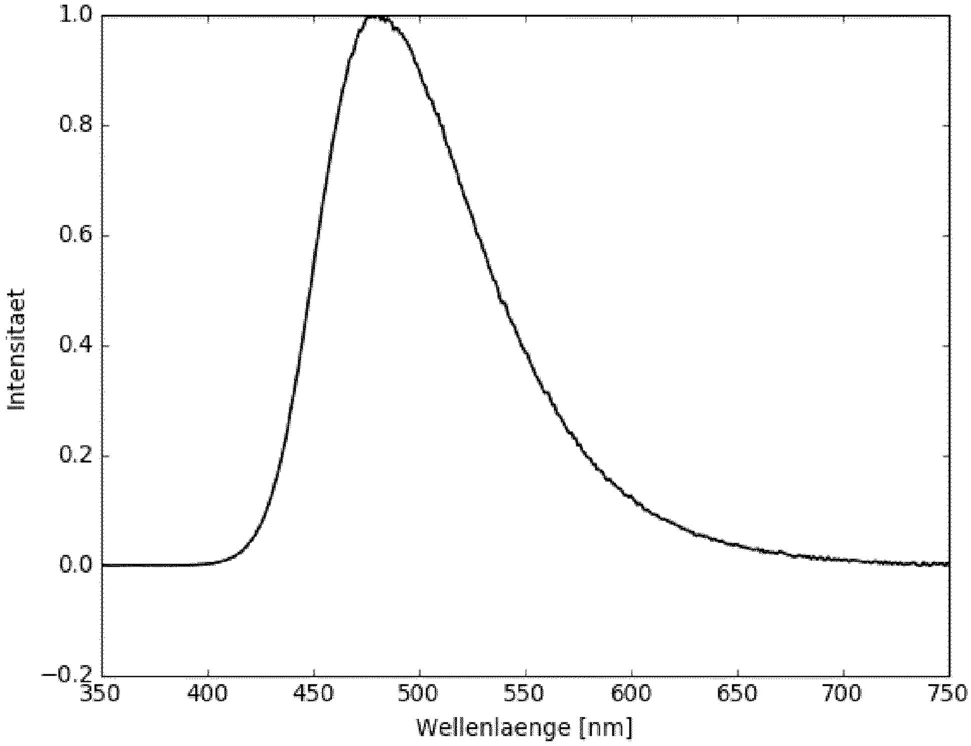

Figur 3

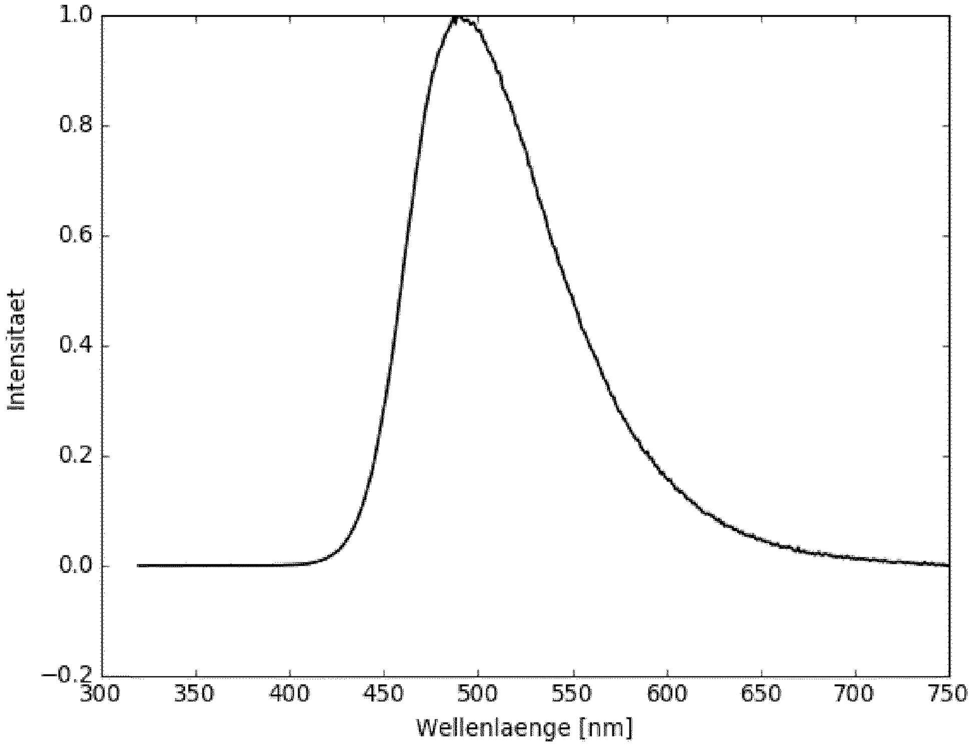


Figur 4

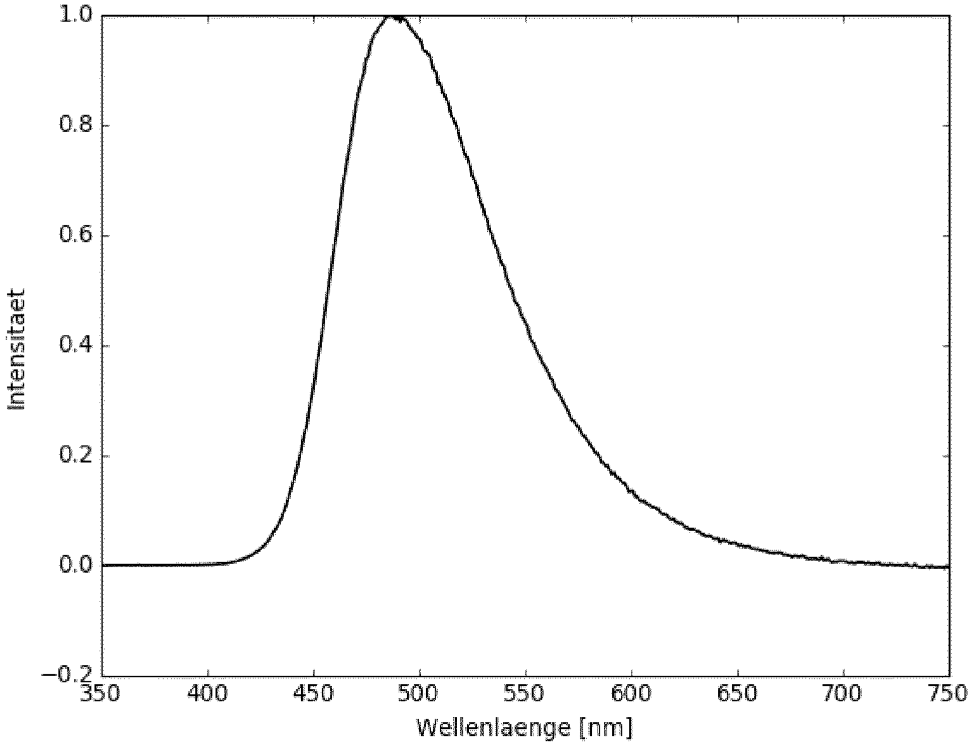

Figur 5

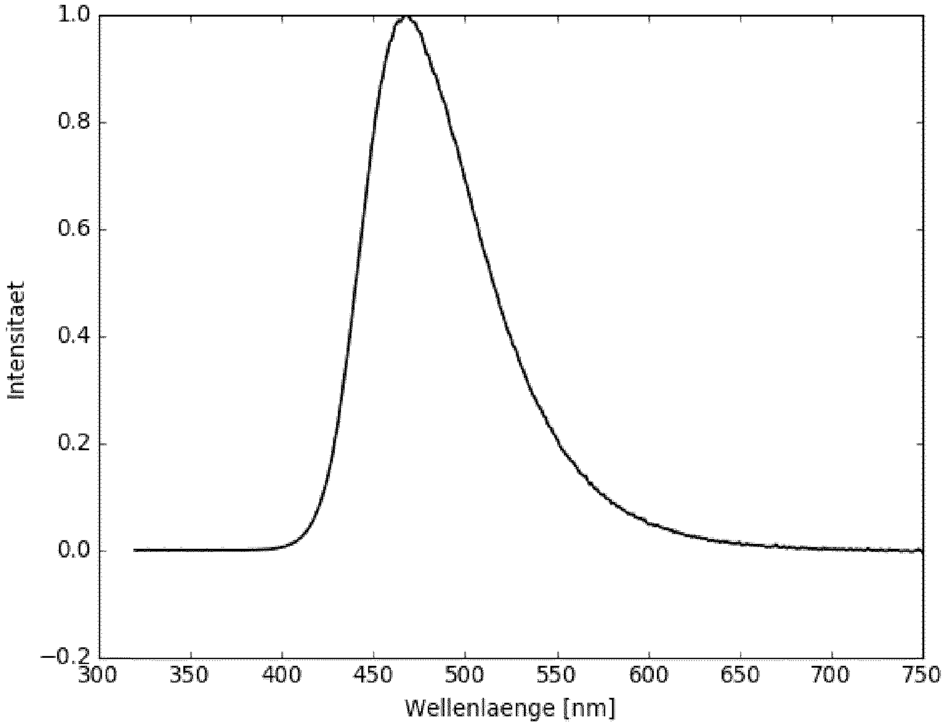


Figur 6

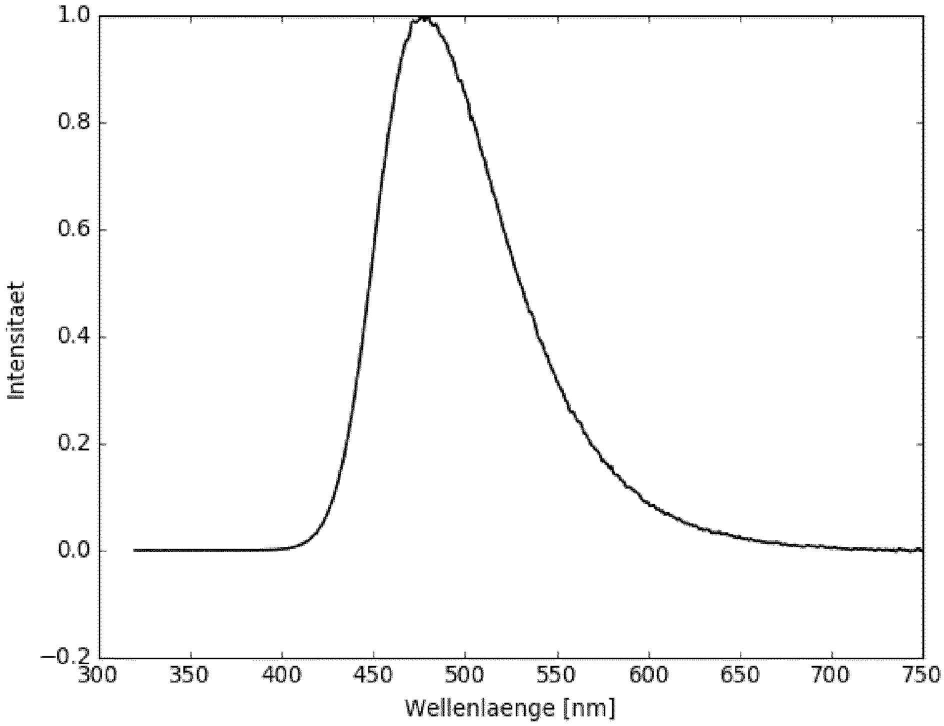

Figur 7

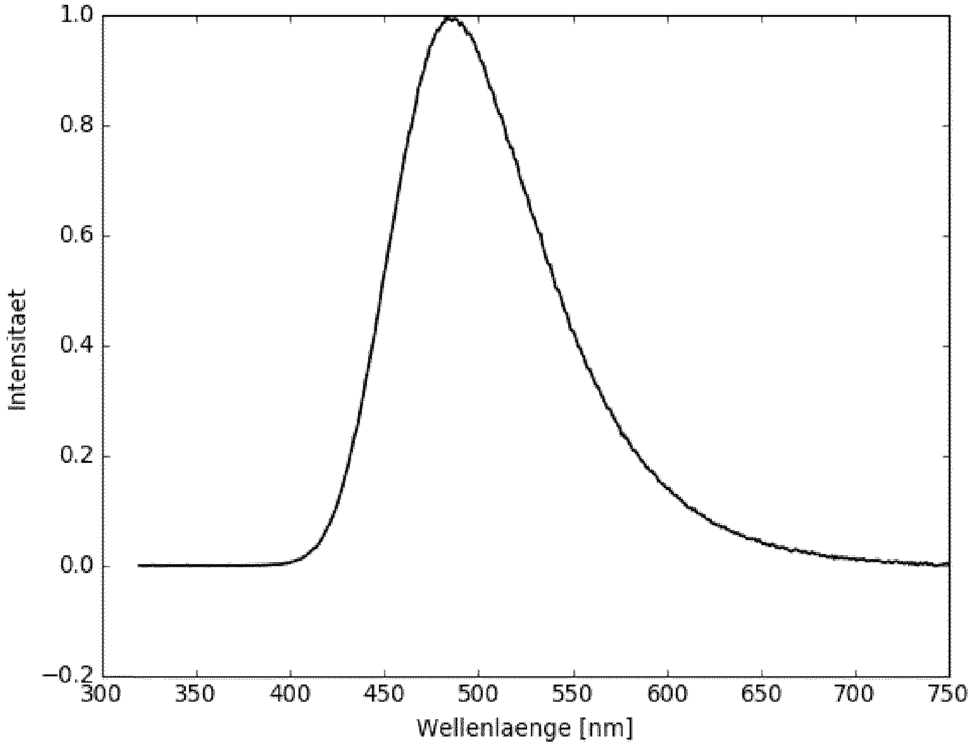


Figur 8

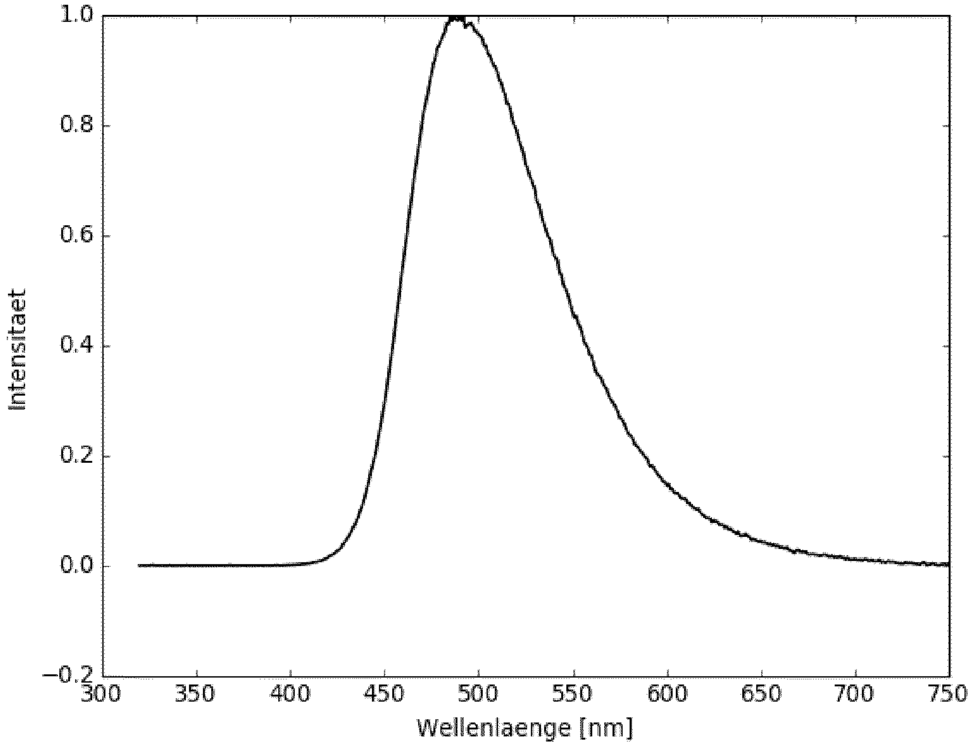

**Figur 9**

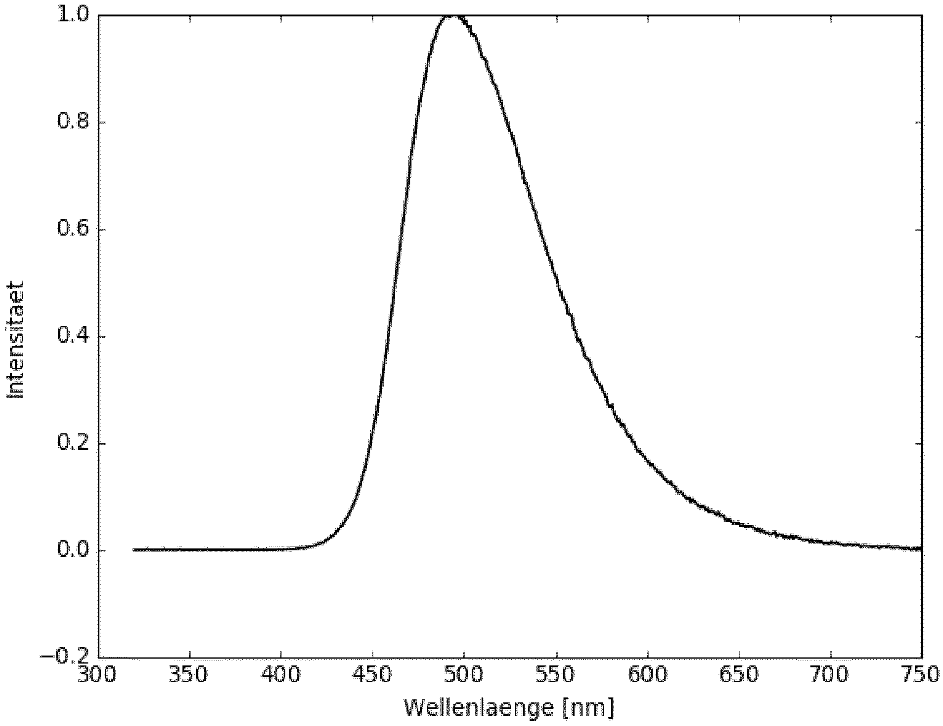


**Figur 10**

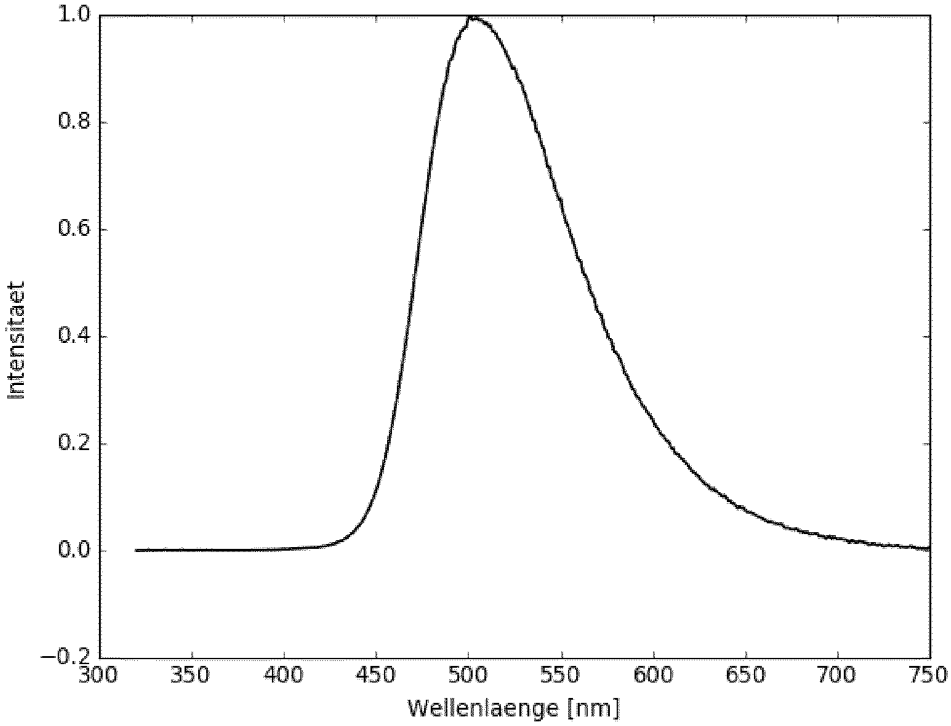

Figur 11

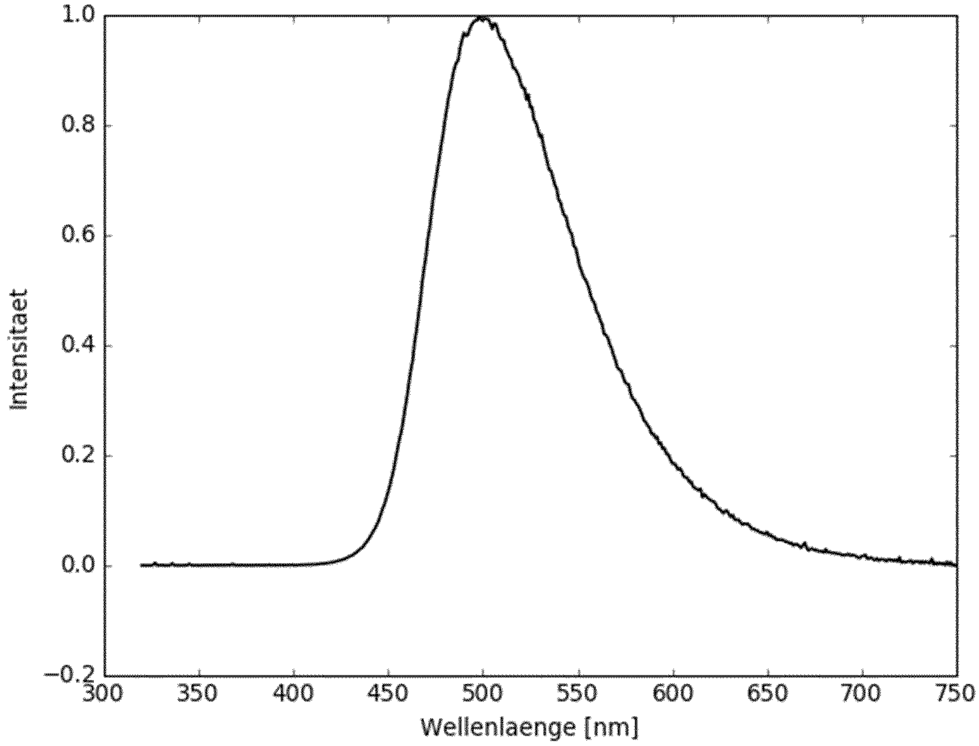


Figur 12

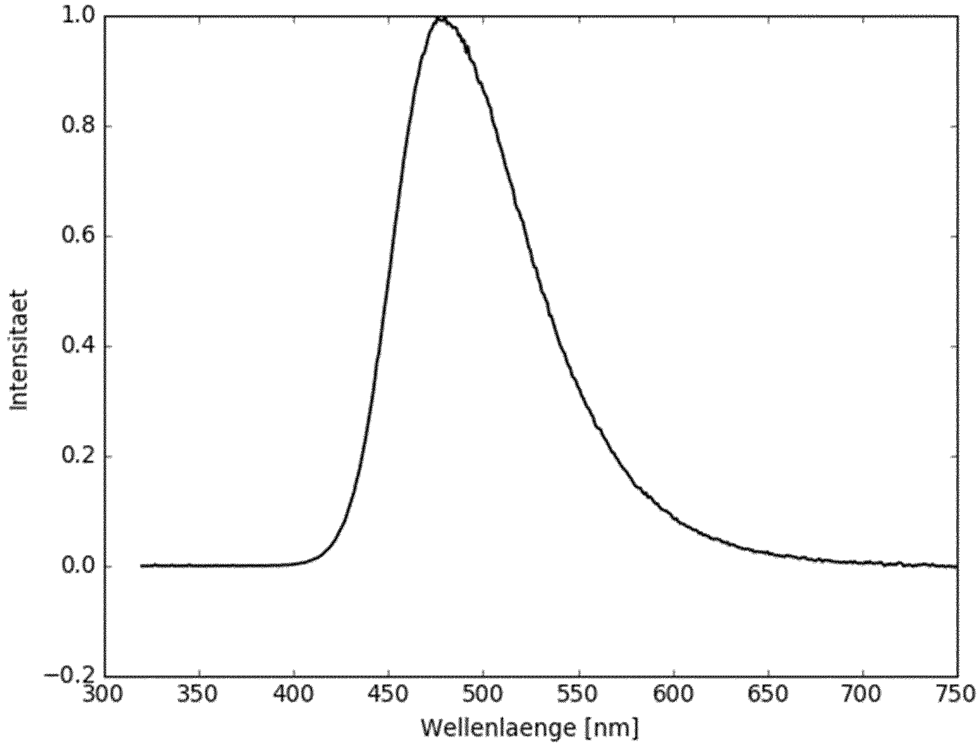

Figur 13

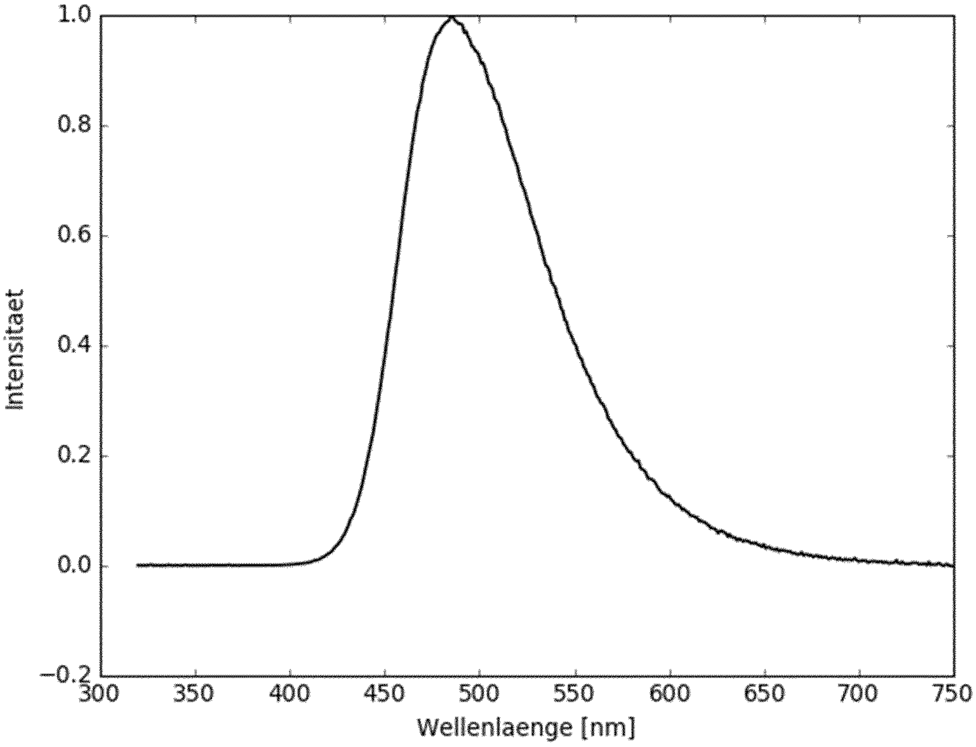


Figur 14

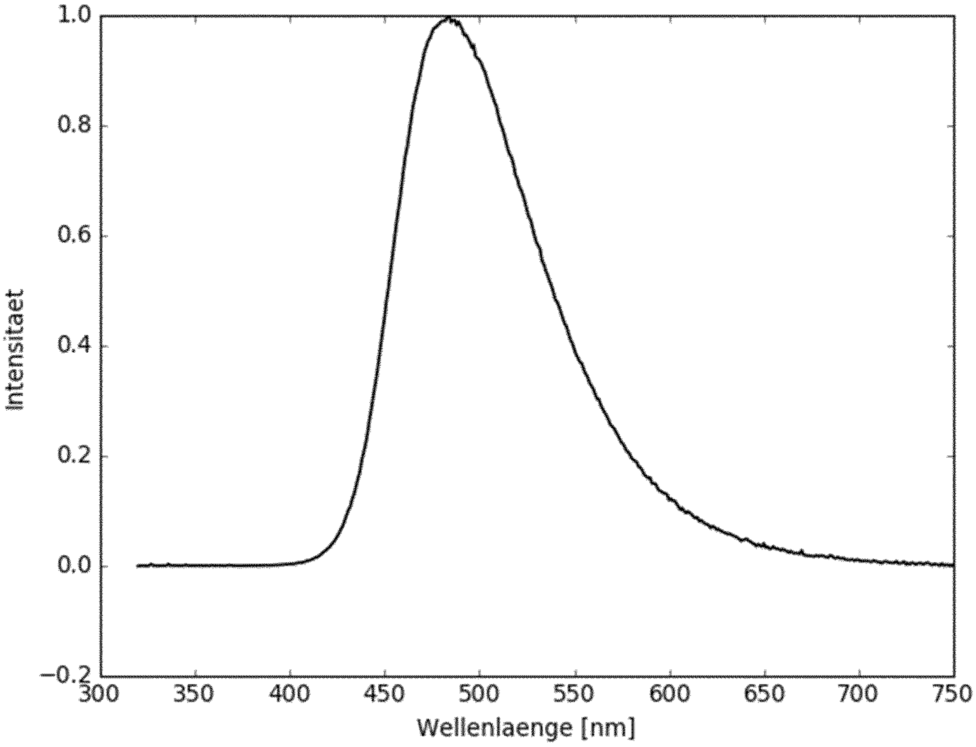

Figur 15

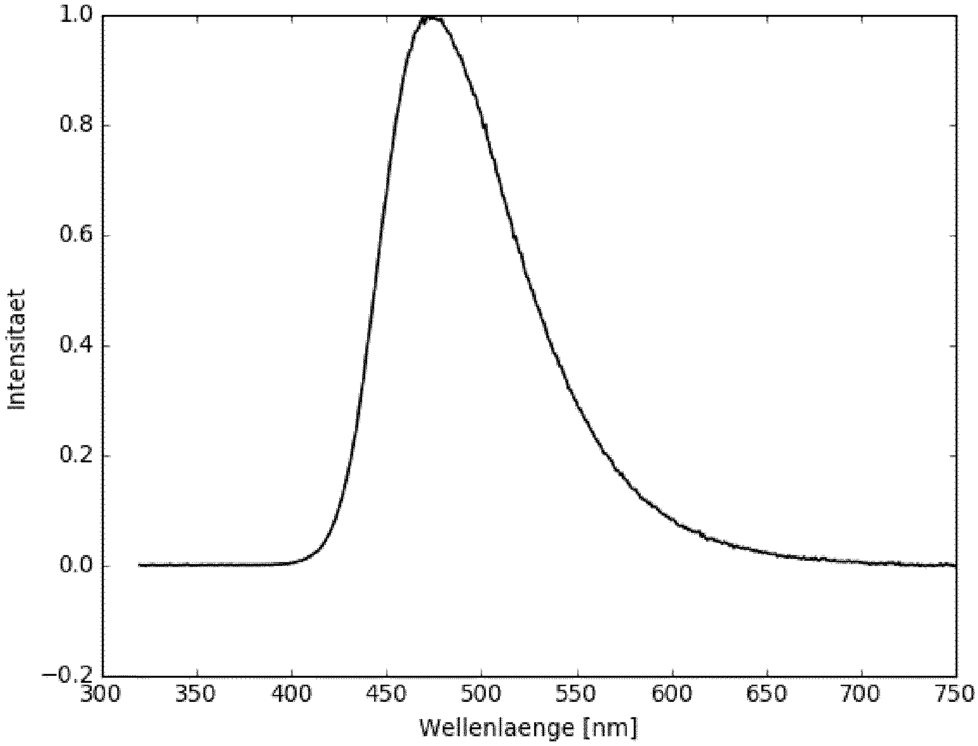


Figur 16

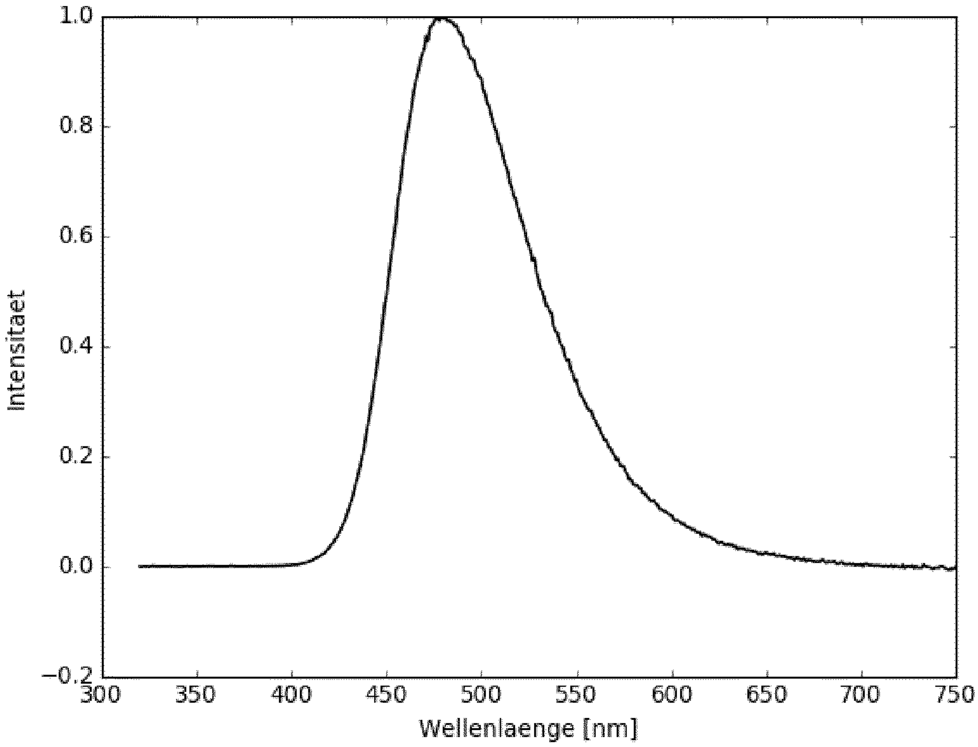

Figur 17

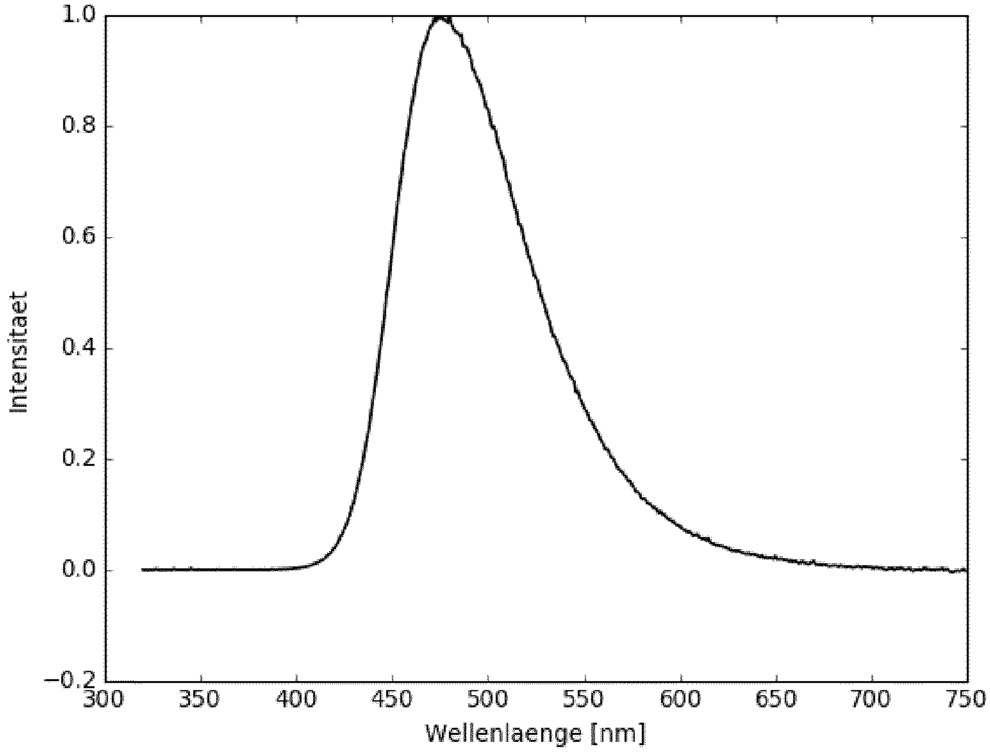
1.0
0.8
0.6
0.4
0.2
0.0
−0.2
Intensitaet
300
350
400
450
500
550
600
650
700
750
Wellenlaenge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2018016898 A1

### In der Beschreibung aufgeführte Nicht-Patentliteratur

- **M.E. THOMPSON et al.** *Chem. Mater.,* 2004, vol. 16, 4743
- *CHEMICAL ABSTRACTS,* 73183-34-3